(19) Europäisches Patentamt
European Patent Office
Office européen des brevets

(11) EP 0 936 218 B1

(12) EUROPEAN PATENT SPECIFICATION

(45) Date of publication and mention of the grant of the patent:
**02.04.2003 Bulletin 2003/14**

(21) Application number: **97929510.2**

(22) Date of filing: **03.07.1997**

(51) Int Cl.[7]: **C07D 235/08**, C07D 235/18, C07D 235/12, C07D 403/06, A61K 31/415

(86) International application number:
**PCT/JP97/02308**

(87) International publication number:
**WO 98/001429 (15.01.1998 Gazette 1998/02)**

# (54) BENZIMIDAZOLE DERIVATIVES

BENZIMIDAZOLDERIVATE

DERIVES DE BENZIMIDAZOLE

(84) Designated Contracting States:
**AT BE CH DE DK ES FI FR GB GR IE IT LI LU MC NL PT SE**

(30) Priority: **05.07.1996 JP 17671196**

(43) Date of publication of application:
**18.08.1999 Bulletin 1999/33**

(73) Proprietor: **MOCHIDA PHARMACEUTICAL CO., LTD.**
**Shinjuku-ku, Tokyo 160-0004 (JP)**

(72) Inventors:
- **MIZUGUCHI, Kiyoshi**
  **Shinjuku-ku, Tokyo 160 (JP)**
- **OHZAWA, Nobuo**
  **Shinjuku-ku, Tokyo 160 (JP)**
- **NAKAI, Yasuhiro**
  **Shinjuku-ku, Tokyo 160 (JP)**
- **MATSUURA, Kazuyuki**
  **Shinjuku-ku, Tokyo 160 (JP)**
- **OHNISHI, Shuhei**
  **Shinjuku-ku, Tokyo 160 (JP)**

(74) Representative: **Grünecker, Kinkeldey, Stockmair & Schwanhäusser Anwaltssozietät**
**Maximilianstrasse 58**
**80538 München (DE)**

(56) References cited:
**WO-A-96/04279** **JP-A- 62 005 966**

- **BUCKLE D R ET AL: "NOVEL 1H-BENZIMIDAZOL-4-OLS WITH POTENT 5-LIPOXYGENASE INHIBITORY ACTIVITY" JOURNAL OF MEDICINAL CHEMISTRY,US,AMERICAN CHEMICAL SOCIETY. WASHINGTON, vol. 30, no. 12, 1 December 1987 (1987-12-01), pages 2216-2221, XP002041812 ISSN: 0022-2623**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 936 218 B1

## Description

TECHNICAL FIELD

This invention relates to novel benzimidazole derivatives, a process for producing the same, and drugs containing as the active ingredient at least one of these compounds, in particular, for preventing and/or treating diseases exhibiting eosinophilia, bronchial asthma and allergic diseases.

BACKGROUND ART

A phenomenon in which eosinophils increase in blood or tissues, namely differentiating, inducing or infiltrating phenomenon, is observed in many diseases. It is important from the clinical point of view to differentiate these diseases between certain diseases in which eosinophilia is frequently observed but its direct concern in morbid states is not probable and other diseases in which eosinophils are probably concerned as the main immune cell in the morbid states of such diseases. Addison disease, ulcerative colitis and the like diseases can be exemplified as diseases which correspond to the former case. Examples of the latter case include parasite infection, hypereosinophilic syndrome (HES), eosinophilic pneumonia, eosinophilic enterogastritis, bronchial asthma and the like diseases. Since eosinophils are closely related to the morbid state of bronchial asthma, so-called eosinophilic bronchitis is recently taking root as a morbid state concept. Particularly, there are some common points among movements of eosinophils which are concerned in these diseases. That is, they are summarized into three points of 1) acceleration of eosinophil production and differentiation by eosinophil growth lymphokines mainly including interleukin 5 (IL-5), 2) migration and accumulation of eosinophils into an involved organ by eosinophil chemotactic activity and 3) activation of eosinophils and prolongation of their life survival in the morbid sites. It is considered that the just described three factors or matters exert tissue damage and inflammation inducing actions of eosinophils in these diseases, thereby concerning in their morbid states, though there are differences in terms of foci and the degree of clinical symptoms. Also, though there are differences in terms of the increasing degree of eosinophils, atopic dermatitis, allergic rhinitis and the like various diseases can be exemplified as the diseases which exhibit eosinophilia (S. Nakajima and J. Shigehara, Meaning of the Clinical Diagnoses of eosinophils, "Eosinophils" ed. by S. Makino and K. Ishikawa, pp. 165 - 173, Kokusai Igaku Shuppan, 1991).

In consequence, a compound which controls eosinophils, or inhibits increment or activation of eosinophils in blood or tissues, could be applied to parasite infection, hypereosinophilic syndrome (HES), eosinophilic pneumonia, eosinophilic enterogastritis, bronchial asthma, atopic dermatitis, allergic rhinitis and the like diseases that exhibit eosinophilia.

Under the present situation, only the administration of steroid drugs is attempted as a symptomatic therapy for the treatment of diseases which exhibit eosinophilia, and there are no therapeutic methods which target eosinophils. It is extremely difficult to use steroid drugs, because they frequently cause peculiar side effects such as reduction of resistance against bacterial infection, hyperglycemia, diabetes, gastric ulcer, hyperkalemia, osteoporosis, obesity and the like, and their use is strictly stipulated such as prohibition of sudden termination of their administration. In addition, conventional asthma-treating drugs have been developed mainly based on histamine. release inhibition action and the like, and it has been revealed that eosinophils are closely concerned also in this morbid state, so that the use of eosinophils as a target could be applied to certain types of asthma which cannot be treated by the conventional method. Under such situation, a compound which has high safety and can strongly control eosinophils seems to be markedly useful in a method of fundamental medical treatment of various diseases in which eosinophilia is concerned, so that realization of such a compound as a pharmaceutical preparation is strongly desired.

With regard to a compound of benzimidazole skeleton having a phenylethyl side chain on its partial structure, JP-A-3-109378 discloses that certain compounds having actions to inhibit both cyclooxygenase (CO) and lipoxygenase (LO) enzymes are useful in treating or alleviating allergic or inflammatory conditions, but it does not disclose their pharmacological data so that the strength of action of each compound and its detailed action mechanism are not clear (the term "JP-A" as used herein means an "unexamined published Japanese patent application"). Also, JP-A-61-65848 discloses a compound which selectively inhibits 5-lipoxygenase and discloses that it is effective in a rat adjuvant arthritis model and inhibits release of SRS-A in rat passive peritoneal anaphylaxis (PPA). However, these prior art benzimidazole derivatives are different from the compounds of the present invention in terms of their structures, and these patents do not disclose about the effect of these derivatives to inhibit increment or activation of eosinophils.

With regard to a compound of benzimidazole skeleton having a carboxylic acid or ester structure on its side chain, U.S. Patent 5,216,003 discloses a compound which is useful as an NMDA antagonist in neurodegenerative diseases and neurotoxin disorders. Structure of this prior art benzimidazole derivative is also different from that of the compound of the present invention, and the patent does not disclose actions against eosinophils.

In developing pharmaceutical preparations, it is important in general that these medicaments show excellent

results not only in pharmacological tests but also in safety tests such as a subacute toxicological test (for example, a two week drug tolerance test in rats), a chronic toxicological test, a reproductive/developmental toxicological test, a mutagenicity test, an experimental carcinogenicity test, a metabolism test and the like. It is very important to provide a drug having excellent pharmacokinetics such as absence of cytochrome P450-related drug metabolism in the liver, serological or pathological abnormality and the like, namely a drug which has high safety, is effective in a small amount and can be handled easily, but nothing has been disclosed in the prior art concerning a compound which resolves these problems and inhibits increment or activation of eosinophils.

The object of the present invention is to provide compounds or salts thereof which are effective against diseases exhibiting eosinophilia (parasite infection, hypereosinophilic syndrome (HES), eosinophilic pneumonia (PIE syndrome), eosinophilic enterogastritis, bronchial asthma, atopic dermatitis, allergic rhinitis and the like) or various allergic diseases (hay fever, pollinosis, allergic enterogastritis, food allergy, drug allergy and the like), through the regulation of eosinophils, namely inhibition of the increment or activation of eosinophils in blood or tissues, or through the inhibition of IgE antibody production.

Another object of the present invention is to provide a process for producing the same and drugs and pharmaceutical compositions containing the same. A particular object is to provide agents for preventing and/or treating diseases exhibiting eosinophilia, bronchial asthma and allergic diseases, in which at least one of the aforementioned problems involved in the prior art is resolved.

DISCLOSURE OF THE INVENTION

It is known that the number of eosinophils in blood or tissues increases in the case of parasite infection, hypereosinophilic syndrome (HES), eosinophilic pneumonia, eosinophilic enterogastritis, bronchial asthma, atopic dermatitis, allergic rhinitis and the like various diseases in which eosinophils seem to be concerned in the morbid states of these diseases, and increment and activation of eosinophils are closely related to the worsening of such morbid states. In consequence, it is considered that a compound which inhibits increment of eosinophils will be markedly useful in treating diseases in which eosinophils are closely taking part in their morbid states.

Taking the aforementioned situation into consideration, the inventors of the present invention have conducted studies for many years on the screening of compounds capable of strongly inhibiting increment of eosinophils. As a result of such efforts, it was found that a compound having a specified benzimidazole skeleton can inhibit increment of eosinophils strongly and has high safety with less side effects, thereby resulting in the accomplishment of the present invention.

A first aspect of the present invention is a compound represented by the following formula (I)

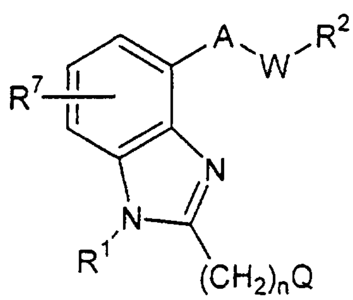

(I)

(wherein $R^1$ represents hydrogen atom or a straight- or branched-chain alkyl group having 1 to 4 carbon atoms, $R^2$ represents cyano group, hydroxymethyl group, 2-(2-imidazolyl)ethenyl group, a phenyl group substituted by one or two $-COOR^3$ groups, or a group $-COOR^3$ or $-CONR^4R^5$, $R^3$ represents hydrogen atom or a straight- or branched-chain alkyl group having 1 to 4 carbon atoms, each of $R^4$ and $R^5$ represents hydrogen atom, an alkyl group having 1 or 2 carbon atoms or a group $-CH_2COOR^6$ or $-CH(CH_2Ph)COOR^6$, wherein $R^4$ and $R^5$ may be the same or different from each other but, when one of $R^4$ and $R^5$ is a group $-CH_2COOR^6$ or $-CH(CH_2Ph)COOR^6$, the other one is hydrogen atom, A represents any one of groups selected from the class consisting of -CO-, $-CH(OR^8)-$, $-CH_2O-$, $-CH(NHR^9)CH_2-$, -CH=CH- and $-CH_2CH_2-$, W represents a group $-CH_2-$ or a single bond, Q represents a phenyl group which may be substituted by one hydroxyl group, n is from 0 to 2, $R^6$ represents a straight- or branched-chain alkyl group having 1 to 4 carbon atoms, $R^7$ represents hydrogen atom, hydroxyl group, a halogen atom or a straight- or branched-chain alkoxyl group having 1 to 4 carbon atoms, $R^8$ represents hydrogen atom or acetyl group and $R^9$ represents hydrogen atom, acetyl group, phenylsulfonyl group or a benzoyl group which may be substituted by one methoxy group) or a salt thereof or a medicament which contains the same as the active ingredient.

The following shows preferred substituent groups or preferred combinations thereof in the compound of the aforementioned formula (I), though the present invention is not restricted thereby. $R^1$ is preferably hydrogen atom. $R^2$ is preferably a phenyl group substituted by one or two -$COOR^3$ groups or a group -$COOR^3$ or -$CONR^4R^5$, more preferably a phenyl group substituted by one or two -$COOR^3$ groups or a group -$COOR^3$. $R^3$ is preferably a straight- or branched-chain alkyl group having 1 to 4 carbon atoms. A is preferably any one of -CO-, -CH($OR^8$)-, -$CH_2O$-. W is preferably a group -$CH_2$-. Q is preferably unsubstituted phenyl group. The symbol n is preferably 1 or 2, more preferably 2. $R^7$ is preferably hydrogen atom, a halogen atom or a straight- or branched-chain alkoxyl group having 1 to 4 carbon atoms.

In a preferred combination of the substituent groups, $R^1$ is hydrogen atom, W is a group -$CH_2$-, A is any one of groups selected from the class consisting of -CO-, -CH($OR^8$)- and -$CH_2O$- and $R^2$ is -$COOR^3$ or a phenyl group substituted by one or two -$COOR^3$ groups, and in a particularly preferred combination, $R^1$ is hydrogen atom, W is a group -$CH_2$- and the combination of A and $R^2$ is respectively a group -CO- or -CH($OR^8$)- and a group -$COOR^3$, or a group -$CH_2O$- and a phenyl group substituted by one or two -$COOR^3$ groups.

A second aspect of the present invention is a process for the production of the compound represented by the aforementioned formula (I) or a salt thereof, which comprises treating a compound represented by the following formula (III)

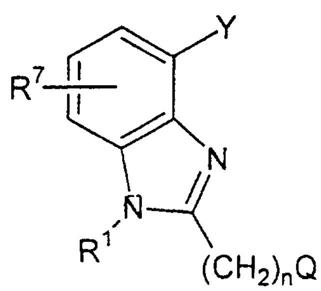

(III)

(wherein Y represents acetyl group, -$COOR^3$, a halogen atom, formyl group, chloroformyl group or bromoformyl group, each of $R^1$ and $R^3$ represents hydrogen atom or a straight- or branched-chain alkyl group having 1 to 4 carbon atoms, $R^7$ represents hydrogen atom, hydroxyl group, a halogen atom or a straight- or branched-chain alkoxyl group having 1 to 4 carbon atoms, Q represents a phenyl group which may be substituted by one hydroxyl group and n is from 0 to 2) or a salt thereof in accordance with any one of steps selected from the group consisting of the following steps (a) to (k), and if necessary, subsequently subjecting the thus obtained compound to a reduction, an oxidation or a substituent change.

(a) The compound is allowed to react with carbon dioxide in the presence of an inorganic base or an organic base and also in the presence, if necessary, of a phase-transfer catalyst, magnesium chloride, sodium iodide or diphenylurea, or with a carbamato complex in an inert solvent, thereby obtaining corresponding carboxylic acid derivatives which, if necessary, is further subjected to esterification under appropriate conditions.
(b) The compound is allowed to react with halogeno-formic acid ester, dialkyl carbonate, phosphonoformic acid ester or oxalic acid ester in the presence of a base, and then subjected to hydrolysis if necessary.
(c) The compound is allowed to react with malonic acid ester in the presence of a base, and then subjected to hydrolysis and subsequent decarboxylation, followed by esterification if necessary.
(d) An acetic acid or an acetic acid ester is prepared into a metal reagent using an appropriate metalating agent, and then the compound is allowed to react with the reagent.
(e) A halogeno-acetic acid derivative is prepared into Reformatsky reagent and then the compound is allowed to react with the reagent.
(f) The compound is allowed to react with Meldrum's acid in the presence of a base to convert it into acyl Meldrum's acid which is then subjected to solvolysis, and decarboxylation, followed by hydrolysis if necessary.
(g) The compound is allowed to react with malonic acid ester, and then subjected to hydrolysis and decarboxylation if necessary.
(h) Using a transition metal complex, the compound is allowed to undergo cross-coupling reaction with an acetylene compound and then hydration reaction is carried out.
(i) The compound is subjected to halogen-metal exchange reaction using an organic lithium reagent, allowed to react with ethylmalonyl chloride and then subjected to hydrolysis and decarboxylation.

(j) The compound is reduced using a metal hydride, allowed to react with substituted benzyl halides in the presence of a base, and then subjected to hydrolysis substituted group if necessary.
(k) The compound is allowed to react with hydrogen cyanide or trimethylsilyl cyanide in the presence of a Lewis acid, and then hydrolyzed, if necessary further carrying out its esterification.

A third aspect of the present invention is a medicament, particularly a pharmaceutical composition, which contains at least one of the compounds represented by the formula (I) or salts thereof as the active ingredient.
A fourth aspect of the present invention is agents for preventing and/or treating diseases exhibiting eosinophilia, which contains a compound represented by the formula (I) or a salt thereof as the active ingredient.
A fifth aspect of the present invention is agents for preventing and/or treating bronchial asthma, which contains a compound represented by the formula (I) or a salt thereof as the active ingredient.
A sixth aspect of the present invention is agents for preventing and/or treating allergic diseases, which contains a compound represented by the formula (I) or a salt thereof as the active ingredient.
In the specification of this invention, the term "diseases exhibiting eosinophilia" means diseases in which eosinophils seem to be concerned in the morbid states of these diseases such as parasite infection, hypereosinophilic syndrome (HES), eosinophilic pneumonia (PIE syndrome), eosinophilic enterogastritis, bronchial asthma, atopic dermatitis, allergic rhinitis, urticaria, hypersensitivity pneumonitis, pulmonary aspergillosis, eosinophilic leukemia and the like.

BRIEF DESCRIPTION OF THE DRAWING

Figs. 1 to 8 are drawings showing chemical structures of the benzimidazole derivatives produced in the Reference Examples and the Examples according to the invention.

BEST MODE OF CARRYING OUT THE INVENTION

The following describes the present invention in detail.
The compound of the present invention is represented by the following formula (I).

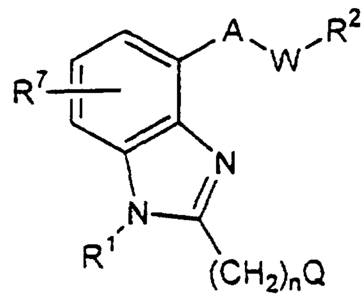

(I)

In the above formula, $R^1$ represents hydrogen atom or a straight- or branched-chain alkyl group having 1 to 4 carbon atoms. More illustratively, the straight- or branched-chain alkyl group having 1 to 4 carbon atoms means methyl group, ethyl group, n-propyl group, isopropyl group, n-butyl group, s-butyl group, isobutyl group, t-butyl group or the like. Preferably, $R^1$ is hydrogen atom or methyl group. More preferably, $R^1$ is hydrogen atom.
$R^2$ represents cyano group, hydroxymethyl group, 2-(2-imidazolyl)ethenyl group, a phenyl group substituted by one or two $-COOR^3$ groups, or a group $-COOR^3$ or $-CONR^4R^5$, $R^3$ represents hydrogen atom or a straight- or branched-chain alkyl group having 1 to 4 carbon atoms, each of $R^4$ and $R^5$ represents hydrogen atom, an alkyl group having 1 or 2 carbon atoms or a group $-CH_2COOR^6$ or $-CH(CH_2Ph)COOR^6$, wherein $R^4$ and $R^5$ may be the same or different from each other with the proviso that, when one of $R^4$ and $R^5$ is a group $-CH_2COOR^6$ or $-CH(CH_2Ph)COOR^6$, the other one is hydrogen atom, and $R^6$ represents a straight- or branched-chain alkyl group having 1 to 4 carbon atoms. More illustratively, the straight- or branched-chain alkyl group having 1 to 4 carbon atoms means methyl group, ethyl group, n-propyl group, isopropyl group, n-butyl group, s-butyl group, isobutyl group, t-butyl group or the like; the alkyl group having 1 or 2 carbon atoms means methyl group or ethyl group. Illustratively, the group $-COOR^3$ is carboxyl group, methoxycarbonyl group, ethoxycarbonyl group, n-propoxycarbonyl group, isopropoxycarbonyl group, n-butoxycarbonyl group, s-butoxycarbonyl group, isobutoxycarbonyl group or t-butoxycarbonyl group; the phenyl group substituted by one or two $-COOR^3$ groups is 2-carboxyphenyl group, 3-carboxyphenyl group, 4-carboxyphenyl group,

2,3-dicarboxyphenyl group, 2,4-dicarboxyphenyl group, 2,5-dicarboxyphenyl group, 2,6-dicarboxyphenyl group, 3,4-dicarboxyphenyl group, 2-methoxycarbonylphenyl group, 3-methoxycarbonylphenyl group, 4-methoxycarbonylphenyl group, 2,3-bis(methoxycarbonyl)phenyl group, 2,4-bis(methoxycarbonyl)phenyl group, 2,5-bis(methoxycarbonyl)phenyl group, 2,6-bis(methoxycarbonyl)phenyl group, 3,4-bis(methoxycarbonyl)phenyl group, 2-ethoxycarbonylphenyl group, 3-ethoxycarbonylphenyl group, 4-ethoxycarbonylphenyl group, 2,3-bis(ethoxycarbonyl)phenyl group, 2,4-bis (ethoxycarbonyl)phenyl group, 2,5-bis(ethoxycarbonyl)phenyl group, 2,6-bis(ethoxycarbonyl)phenyl group, 3,4-bis (ethoxycarbonyl)phenyl group, 2-n-propoxycarbonylphenyl group, 3-n-propoxycarbonylphenyl group, 4-n-propoxycarbonylphenyl group, 2,3-bis(n-propoxycarbonyl)phenyl group, 2,4-bis(n-propoxycarbonyl)phenyl group, 2,5-bis(n-propoxycarbonyl)phenyl group, 2,6-bis(n-propoxycarbonyl)phenyl group, 3,4-bis(n-propoxycarbonyl)phenyl group, 2-isopropoxycarbonylphenyl group, 3-isopropoxycarbonylphenyl group, 4-isopropoxycarbonylphenyl group, 2,3-bis(isopropoxycarbonyl)phenyl group, 2,4-bis(isopropoxycarbonyl)phenyl group, 2,5-bis(isopropoxycarbonyl)phenyl group, 2,6-bis(isopropoxycarbonyl)phenyl group, 3,4-bis(isopropoxycarbonyl)phenyl group, 2-n-butoxycarbonylphenyl group, 3-n-butoxycarbonylphenyl group, 4-n-butoxycarbonylphenyl group, 2,3-bis(n-butoxycarbonyl)phenyl group, 2,4-bis(n-butoxycarbonyl)phenyl group, 2,5-bis(n-butoxycarbonyl)phenyl group, 2,6-bis(n-butoxycarbonyl)phenyl group, 3,4-bis (n-butoxycarbonyl)phenyl group, 2-s-butoxycarbonylphenyl group, 3-s-butoxycarbonylphenyl group, 4-s-butoxycarbonylphenyl group, 2,3-bis(s-butoxycarbonyl)phenyl group, 2,4-bis(s-butoxycarbonyl)phenyl group, 2,5-bis(s-butoxycarbonyl)phenyl group, 2,6-bis(s-butoxycarbonyl)phenyl group, 3,4-bis(s-butoxycarbonyl)phenyl group, 2-isobutoxycarbonylphenyl group, 3-isobutoxycarbonylphenyl group, 4-isobutoxycarbonylphenyl group, 2,3-bis(isobutoxycarbonyl)phenyl group, 2,4-bis(isobutoxycarbonyl)phenyl group, 2,5-bis(isobutoxycarbonyl)phenyl group, 2,6-bis(isobutoxycarbonyl)phenyl group, 3,4-bis(isobutoxycarbonyl)phenyl group, 2-t-butoxycarbonylphenyl group, 3-t-butoxycarbonylphenyl group, 4-t-butoxycarbonylphenyl group, 2,3-bis(t-butoxycarbonyl)phenyl group, 2,4-bis(t-butoxycarbonyl) phenyl group, 2,5-bis(t-butoxycarbonyl)phenyl group, 2,6-bis(t-butoxycarbonyl)phenyl group or 3,4-bis(t-butoxycarbonyl)phenyl group; and the group -CONR$^4$R$^5$ is carbamoyl group, N-methylcarbamoyl group, N-ethylcarbamoyl group, N,N-ethylmethylcarbamoyl group, N,N-diethylcarbamoyl group, N,N-dimethylcarbamoyl group, N-(methoxycarbonylmethyl)carbamoyl group, N-(ethoxycarbonylmethyl)carbamoyl group, N-(propoxycarbonylmethyl)carbamoyl group, N-(isopropoxycarbonylmethyl)carbamoyl group, N-(butoxycarbonylmethyl)carbamoyl group, N-(s-butoxycarbonylmethyl)carbamoyl group, N-(isobutoxycarbonylmethyl)carbamoyl group, N-(t-butoxycarbonylmethyl) carbamoyl group, N-(1-methoxycarbonyl-2-phenylethyl) carbamoyl group, N-(1-ethoxycarbonyl-2-phenylethyl) carbamoyl group, N-(1-propoxycarbonyl-2-phenylethyl)carbamoyl group, N-(1-isopropoxycarbonyl-2-phenylethyl)carbamoyl group, N-(1-butoxycarbonyl-2-phenylethyl)carbamoyl group, N-(1-s-butoxycarbonyl-2-phenylethyl)carbamoyl group, N-(1-isobutoxycarbonyl-2-phenylethyl)carbamoyl group or N-(1-t-butoxycarbonyl-2-phenylethyl)carbamoyl group.

Preferably, R$^2$ is a phenyl group substituted by one or two -COOR$^3$ groups or a group -COOR$^3$ or -CONR$^4$R$^5$, illustratively, the phenyl group substituted by one or two -COOR$^3$ groups is 2-carboxyphenyl group, 3-carboxyphenyl group, 4-carboxyphenyl group, 2,3-dicarboxyphenyl group, 2,4-dicarboxyphenyl group, 2,5-dicarboxyphenyl group, 2,6-dicarboxyphenyl group, 3,4-dicarboxyphenyl group, 2-methoxycarbonylphenyl group, 3-methoxycarbonylphenyl group, 4-methoxycarbonylphenyl group, 2,3-bis(methoxycarbonyl)phenyl group, 2,4-bis(methoxycarbonyl)phenyl group, 2,5-bis(methoxycarbonyl)phenyl group, 2,6-bis(methoxycarbonyl)phenyl group, 3,4-bis(methoxycarbonyl)phenyl group, 2-ethoxycarbonylphenyl group, 3-ethoxycarbonylphenyl group, 4-ethoxycarbonylphenyl group, 2,3-bis (ethoxycarbonyl)phenyl group, 2,4-bis(ethoxycarbonyl)phenyl group, 2,5-bis(ethoxycarbonyl)phenyl group, 2,6-bis (ethoxycarbonyl)phenyl group, 3,4-bis(ethoxycarbonyl)phenyl group, 2-n-propoxycarbonylphenyl group, 3-n-propoxycarbonylphenyl group, 4-n-propoxycarbonylphenyl group, 2,3-bis(n-propoxycarbonyl)phenyl group, 2,4-bis(n-propoxycarbonyl)phenyl group, 2,5-bis(n-propoxycarbonyl)phenyl group, 2,6-bis(n-propoxycarbonyl)phenyl group, 3,4-bis(n-propoxycarbonyl)phenyl group, 2-isopropoxycarbonylphenyl group, 3-isopropoxycarbonylphenyl group, 4-isopropoxycarbonylphenyl group, 2,3-bis(isopropoxycarbonyl)phenyl group, 2,4-bis(isopropoxycarbonyl)phenyl group, 2,5-bis (isopropoxycarbonyl)phenyl group, 2,6-bis(isopropoxycarbonyl)phenyl group, 3,4-bis(isopropoxycarbonyl)phenyl group, 2-n-butoxycarbonylphenyl group, 3-n-butoxycarbonylphenyl group, 4-n-butoxycarbonylphenyl group, 2,3-bis(n-butoxycarbonyl)phenyl group, 2,4-bis(n-butoxycarbonyl)phenyl group, 2,5-bis (n-butoxycarbonyl)phenyl group, 2,6-bis (n-butoxycarbonyl)phenyl group, 3,4-bis(n-butoxycarbonyl)phenyl group, 2-s-butoxycarbonylphenyl group, 3-s-butoxycarbonylphenyl group, 4-s-butoxycarbonylphenyl group, 2,3-bis(s-butoxycarbonyl)phenyl group, 2,4-bis(s-butoxycarbonyl)phenyl group, 2,5-bis(s-butoxycarbonyl)phenyl group, 2,6-bis(s-butoxycarbonyl)phenyl group, 3,4-bis(s-butoxycarbonyl)phenyl group, 2-isobutoxycarbonylphenyl group, 3-isobutoxycarbonylphenyl group, 4-isobutoxycarbonylphenyl group, 2,3-bis(isobutoxycarbonyl)phenyl group, 2,4-bis(isobutoxycarbonyl)phenyl group, 2,5-bis(isobutoxycarbonyl)phenyl group, 2,6-bis(isobutoxycarbonyl)phenyl group, 3,4-bis(isobutoxycarbonyl)phenyl group, 2-t-butoxycarbonylphenyl group, 3-t-butoxycarbonylphenyl group, 4-t-butoxycarbonylphenyl group, 2,3-bis(t-butoxycarbonyl)phenyl group, 2,4-bis(t-butoxycarbonyl)phenyl group, 2,5-bis(t-butoxycarbonyl)phenyl group, 2,6-bis(t-butoxycarbonyl)phenyl group or 3,4-bis(t-butoxycarbonyl)phenyl group; the group -COOR$^3$ is carboxyl group, methoxycarbonyl group, ethoxycarbonyl group, n-propoxycarbonyl group, isopropoxycarbonyl group, n-butoxycarbonyl group, s-butoxycarbonyl group, isobutoxycarbonyl group or t-butoxycarbonyl group; and the group -CONR$^4$R$^5$ is carbamoyl group, N-methylcarbamoyl group, N-ethylcarbamoyl group, N,N-ethylmethylcarbamoyl group, N,N-diethylcarbamoyl group, N,N-dimethylcarbamoyl group, N-(methoxycarbonylmethyl)carbamoyl group, N-(ethoxycarbonylmethyl)carbamoyl group, N-(propoxycarbonylmethyl)carbamoyl group, N-(isopropoxycarbonylmethyl)carbamoyl group, N-(butoxycarbonylmethyl)carbamoyl group, N-(s-butoxycarbonylmethyl)carbamoyl group, N-(isobutoxycarbonylmethyl)carbamoyl group, N-(t-butoxycarbonylmethyl)carbamoyl group, N-(1-methoxycarbonyl-2-phenylethyl)carbamoyl group, N-(1-ethoxycarbonyl-2-phenylethyl)carbamoyl group, N-(1-propoxycarbonyl-2-phenylethyl)carbamoyl group, N-(1-isopropoxycarbonyl-2-phenylethyl) carbamoyl group, N-(1-butoxycarbonyl-2-phenylethyl) carbamoyl group, N-(1-s-butoxycarbonyl-2-phenylethyl)carbamoyl group, N-(1-isobutoxycarbonyl-2-phenylethyl) carbamoyl group or N-(1-t-butoxycarbonyl-2-phenylethyl)carbamoyl group.

More preferably, $R^2$ is a phenyl group substituted by one or two $-COOR^3$ groups or a group $-COOR^3$, illustratively, the phenyl group substituted by one or two $-COOR^3$ groups is 2-carboxyphenyl group, 3-carboxyphenyl group, 4-carboxyphenyl group, 2,3-dicarboxyphenyl group, 2,4-dicarboxyphenyl group, 2,5-dicarboxyphenyl group, 2,6-dicarboxyphenyl group, 3,4-dicarboxyphenyl group, 2-methoxycarbonylphenyl group, 3-methoxycarbonylphenyl group, 4-methoxycarbonylphenyl group, 2,3-bis(methoxycarbonyl)phenyl group, 2,4-bis(methoxycarbonyl)phenyl group, 2,5-bis(methoxycarbonyl)phenyl group, 2,6-bis(methoxycarbonyl)phenyl group, 3,4-bis(methoxycarbonyl)phenyl group, 2-ethoxycarbonylphenyl group, 3-ethoxycarbonylphenyl group, 4-ethoxycarbonylphenyl group, 2,3-bis(ethoxycarbonyl)phenyl group, 2,4-bis(ethoxycarbonyl)phenyl group, 2,5-bis(ethoxycarbonyl)phenyl group, 2,6-bis(ethoxycarbonyl)phenyl group, 3,4-bis(ethoxycarbonyl)phenyl group, 2-n-propoxycarbonylphenyl group, 3-n-propoxycarbonylphenyl group, 4-n-propoxycarbonylphenyl group, 2,3-bis(n-propoxycarbonyl)phenyl group, 2,4-bis(n-propoxycarbonyl) phenyl group, 2,5-bis(n-propoxycarbonyl)phenyl group, 2,6-bis(n-propoxycarbonyl)phenyl group, 3,4-bis(n-propoxycarbonyl)phenyl group, 2-isopropoxycarbonylphenyl group, 3-isopropoxycarbonylphenyl group, 4-isopropoxycarbonylphenyl group, 2,3-bis(isopropoxycarbonyl)phenyl group, 2,4-bis(isopropoxycarbonyl)phenyl group, 2,5-bis(isopropoxycarbonyl)phenyl group, 2,6-bis(isopropoxycarbonyl)phenyl group, 3,4-bis(isopropoxycarbonyl)phenyl group, 2-n-butoxycarbonylphenyl group, 3-n-butoxycarbonylphenyl group, 4-n-butoxycarbonylphenyl group, 2,3-bis(n-butoxycarbonyl)phenyl group, 2,4-bis(n-butoxycarbonyl)phenyl group, 2,5-bis(n-butoxycarbonyl)phenyl group, 2,6-bis(n-butoxycarbonyl)phenyl group, 3,4-bis(n-butoxycarbonyl)phenyl group, 2-s-butoxycarbonylphenyl group, 3-s-butoxycarbonylphenyl group, 4-s-butoxycarbonylphenyl group, 2,3-bis(s-butoxycarbonyl)phenyl group, 2,4-bis(s-butoxycarbonyl)phenyl group, 2,5-bis(s-butoxycarbonyl)phenyl group, 2,6-bis(s-butoxycarbonyl)phenyl group, 3,4-bis(s-butoxycarbonyl) phenyl group, 2-isobutoxycarbonylphenyl group, 3-isobutoxycarbonylphenyl group, 4-isobutoxycarbonylphenyl group, 2,3-bis(isobutoxycarbonyl)phenyl group, 2,4-bis(isobutoxycarbonyl)phenyl group, 2,5-bis(isobutoxycarbonyl)phenyl group, 2,6-bis(isobutoxycarbonyl)phenyl group, 3,4-bis(isobutoxycarbonyl)phenyl group, 2-t-butoxycarbonylphenyl group, 3-t-butoxycarbonylphenyl group, 4-t-butoxycarbonylphenyl group, 2,3-bis(t-butoxycarbonyl)phenyl group, 2,4-bis(t-butoxycarbonyl)phenyl group, 2,5-bis(t-butoxycarbonyl)phenyl group, 2,6-bis(t-butoxycarbonyl)phenyl group or 3,4-bis(t-butoxycarbonyl)phenyl group; and the group $-COOR^3$ is carboxyl group, methoxycarbonyl group, ethoxycarbonyl group, n-propoxycarbonyl group, isopropoxycarbonyl group, n-butoxycarbonyl group, s-butoxycarbonyl group, isobutoxycarbonyl group or t-butoxycarbonyl group.

A represents any one of groups selected from the class consisting of -CO-, $-CH(OR^8)-$, $-CH_2O-$, $-CH(NHR^9)CH_2-$, -CH=CH- and $-CH_2CH_2-$, $R^8$ represents hydrogen atom or acetyl group and $R^9$ represents hydrogen atom, acetyl group, phenylsulfonyl group or a benzoyl group which may be substituted by one methoxy group. More illustratively, $-CH(OR^8)-$ is -CH(OH)- or $-CH(OCOCH_3)-$, and $-CH(NHR^9)CH_2-$ is $-CH(NH_2)CH_2-$, $-CH(NHCOCH_3)CH_2-$, $-CH(NHSO_2Ph)CH_2-$, $-CH(NHCOPh)CH_2-$, $-CH(NHCO(2-OCH_3-C_6H_4)CH_2-$, $-CH(NHCO(3-OCH_3-C_6H_4)CH_2-$ or $-CH(NHCO(4-OCH_3-C_6H_4)CH_2-$.

Preferably, A is -CO-, -CH(OH)-, $-CH(OCOCH_3)-$ or $-CH_2O-$.

W represents a group $-CH_2-$ or a single bond. Preferably, W is a group $-CH_2-$.

Q represents a phenyl group which may be substituted by one hydroxyl group. More illustratively, the phenyl group which may be substituted by one hydroxyl group is unsubstituted phenyl group, 2-hydroxyphenyl group, 3-hydroxyphenyl group or 4-hydroxyphenyl group. Preferably, Q is unsubstituted phenyl group or 4-hydroxyphenyl group. More preferably, Q is unsubstituted phenyl group.

The numeral n is from 0 to 2. Preferably, n is 1 or 2, more preferably 2. In consequence, illustrative examples of $-(CH_2)n-Q$ include unsubstituted phenyl group, 2-hydroxyphenyl group, 3-hydroxyphenyl group, 4-hydroxyphenyl group, unsubstituted benzyl group, 2-hydroxybenzyl group, 3-hydroxybenzyl group, 4-hydroxybenzyl group, unsubstituted phenylethyl group, 2-(2-hydroxyphenyl)ethyl group, 2-(3-hydroxyphenyl)ethyl group and 2-(4-hydroxyphenyl) ethyl group. Preferably, $-(CH_2)n-Q$ is unsubstituted benzyl group, 2-hydroxybenzyl group, 3-hydroxybenzyl group, 4-hydroxybenzyl group, unsubstituted phenylethyl group, 2-(2-hydroxyphenyl)ethyl group, 2-(3-hydroxyphenyl)ethyl group or 2-(4-hydroxyphenyl)ethyl group. More preferably, $-(CH_2)n-Q$ is unsubstituted phenylethyl group, 2-(2-hydroxyphenyl) ethyl group, 2-(3-hydroxyphenyl)ethyl group or 2-(4-hydroxyphenyl)ethyl group, and unsubstituted phenylethyl group is particularly preferred.

$R^7$ represents hydrogen atom, hydroxyl group, a halogen atom or a straight- or branched-chain alkoxyl group

having 1 to 4 carbon atoms. More illustratively, the halogen atom is fluorine atom, chlorine atom, bromine atom or iodine atom, and the straight- or branched-chain alkoxyl group having 1 to 4 carbon atoms is methoxy group, ethoxy group, n-propoxy group, isopropoxy group, n-butoxy group, s-butoxy group, isobutoxy group, t-butoxy group or the like. Preferably, $R^7$ is hydrogen atom, fluorine atom, chlorine atom, bromine atom, iodine atom, methoxy group, ethoxy group, n-propoxy group, isopropoxy group, n-butoxy group, s-butoxy group, isobutoxy group or t-butoxy group. More preferably, $R^7$ is hydrogen atom.

In the aforementioned formula (I), preferred combination of -A-W- is CO-$CH_2$-, -CH(OH)-$CH_2$-, -CH($OCOCH_3$) -$CH_2$- or -$CH_2$O-$CH_2$-.

Preferred combination of -A-W- and $R_2$ is one of the groups CO-$CH_2$-, -CH(OH)-$CH_2$- and -CH($OCOCH_3$) -$CH_2$- and the group -$COOR^3$, or the group -$CH_2$O-$CH_2$- and the phenyl group substituted by one or two -$COOR^3$ groups.

In a preferred combination of the aforementioned substituent groups, $R^1$ is a hydrogen atom, W is group -$CH_2$-, A is any one of groups selected from the class consisting of -CO-, -CH(OR)- and -$CH_2$O-, and $R^2$ is -$COOR^3$ or a phenyl group substituted by one or two -$COOR^3$ groups, and in a particularly preferred combination, $R^1$ is hydrogen atom, W is the group -$CH_2$- and the combination of A and $R^2$ is the group -CO- or -CH($OR^8$)- and -$COOR^3$, or the group -$CH_2$O- and the phenyl group substituted by one or two -$COOR^3$ groups.

Protecting groups of the compound of formula (I) can be introduced optionally during the reaction steps, and said protecting groups can be removed at the final step. Examples of the protecting group of hydroxyl group or carboxyl group include lower alkyl groups such as methyl group, ethyl group, t-butyl group, aralkyl groups such as benzyl group, 4-nitrobenzyl group, substituted silyl groups such as trimethylsilyl group, acyl groups such as acetyl group, benzoyl group, arylsulfonyl groups such as benzenesulfonyl group, tosyl group and methoxymethyl group, tetrahydropyranyl group and the like groups. Examples of the protecting group of NH group on the benzimidazole ring include benzyl group, p-methoxybenzyl group, trityl group, tosyl group, mesyl group, formyl group, chloroacetyl group, t-butoxycarbonyl group and the like. Protection of carbonyl group can be effected for example by converting it into 1,3-dioxolan or 1,3-dithian.

Next, stereoisomers of the compound of the present invention are described.

When $R^1$ in the formula (I) is hydrogen atom (the following formula (I)-e), equilibrium may exist in the benzimidazole ring between this formula and the following formula (I)-g as shown below. The existing ratio of each isomer in the following equilibrium varies depending on the conditions of the compound, such as its solid state or solution in an appropriate solvent.

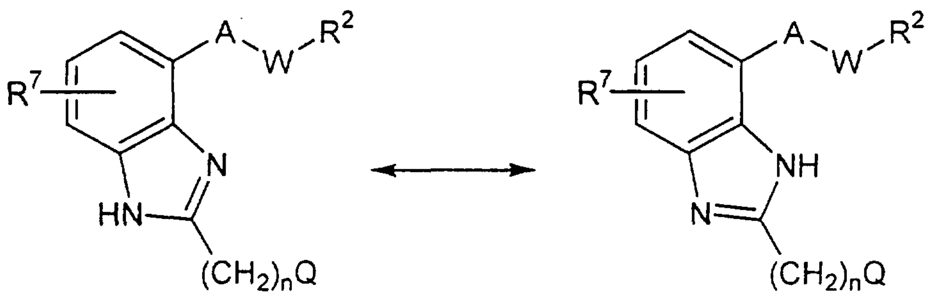

( I )-e ( I )-g

When A in the formula (I) is carbonyl group and W is methylene group (the following formula (I)-h), keto-enol equilibrium may exist between this formula and the following formula (XIV) as shown below. The existing ratio of each isomer in the following equilibrium varies depending on the conditions of the compound, such as its solid state or solution in an appropriate solvent or temperature.

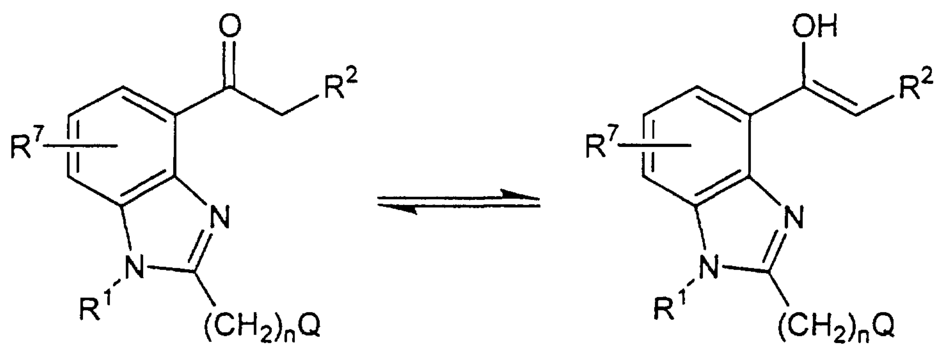

(I)-h (XIV)

When A in the formula (I) is a group $-(CH)(OR^8)-$ or $-CH(NHR^9)CH_2-$, an asymmetric carbon exists so that optical isomers exist. Also, diastereomers exist when A is a group $-(CH)(OR^8)-$ or $-CH(NHR^9)CH2-$ and $R^2$ represents $-CONR^4R^5$ at the same time wherein one of $R^4$ and $R^5$ is a group $-CH(CH_2Ph)COOR^6$ and the other is hydrogen atom.

The present invention includes all of these optically active or inactive stereoisomer forms and equilibrium mixtures, as well as optional mixtures thereof.

The compound of the present invention can form salts with inorganic or organic acids. Examples of such salts include salts with inorganic acids such as hydrochlorides, hydrobromides, phosphates, sulfates, salts with organic acids such as acetate, oxalate, citrate, tartarate, maleate, alginate, p-toluenesulfonate, salicylate and salts with acidic amino acids such as glutamate, aspartate. The compound of the present invention can form salts with inorganic or organic bases depending on the substituent groups. Examples of such salts include alkali metal salts such as sodium salt, potassium salt, alkaline earth metal salts such as magnesium salt, calcium salt, salts with inorganic bases such as ammonium salt, salts with organic bases such as triethylamine salt, pyridine salt and salts with basic amino acids such as arginine salt, lysine salt, histidine salt. In addition, the compound of the present invention or salts thereof can form solvates with water, ethanol, glycerol and the like solvents, and such solvates are also included in the present invention.

The benzimidazole derivatives of the present invention or salts thereof can be produced by the procedures described in the following or by slight modifications thereof. In each of the following formulae, substituent groups of the formula (I) are as defined in the foregoing, and in other formulae, unless otherwise noted, $R^1$ represents hydrogen atom or a straight- or branched-chain alkyl group having 1 to 4 carbon atoms, $R^2$ represents cyano group, hydroxymethyl group, 2-(2-imidazolyl)ethenyl group, a phenyl group substituted by one or two $-COOR^3$ groups, or a group $-COOR^3$ or $-CONR^4R^5$, $R^3$ represents hydrogen atom or a straight- or branched-chain alkyl group having 1 to 4 carbon atoms, each of $R^4$ and $R^5$ represents hydrogen atom, an alkyl group having 1 or 2 carbon atoms or a group $-CH_2COOR^6$ or $-CH(CH_2Ph)COOR^6$, wherein $R^4$ and $R^5$ may be the same or different from each other but, when one of $R^4$ and $R^5$ is a group $-CH_2COOR^6$ or $-CH(CH_2Ph)COOR^6$, the other one is hydrogen atom, A represents any one of groups selected from the class consisting of -CO-, $-CH(OR^8)-$, $-CH_2O-$, $-CH(NHR^9)CH_2-$, -CH=CH- and $-CH_2CH_2-$, W represents a group $-CH_2-$ or a single bond, Q represents a phenyl group which may be substituted by one hydroxyl group, n is from 0 to 2, $R^6$ represents a straight- or branched-chain alkyl group having 1 to 4 carbon atoms, $R^7$ represents hydrogen atom, hydroxyl group, a halogen atom or a straight- or branched-chain alkoxyl group having 1 to 4 carbon atoms, $R^8$ represents hydrogen atom or acetyl group, $R^9$ represents hydrogen atom, acetyl group, phenylsulfonyl group or a benzoyl group which may be substituted by one methoxy group, and Y represents acetyl group, $-COOR^3$, a halogen atom, formyl group, chloroformyl group or bromoformyl group. The halogen atom means fluorine atom, chlorine atom, bromine atom or iodine atom.

Next, the compound of the present invention can be produced by general procedures represented by the following reaction formula. The following describes such methods in detail.

<Reaction formula>

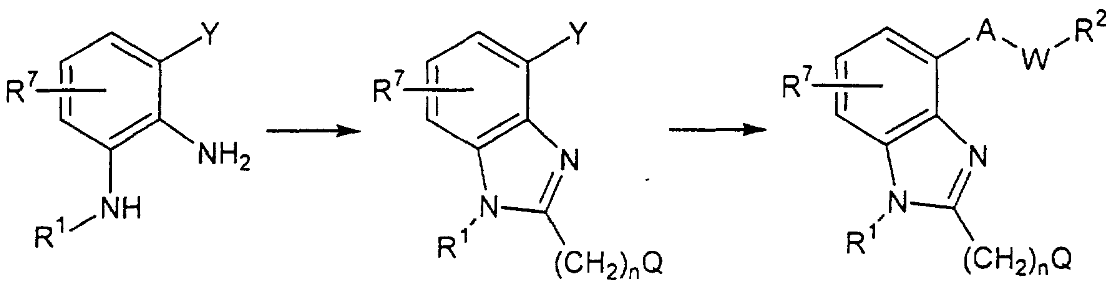

(II) (III) (I)

A compound represented by a formula (I)-b in which A in the formula (I) is carbonyl group, W is methylene group, $R^1$ is hydrogen atom and $R^2$ is $-COOR^3$ can be synthesized in accordance with the methods described for example in "New Experimental Chemistry Courses, Vol. 14, Synthesis and Reaction of Organic Compounds" edited by The Chemical Society of Japan (published by Maruzen), " Experimental Chemistry Courses 4th Edition, Vol. 22, Organic Synthesis IV" edited by The Chemical Society of Japan (published by Maruzen) or "Comprehensive Organic Transformations" edited by R.C. Larock (published by VCH, 1989).

<Procedure A>

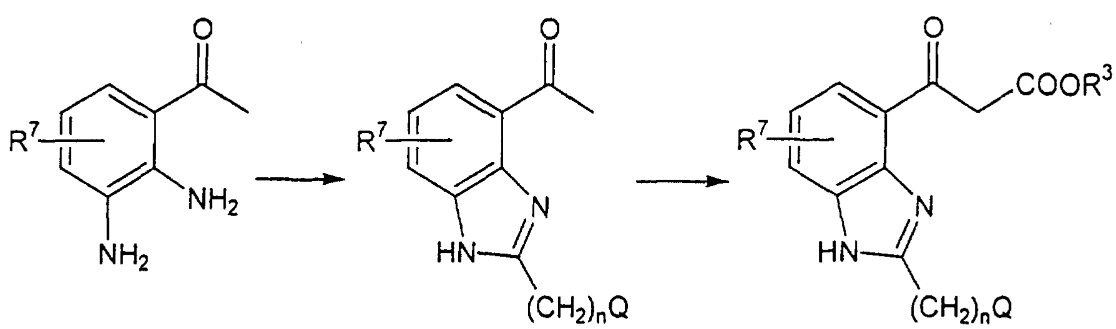

(II)-b (III)-b (I)-b

For example, a compound of the formula (I)-b in which $R^3$ is hydrogen atom (a carboxylic acid derivative) can be obtained by allowing a benzimidazole derivative represented the formula (III)-b, which is obtained from a 2,3-diaminoacetophenone derivative as a compound of the formula (II)-b, to react with carbon dioxide in a reaction inert solvent such as a halogenated hydrocarbon solvent (chloroform, dichloromethane or the like for example), an ether solvent (diethyl ether, tetrahydrofuran (THF) or the like for example) or acetonitrile, dimethyl sulfoxide (DMSO), N,N-dimethylformamide (DMF) or the like polar solvent, preferably in DMSO, in the presence of potassium carbonate, sodium carbonate, calcium carbonate, sodium hydride, sodium amide or the like inorganic base or triethylamine, 1,8-diazabicyclo[5.4.0]-7-undecene or the like organic base, preferably in the presence of potassium carbonate, if necessary by adding a phase-transfer catalyst such as 18-crown-6-ether or the like crown ether compound, or magnesium chloride, sodium iodide or diphenylurea, at a temperature of from -78°C to reflux temperature of the solvent used, preferably from 0°C to 30°C. The carboxylic acid derivative can also be obtained by allowing a carbamato complex, which is

obtained for example by a combination of 2-imidazolidinthione, ethylmagnesium bromide and carbon dioxide, to react with the compound (III)-b in DMF or the like reaction inert solvent. The carboxylic acid derivative can also be converted into a compound of the formula (I)-b in which $R^3$ is a straight- or branched-chain alkyl group having 1 to 4 carbon atoms (an ester derivative), using diazomethane, trimethylsilyldiazomethane, diethyl sulfate or the like dialkyl sulfate or methyl iodide or the like alkyl halide as an alkylating agent. It can also be converted into the ester derivative by dehydrated and condensed reaction at a temperature of from -10°C to reflux temperature of the solvent used, preferably from 0°C to 30°C, using dicyclohexylcarbodiimide (DCC), 1,1'-carbonyldiimidazole (CDI), 2-chloro-1,3-dimethylimidazolinium hexafluorophosphate (CIP) or the like condensing agent, preferably using CIP, using an appropriate alcohol (methanol, ethanol, t-butanol or the like for example), a halogenated hydrocarbon solvent (chloroform, dichloromethane or the like for example), an aromatic hydrocarbon solvent (toluene or the like for example), an ether solvent (diethyl ether, THF or the like for example) or the like reaction inert solvent, preferably using dichloromethane or the like halogenated hydrocarbon solvent, and using potassium carbonate or the like inorganic base or pyridine, triethylamine or the like organic base, preferably using triethylamine or the like organic base. In addition, the ester derivative can also be synthesized using methanol, ethanol or the like appropriate alcohol in the presence of a catalyst such as sulfuric acid or the like mineral acid or boron trifluoride etherate ($BF_3 \cdot Et_2O$) or the like Lewis acid at a temperature of from 0°C to reflux temperature of the solvent used, preferably with heating under reflux. Ester interchange of the thus obtained ester derivative can be effected by allowing it to react with an appropriate alcohol in the presence of the aforementioned mineral acid, Lewis acid or the like catalyst, and a desired ester can also be obtained by allowing the ester derivative to react with alkoxide of an appropriate alcohol.

In the aforementioned method, a compound represented by the formula (I)-b in which an alkoxycarbonyl group is introduced into the acetyl group and $R^3$ is a straight- or branched-chain alkyl group having 1 to 4 carbon atoms (an ester derivative) can be obtained by allowing a halogeno-formic acid ester (ethyl chloroformate or the like for example), a carbonic acid diester (diethyl carbonate or the like for example), a phosphonoformic acid ester (ethyl phosphonoformate for example) or a oxalic acid ester (ethyl oxalate for example) and the like esters, in stead of carbon dioxide, to react with a compound represented by the formula (III)-b. A compound in which $R^3$ in the formula (I)-b is hydrogen atom (a carboxylic acid derivative) can be obtained for example by carrying out hydrolysis of these compounds (ester derivatives) at a temperature of from -78°C to reflux temperature of the solvent used, preferably from 0°C to reflux temperature of the solvent used, using an alkaline aqueous solution such as of sodium hydroxide, potassium hydroxide, sodium carbonate or the like, preferably using sodium hydroxide aqueous solution, and using an alcohol solvent (methanol, ethanol or the like for example) or an ether solvent (THF, dioxane or the like for example). (Procedure A)

The compound represented by the formula (III)-b to be used in the procedure A can be obtained by the method shown in the following procedure A-1.

<Procedure A-1>

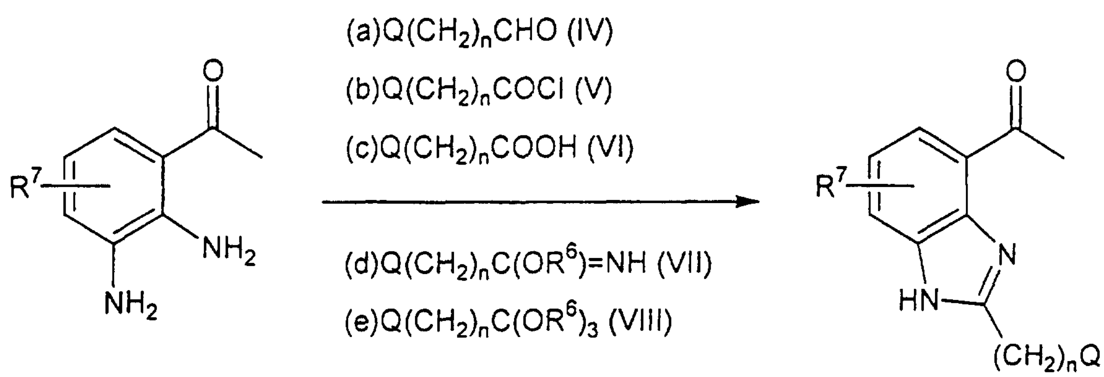


(II)-b (III)-b

That is, it can be obtained by allowing a compound represented by the formula (II)-b to react with a compound represented by the following formula (IV)

$$Q(CH_2)_n\text{-}CHO \quad (IV)$$

(wherein Q and n are as defined in the aforementioned formula (I)),
a compound represented by the following formula (V)

$$Q(CH_2)_n\text{-}COCl \quad (V)$$

(wherein Q and n are as defined in the aforementioned formula (I)),
a compound represented by the following formula (VI)

$$Q(CH_2)_n\text{-}COOH \quad (VI)$$

(wherein Q and n are as defined in the aforementioned formula (I)),
a compound represented by the following formula (VII)

$$Q(CH_2)_n\text{-}C(OR^6)=NH \quad (VII)$$

(wherein Q and n are as defined in the aforementioned formula (I), and $R^6$ is a straight- or branched-chain alkyl group having 1 to 4 carbon atoms), or
a compound represented by the following formula (VIII)

$$Q(CH_2)_n\text{-}C(OR^6)_3 \quad (VIII)$$

(wherein $R^6$, Q and n are as defined in the aforementioned formula (I)), under appropriate conditions. These reactions can be carried out in accordance with the methods disclosed in JP-A-3-27382 and WO 93/22313.

<Procedure B>

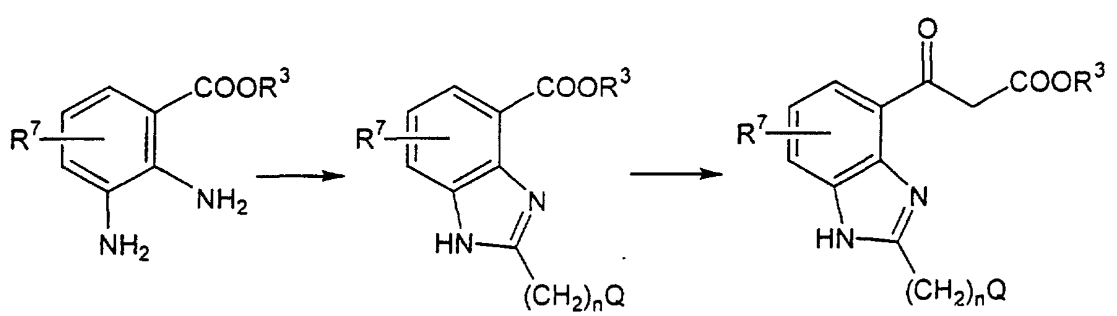

(IX) (X) (I)-b

A compound represented by the formula (I)-b can also be synthesized using a benzimidazolylcarboxylic acid derivative represented by the formula (X) as the starting material which is obtained from a 2,3-diaminobenzoic acid derivative represented by the formula (IX). For example, a compound represented by the formula (I)-b can be synthesized by allowing a benzimidazolylcarboxylic acid ester derivative represented by the formula (X) to react with a malonic acid diester (diethyl malonate for example) or a malonic acid monoester (monoethyl malonate) in a reaction inert solvent such as an alcohol solvent (methanol, ethanol or the like for example), an ether solvent (diethyl ether, THF or the like for example) or DMSO, DMF or the like polar solvent, in the presence of sodium hydride, sodium alkoxide or the like

base, subsequently hydrolyzing the ester to obtain a dicarboxylic acid monoester which is then subjected to decarboxylation. Alternatively, a compound in which $R^3$ in the formula (I)-b is hydrogen atom or a straight- or branched- chain alkyl group having 1 to 4 carbon atoms can be synthesized by preparing acetic acid or an acetic acid ester into a metal reagent using appropriate metalating agent such as butyl lithium and then allowing the reagent to react with a compound represented by the formula (X) in a reaction inert solvent such as an ether solvent (diethyl ether, THF or the like for example), an aromatic hydrocarbon solvent (benzene, toluene or the like for example) or DMSO, DMF or the like polar solvent, or by preparing a halogeno-acetic acid derivative (for example, ethyl chloroacetate, ethyl bromoacetate or ethyl iodoacetate) into Reformatsky reagent and then allowing the reagent to react with a compound represented by the formula (X). Also, a compound in which $R^3$ in the formula (I)-b is a straight or branched chain alkyl group having 1 to 4 carbon atoms (an ester derivative) can be obtained by allowing an acyl halide or an active amide (for example, an acylimidazole derivative or the like), which is obtained from a compound of the formula (IX) in which $R^3$ is hydrogen atom, to react with Meldrum's acid in a halogenated hydrocarbon solvent (chloroform, dichloromethane or the like for example) in the presence of pyridine, triethylamine or the like base, thereby converting it into acyl Meldrum's acid which is then subjected to solvolysis using an appropriate alcohol and subsequent decarboxylation. Also, the just described acyl halide or active amide can be converted into the ester derivative by allowing it to react with magnesium salt of a malonic acid monoester, magnesium salt of ethyl acetoacetate or lithium salt of ethyl acetate in a reaction inert solvent such as an ether solvent (diethyl ether, THF or the like for example), an aromatic hydrocarbon solvent (benzene, toluene or the like for example) or acetonitrile, if necessary in the presence of triethylamine or the like base, subsequently carrying out hydrolysis, decarboxylation by heating or deacetylation as occasion demands. In addition, a compound represented by the formula (I)-b can also be synthesized by allowing the aforementioned acyl halide or active amide to react with a malonic acid diester (diethyl malonate for example) or a malonic acid monoester (monoethyl malonate) in a reaction inert solvent such as an ether solvent (diethyl ether, THF or the like for example) or DMSO, DMF or the like polar solvent, in the presence of sodium hydride, sodium alkoxide or the like base, subsequently hydrolyzing the ester as occasion demands to obtain a dicarboxylic acid monoester which is then subjected to decarboxylation. (Procedure B)

<Procedure C>

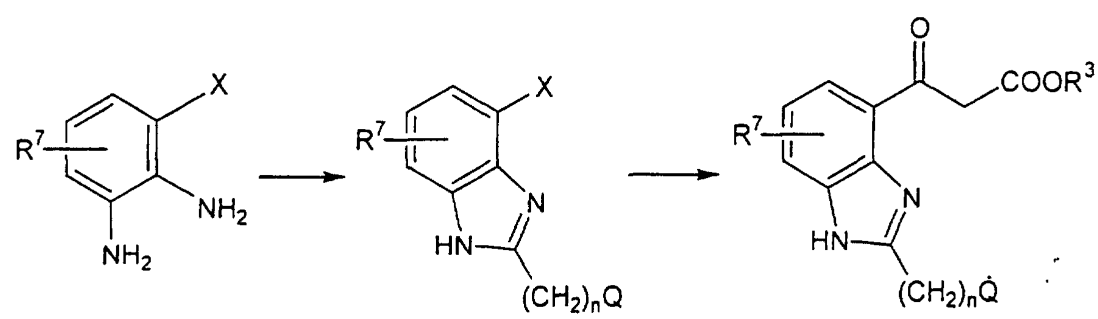

(XI) (XII) (I)-b

In addition, a compound in which $R^3$ in the formula (I)-b is a straight or branched chain alkyl group having 1 to 4 carbon atoms (an ester derivative) can be synthesized by allowing a halogeno-benzimidazole compound represented by the formula (XII) (X means a halogen atom), which can be obtained easily from a 2,3-diaminohalogenobenzene derivative represented by the formula (XI), directly as the compound (XII) or after preparing the compound (XII) into an organic metal reagent such as a boron reagent or tin reagent as occasion demands, to react with ethyl propionate or the like acetylene compound in an reaction inert solvent using a transition metal complex such as palladium acetate or the like palladium complex or tetrakis(triphenylphosphine)nickel ($Ni(PPh_3)_4$) or the like nickel complex, thereby effecting their cross-coupling reaction to introduce an acetylene side chain and then carrying out hydration reaction of the acetylene group, for example using mercury oxide, or by preparing a compound represented by the formula (XII) into an organic metal reagent through a halogen-metal exchange reaction of its halogen atom using butyl lithium or the like organic lithium reagent and then allowing the resulting product to react with ethyl malonate chloride, subsequently carrying out its hydrolysis and decarboxylation in accordance with the aforementioned method. The ester derivative can be hydrolyzed in the usual way. (Procedure C)

A compound represented by a formula (I)-c in which A in the formula (I) is -CH(OH)-, W is methylene group, $R^1$ is hydrogen atom and $R^2$ is $-COOR^3$ ($R^3$ is as defined in the foregoing) can be synthesized in accordance with the methods described for example in "Shin Jikken Kagaku Koza, Vol. 14, Synthesis and Reaction of Organic Compounds" edited by The Chemical Society of Japan (published by Maruzen), " Experimental Chemistry Courses 4th Edition, Vol. 22, Organic Synthesis II" edited by The Chemical Society of Japan (published by Maruzen) or "Comprehensive Organic Transformations" edited by R.C. Larock (published by VCH, 1989). For example, it can be synthesized by the following procedure.

<Procedure D>

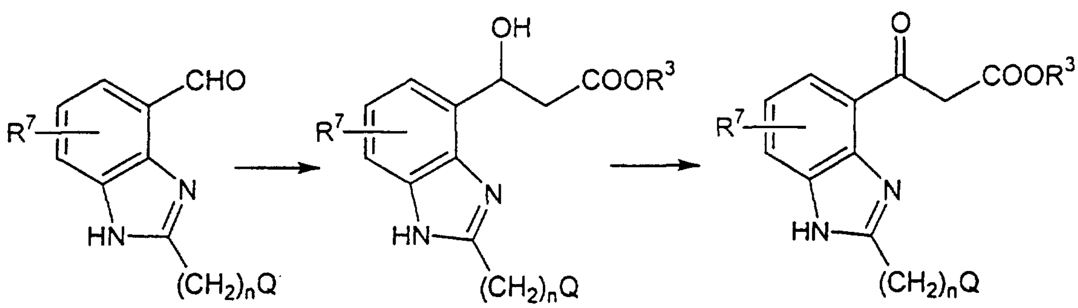

(XIII) (I)-c (I)-b

A compound represented by the formula (XIII) which is obtained from a 2,3-diaminobenzaldehyde derivative (as occasion demands, the formyl group may be protected with a protecting group described in "Protective Groups in Organic Synthesis 2nd Edition" edited by T.W. Greene and P.G.M. Wutz, published by John Wiley and Sons, 1991, or in "Protecting Groups" edited by P.J. Kocienski, published by Georg Thieme Verlag, 1994, during the reaction and then deprotected after completion of the reaction) can be converted into the compound (I)-c using the same reaction conditions and reagents described in the procedure B. That is, the compound of formula (I)-c can be obtained by preparing acetic acid or an acetic acid ester into a metal reagent--using butyl lithium or the like appropriate metalation agent and then allowing the reagent to react with the compound of formula (XIII) in a reaction inert solvent such as an ether solvent (diethyl ether, THF or the like for example), an aromatic hydrocarbon solvent (benzene, toluene or the like for example) or DMSO, DMF or the like polar solvent. The compound of formula (I)-c can also be obtained by the use of a malonic acid ester derivative or Meldrum's acid as described in the procedure B. The thus obtained compound represented by the formula (I)-c can be converted into the compound of formula (I)-b using chromium oxide, potassium peroxide, manganese dioxide or the like metallic oxidizing agent or pyridinium chlorochromate, pyridinium dichromate or the like organic oxidizing agent or by Swern oxidation. (Procedure D)

<Procedure E>

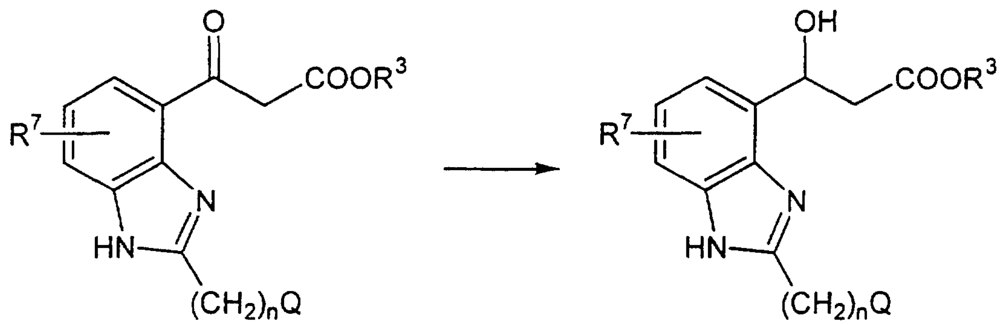

( I )-b ( I )-c

The compound represented by the formula (I)-b obtained by the procedure A, B, C or D can be converted into the compound of formula (I)-c by carrying out its reaction with sodium borohydride ($NaBH_4$), lithium aluminum hydride ($LiAlH_4$), borane ($BH_3$), alane ($AlH_3$) or the like metal hydride or a reducing reagent prepared therefrom by partial or entire appropriate substitution, preferably sodium borohydride, in methanol or ethanol at a temperature of from -50°C to reflux temperature of the solvent used, preferably from 0°C to 30°C. It can also be converted into a compound represented by the formula (I)-c in which $R^3$ is a straight- or branched-chain alkyl group having 1 to 4 carbon atoms (an ester derivative) for example by its hydrogenation through catalytic reduction using palladium/carbon or the like catalyst. The thus obtained ester derivative (I)-c can be converted into a carboxylic acid derivative by its hydrolysis in accordance with the aforementioned method.

(Procedure E)

The compound (I)-c may exist in optical isomer forms, and each of the optically active substances can be obtained by the methods described for example in "Asymmetric Synthesis and Advance in Optical Resolution" (edited by Otsuka and Mukaiyama, 1982, Kagaku Zokan 97, Kagaku Dojin Shuppan) or "High Selectivity Reaction" (edited by Nozaki, Mukaiyama and Noyori, 1981, Kagaku Zokan 91, Kagaku Dojin Shuppan). An example of asymmetric reduction is described in the following procedure F, and an example of optical resolution is described in procedure G.

<Procedure F>

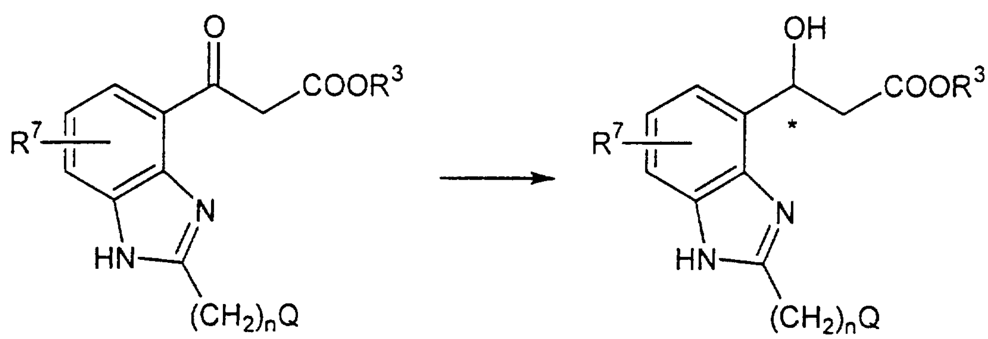

( I )-b ( I )-d

An optically active compound represented by the formula (I)-d can be obtained by allowing a β-ketocarboxylic acid derivative represented by the formula (I)-b to react with an appropriate reducing reagent shown below. In a first method, the compound is allowed to react with an asymmetric reducing agent (for example, BINAL-H, DIP-Cl or the like) prepared by modifying lithium aluminum hydride ($LiAlH_4$), sodium borohydride ($NaBH_4$), borane ($BH_3$) or the like metal hydride reducing agent partially or entirely with an asymmetric substituent group in a reaction inert solvent such as an ether solvent (diethyl ether, THF or the like for example) at a temperature of from -100°C to reflux temperature of the solvent used. In a second method, catalytic asymmetric hydrogenation is carried out using a complex catalyst of ruthenium, rhodium or the like transition metal having an optically active phosphine as a ligand, typically BINAP-Ru $(OCOMe)_2$, in a reaction inert solvent such as an ether solvent (diethyl ether, THF or the like) at a temperature of from -100°C to reflux temperature of the solvent used. In a third method, asymmetric reduction is carried out using an enzyme which catalyzes asymmetric reduction, or a microorganism (baker's yeast for example) which contains said enzyme, as the asymmetric catalyst. (Procedure F)

<Procedure G>

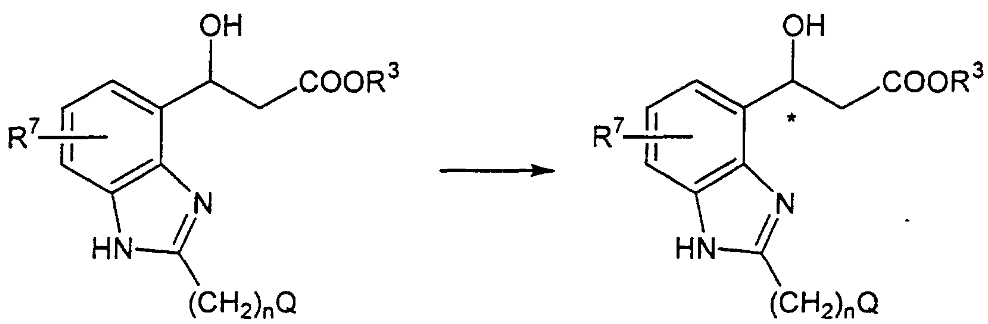

( I )-c ( I )-d

An optically active compound represented by the formula (I)-d can be obtained by carrying out optical resolution of the compound of formula (I)-c obtained by the procedure D or E.

Diastereomers are derived from the racemic compound (derivation by covalent bond) using a reagent having asymmetric property (camphanic chloride, menthoxyacetyl chloride or the like for example), or diastereomer salts are formed (ionic bonding compound) by adding an acid or base having appropriate asymmetric property to the racemic compound, and then their separation is carried out based on the difference in their solubility and the like physical properties or by a chromatography and the like means. Thereafter, an optically active pure compound represented by the formula (I)-d can be obtained by removing the optically active modification group through chemical conversion and dissociation of the salt from the thus separated derivative.

As a direct method, an optically active compound represented by the formula (I)-d can also be separated from a compound represented by the formula (I)-c, for example, by a high performance liquid chromatography (HPLC) using ChiralPack AD™, ChiralCell OD™ (both manufactured by Daicel Chemical Industries) or the like appropriate optically active column.

(Procedure G)

Compounds in which $R^1$ in the formula (I) is a straight- or branched-chain alkyl group having 1 to 4 carbon atoms can be obtained by carrying out the reactions described in the procedures A to G, using a compound in which $R^1$ in the formula (II) is a straight- or branched-chain alkyl group having 1 to 4 carbon atoms and Y is acetyl group, $-COOR^3$ ($R^3$ is as defined in the foregoing), a halogen atom or formyl group, such as 2-amino-3-N-alkylaminoacetophenone, 2-amino-3-N-alkylaminobenzoic acid ester, 2-amino-3-N-alkylaminohalogenobenzene or 2-amino-3-N-alkylaminobenzaldehyde, in stead of the 2,3-diaminoacetophenone derivative, 2,3-diaminobenzoic acid ester derivative, 2,3-diaminohalogenobenzene derivative or 2,3-diaminobenzaldehyde derivative used as the material in the procedures A to G.

In consequence, compounds of the present invention can be produced by the procedure shown by the following reaction formula.

<Reaction formula>

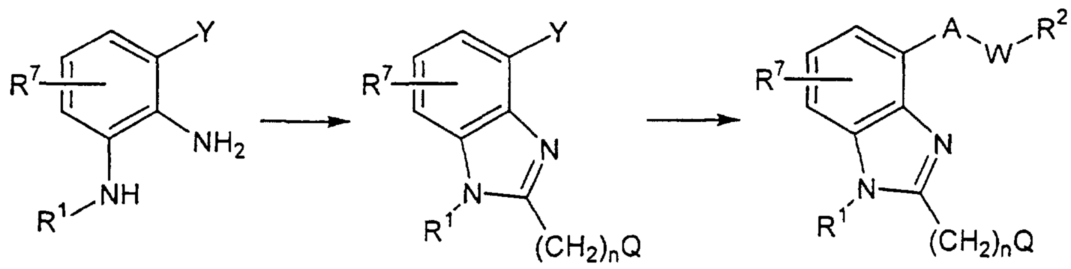

(II) (III) (I)

Compounds represented by the formula (I) can also be synthesized by the following methods.

<Procedure H>

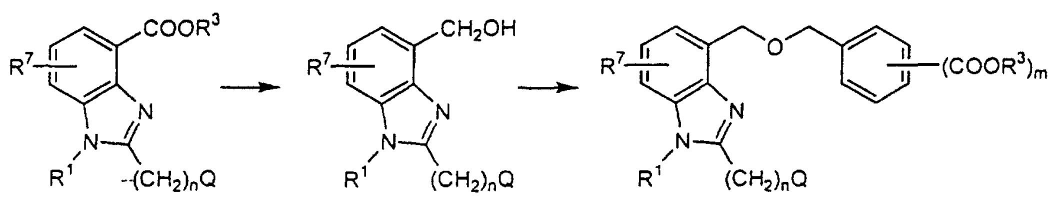

(X)-b (XV) (I)-j

A compound represented by the formula (X)-b can be converted into a compound represented by the formula (XV) by carrying out its reduction in a solvent such as an ether solvent (diethyl ether, THF or the like for example) using lithium aluminum hydride ($LiAlH_4$), borane ($BH_3$), alane ($AlH_3$) or the like metal hydride or a reducing agent prepared therefrom by partial or entire appropriate substitution, preferably lithium aluminum hydride.

A compound represented by the formula (I)-j in which A is a group $-CH_2O-$ and W is a group $-CH_2-$ (m is 1 or 2, and m means the number of substituent groups) can be obtained by allowing the compound of formula (XV) to react with a benzyl halide mono- or di-substituted by carboxyl group which may be protected, in an reaction inert solvent such as an ether solvent (diethyl ether, THF or the like for example) in the presence of sodium hydride or the like base at a temperature of from -100°C to reflux temperature of the solvent used, preferably from -20°C to room temperature. The thus obtained compound may be subjected to hydrolysis and the like treatment as occasion demands.

(Procedure H)

<Procedure J>

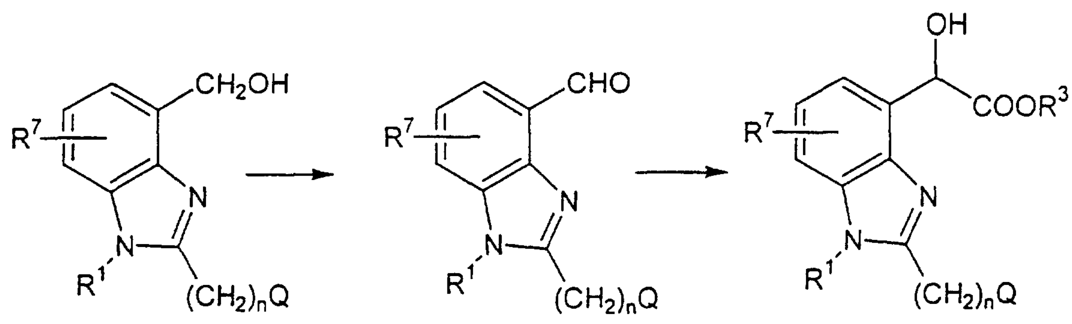

(XV) (XIII)-b ( I )-k

A compound represented by the formula (XIII)-b can be obtained by a method similar to the procedure D or by oxidizing the compound of formula (XV) using activated manganese dioxide, chromium trioxide or the like oxidizing agent in a reaction inert solvent such as an ether solvent (diethyl ether, THF or the like for example), a halogenated hydrocarbon solvent (chloroform, dichloromethane or the like for example), ethyl acetate or acetone.

A compound represented by the formula (I)-k can be obtained by allowing the compound of formula (XIII)-b to undergo the reaction in a halogenated hydrocarbon solvent (chloroform, dichloromethane or the like for example) in the presence of hydrogen cyanide or trimethylsilyl cyanide and zinc(II) iodide, cerium chloride or the like Lewis acid, thereby obtaining a cyanohydrin derivative which is subsequently hydrolyzed with hydrochloric acid or the like. The thus obtained carboxylic acid derivative may be subjected to its esterification in accordance with a method described in the procedure A.

(Procedure J)

<Procedure K>

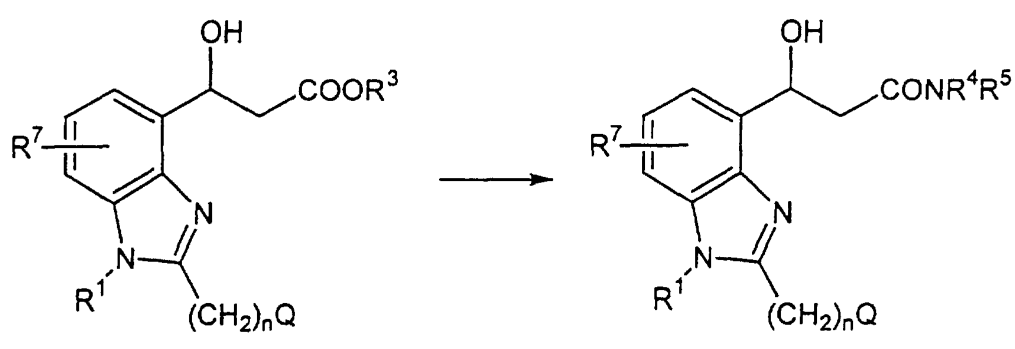

( I )-m ( I )-n

A compound represented by the formula (I)-n can be obtained by allowing a compound represented by the formula (I)-m to react with ammonia, a primary amine or a secondary amine using water, an alcohol solvent (methanol,

ethanol or the like for example) or the like as the solvent.

The compound of formula (I)-n can also be obtained by allowing a carboxylic acid of the formula (I)-m in which $R^3$ is H to react with a primary amine, a secondary amine, an amino acid or the like amine in DMF or the like reaction inert solvent using dicyclohexylcarbodiimide (DCC), benzotriazol-1-yloxytris(dimethylamino)phosphonium hexafluorophosphate (BOP) or the like condensing agent. (Procedure K)

<Procedure L>

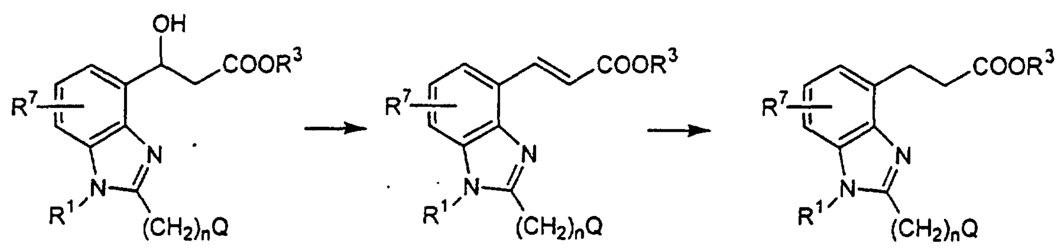

(I)-m (I)-p (I)-q

A compound represented by the formula (I)-p can be obtained by converting the compound of formula (I)-m into a halogeno compound by the use of a halogenating reagent such as thionyl chloride or phosphorus oxychloride or a corresponding bromide in a halogenated hydrocarbon solvent (dichloromethane, chloroform or the like for example) or the like reaction inert solvent, pyridine, triethylamine or the like basic solvent or a mixed solvent thereof, or into a corresponding sulfonyloxy compound using mesyl chloride, tosyl chloride or the like sulfonylating reagent, and then allowing the thus obtained compound to react with 1,8-diazabicyclo[5.4.0]-7-undecene (DBU) or the like base. The thus obtained compound represented by the formula (I)-p can be converted into a compound represented by the formula (I)-q by carrying out its hydrogenation under the same conditions of the procedure P which will be described later.

(Procedure L)

<Procedure M>

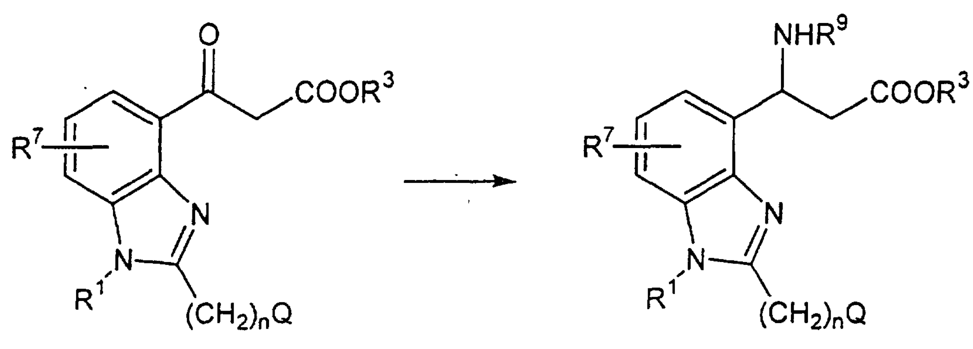

(I)-r (I)-s

A compound represented by the formula (I)-s in which $R^9$ is H (an amino compound) can be obtained by converting a compound represented by the formula (I)-r into an oxime derivative by allowing it to react with hydroxylamine, O-benzylhydroxylamine or the like, if necessary in the presence of sodium hydroxide, triethylamine or the like

base, in an alcohol solvent (methanol or ethanol for example) or the like reaction inert solvent, pyridine or the like basic solvent or a mixed solvent thereof, and then carrying out hydrogenation in an alcohol solvent (methanol, ethanol or the like for example), ethyl acetate or the like reaction inert solvent in the presence of palladium/carbon or the like catalyst. The compound of formula (I)-s can also be synthesized by effecting alkylation, acylation or sulfonylation of the thus obtained amino compound using an alkyl halide or the like alkylation reagent, acetyl chloride or the like acylating reagent or tosyl chloride or the like sulfonylating reagent in a reaction inert solvent such as a halogenated hydrocarbon solvent (chloroform, dichloromethane or the like for example) in the presence of pyridine or the like base.

Also, the compound of formula (I)-s can be synthesized by allowing the compound of formula (I)-r to form an imine with ammonium acetate or the like ammonium salt in a reaction inert solvent such as an alcohol solvent (methanol, ethanol or the like for example), and reducing the imine using sodium cyanoborohydride, sodium triacetoxyborohydride or the like reducing agent.

In addition, the compound of formula (I)-s can also be obtained by allowing the compound (I)-p obtained in the aforementioned procedure L to undergo addition reaction of an amine or addition of hydroxylamine and reduction. (Procedure M)

<Procedure N>

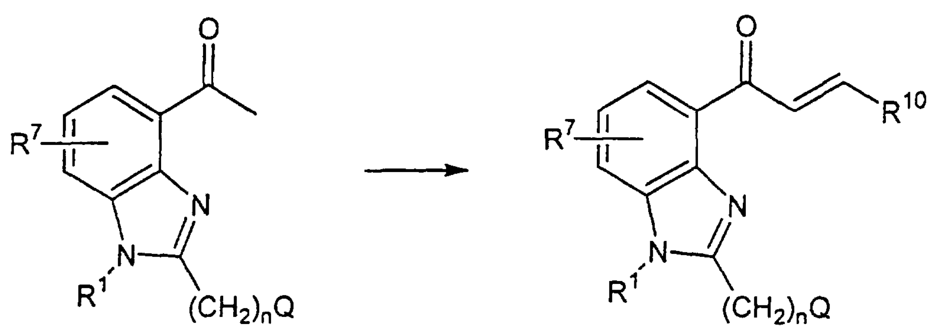

(III)-c (I)-t

A compound represented by the formula (I)-t can be obtained by allowing a compound represented by the formula (III)-c to react with an aldehyde ($R^{10}CHO$ ($R^{10}$ represents 2-imidazolyl group)) in the presence of sodium hydroxide or the like base in water or an alcohol solvent (for example, methanol or ethanol) or the like reaction inert solvent at a temperature of from 0°C to reflux temperature of the solvent used. (Procedure N)

<Procedure P>

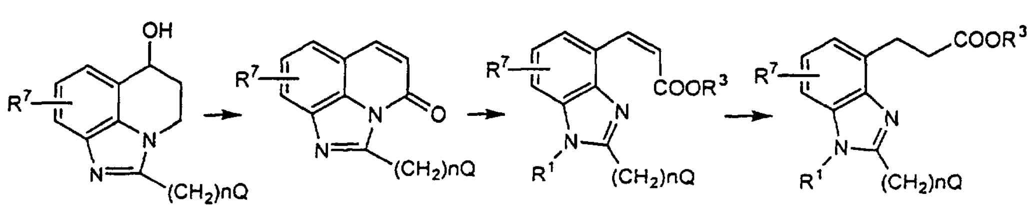

(XVI) (XVII) (I)-u (I)-q

A compound represented by the formula (XVI) can be converted into a compound represented by the formula (I)-u by preparing it into a halogeno compound or a sulfonyloxy compound in accordance with a method described in

the procedure L, forming a double bond through the elimination of a hydrogen halide or sulfonic acid by carrying out the reaction in DMF or the like reaction inert solvent at a temperature of from room temperature to reflux temperature of the solvent used in the presence of potassium carbonate, triethylamine, DBU or the like base as occasion demands and then allowing the resulting compound to react with potassium permanganate or the like oxidizing agent in a halogenated hydrocarbon solvent (chloroform or dichloromethane for example) or the like reaction inert solvent in the presence of butyltriethylammonium chloride or the like catalyst to obtain a compound represented by the formula (XVII) which is subsequently hydrolyzed. A compound represented by the formula (I)-q can be obtained by carrying out hydrogenation of the thus obtained compound in an alcohol solvent (methanol or ethanol for example) or the like reaction inert solvent in the presence of palladium/carbon or the like catalyst. The compound (I)-q can also be obtained by carrying out hydrogenation of the compound (I)-p described in the procedure L. (Procedure P)

<Procedure Q>

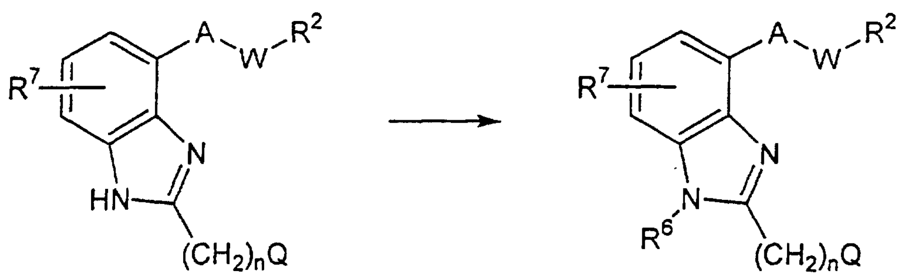

(I)-e (I)-f

A compound represented by the formula (I)-f in which $R^1$ in the formula (I) is a straight or branched-chain alkyl group having 1 to 4 carbon atoms can be obtained by carrying out N-alkylation of a compound represented by the formula (I)-e in which $R^1$ is hydrogen atom. More illustratively, as shown in the above reaction formula, the compound represented by the formula (I)-f in which $R^1$ in the formula (I) is a straight or branched-chain alkyl group having 1 to 4 carbon atoms can be obtained by allowing the compound of formula (I)-e to react with dimethyl sulfate, methyl iodide or the like alkylating agent in a reaction inert solvent such as a halogenated hydrocarbon solvent (chloroform or dichloromethane for example), an ether solvent (diethyl ether or THF for example) or acetone, DMSO, DMF or the like polar solvent in the presence of potassium carbonate, sodium carbonate, sodium bicarbonate or the like inorganic base or pyridine, triethylamine or the like organic base, preferably by allowing it to react with dimethyl sulfate in acetone solvent in the presence of sodium bicarbonate. (Procedure (Q)

When $R^1$ is H and NH group is present on the benzimidazole ring or when hydroxyl group, carboxyl group, carbonyl group or the like reactive group is present as a substituent group in the procedures A to Q of the present invention, these groups can be protected optionally and said protecting group can be removed at the final step or at a necessary step in each procedure. Methods for the introduction and removal of these protecting groups are optionally selected depending on the types of the groups to be protected or the protecting groups and can be carried out for example by the methods described in "Protective Groups in Organic Synthesis 2nd Edition" edited by T.W. Greene and P.G.M. Wutz, published by John Wiley and Sons (1991) or in "Protecting Groups" edited by P.J. Kocienski, published by Georg Thieme Verlag, 1994.

For example, various protecting groups can be cited as the protecting group of hydroxyl group or carboxyl group, such as methyl group, ethyl group, t-butyl group and the like lower alkyl groups, benzyl group, 4-nitrobenzyl group and the like aralkyl groups, trimethylsilyl group and the like substituted silyl groups, acetyl group, benzoyl group and the like acyl groups, benzenesulfonyl group, tosyl group and the like arylsulfonyl groups and methoxymethyl group, tetrahydropyranyl group and the like groups. As the protecting group of NH group on the benzimidazole ring, benzyl group, p-methoxybenzyl group, trityl group, tosyl group, mesyl group, formyl group, chloroacetyl group, t-butoxycarbonyl group and the like can be exemplified. Protection of carbonyl group can be effected for example by converting it into 1,3-dioxolan or 1,3-dithian.

When $R^1$ in the formula (I) is hydrogen atom (the following formula (I)-e), equilibrium may exist in the benzimidazole moiety between this formula and the following formula (I)-g as shown below. The existing ratio of each isomer

in the following equilibrium varies depending on the conditions of the compound, such as its solid state or solution in an appropriate solvent. Isomers in the equilibrium shown below cannot be separated, but their existing ratio can be analyzed by a spectroscopic means such as nuclear magnetic resonance (NMR). However, since the measurement is carried out generally in a solution in the case of the NMR analysis, there is a possibility that the existing ratio will change depending on the difference in the measuring solvent.

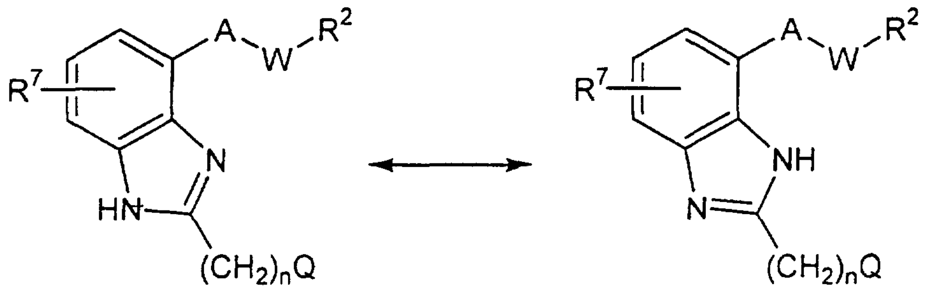

( I )-e ( I )-g

When A in the formula (I) is carbonyl group and W is methylene group (the following formula (I)-h), keto-enol equilibrium may exist between this formula and the following formula (XIV) as shown below. The existing ratio of each isomer in the following equilibrium varies depending on the conditions of the compound, such as its solid state or solution in an appropriate solvent or temperature. Isomers in the equilibrium shown below cannot be separated, but their existing ratio can be analyzed by a spectroscopic means such as nuclear magnetic resonance (NMR). However, since the measurement is carried out generally in a solution in the case of the NMR analysis, there is a possibility that the existing ratio will change depending on the difference in the measuring solvent.

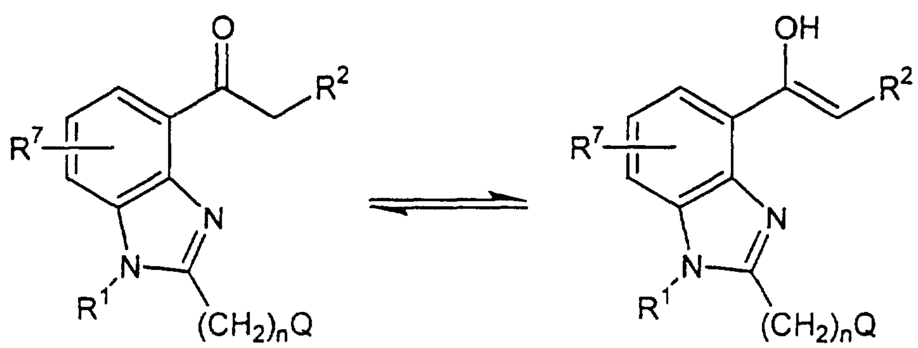

( I )-h (XIV)

Next, actions of the compound of the present invention and pharmaceutical compositions of the present invention are described in detail. Actions of typical compounds are shown as test examples regarding their illustrative pharmacological actions, toxicity and the like, but the present invention is not restricted thereby.

(Test Example 1) Action on intraperitoneal eosinophilia model mice

Using five animals per group of BALB/c male mice (about 25 g in body weight), 0.11 mL of physiological saline containing 0.1 mg of swine roundworm extract was administered into the peritoneal cavity of each mouse to sensitize. The same treatment was repeated 7 days after the administration and, 3 days thereafter, each animal was sacrificed by phlebotomy, 3 mL of physiological saline containing 1% dipotassium ethylenediaminetetraacetate (EDTA) was administered by intraperitoneal injection and its abdominal region was massaged for about 30 seconds. The abdominal

region was incised to collect intraperitoneal fluid which was subsequently centrifuged at 130 x g for 10 minutes, and the resulting precipitate was mixed with 500 μl of fetal calf serum containing 1% EDTA to prepare a cell suspension. A portion of the cell suspension was daubed on a slide glass using a spinner to carry out Diff-Quik staining, and then total leukocytes and eosinophils were counted to calculate the ratio of eosinophils in the total leukocytes. The number of total leukocytes in the cell suspension was measured using an automatic blood cell counter. The number of eosinophils in the cell suspension was calculated by multiplying the ratio of eosinophils in the total leukocytes by the number of total leukocytes.

Each of the compounds to be tested was suspended in 5% acacia aqueous solution and orally administered once a day for a total of 10 times starting immediately after the initial sensitization (test compound-administered group). Also, 5% acacia aqueous solution was orally administered to a group of mice in which sensitization and administration of test compounds were not carried out (non-treated group) and another group of mice in which sensitization was carried out but test compounds were not administered (control group).

The dose of each compound to be tested and the intraperitoneal eosinophilia inhibition rate calculated by the following formula are shown in Table 1.

Eosinophilia inhibition rate (%) = {1 - (test compound-administered group - non-treated group)/(control group - non-treated group)} x 100

Table 1

| Test compound (Example No.) | Dose (mg/kg/day) | Intraperitoneal eosinophilia inhibition rate (%) |
|---|---|---|
| 1 | 3 | 67 |
| | 30 | 78 |
| 2 | 100 | 42 |
| 3 | 10 | 67 |
| | 30 | 75 |
| 5 | 100 | 62 |
| 6 | 3 | 35 |
| | 10 | 59 |
| 7 | 0.3 | 55 |
| Prednisolone acetate | 10 | 61 - 85 |
| Prednisolone acetate: 11β,17α,21-trihydroxypregna-1,4-diene-3,20-dione 21-acetate | | |

Each of compounds of the present invention showed significant intraperitoneal eosinophilia inhibition action in BALB/c mice sensitized with the parasite extract.

(Test Example 2) Toxicity test

Toxicity of compounds of the present invention was examined. When each of the compounds of Inventive Example Nos. 1, 2, 5, 7 and 10 of the present invention was suspended in 5% acacia aqueous solution and orally administered to BALB/c male mice (about 25 g in body weight) in a dose of 100 mg/kg/day for 7 days, and the animals were observed for 3 days after completion of the administration, no mortal case was found and no abnormality was observed in terms of the body weight and general symptoms.

As is evident from the above description and results of the test examples, the compound of the present invention strongly inhibits intraperitoneal eosinophilia in the experimental animal model sensitized with a parasite extract. Also, the compound of the present invention has a selective IgE antibody production inhibition action and shows its efficacy in a bronchoconstrictive reaction model of sensitized animals. In addition, the compound of the present invention shows excellent oral bioavailability and has high safety with extremely low toxicity.

The compound of the present invention having benzimidazole nucleus is effective for the prevention, protection against onset, protection against worsening of symptoms, improvement of symptoms and treatment, including remedy, of diseases which exhibit eosinophilia, namely parasite infection, hypereosinophilic syndrome (HES), eosinophilic pneumonia (PIE syndrome), eosinophilic enterogastritis, bronchial asthma, atopic dermatitis, allergic rhinitis, nettle

rash, hypersensitivity pneumonitis, pulmonary aspergillosis, eosinophilic leukemia and the like diseases, and is particularly effective for the prevention or treatment of various allergic diseases including bronchial asthma.

In addition to the above diseases, it is possible to use the compound of the present invention in IgE antibody-induced diseases, namely various allergic diseases such as hay fever, angioneurotic edema, serous otitis media, pollinosis, allergic enterogastritis, food allergy, drug allergy and the like.

The pharmaceutical composition of the present invention is effective in controlling various symptoms of the diseases cited above and also can be used in preventive administration; for example, in the case of its administration to a patient suffering from a seasonal allergic disease (pollinosis for example), the patient can go through the season showing substantially no symptoms or with markedly slight symptoms when its administration is started just before the required season and continued until the end of the season.

In general, the compound of the present invention or a salt thereof is orally administered as a medicament to human and other animals, but it can also be administered parenterally (for example, intravenous injection, intramuscular injection, subcutaneous injection, rectal administration, percutaneous absorption, transmucosal absorption and the like).

The medicament of the present invention is administered in the form of a pharmaceutical composition.

The pharmaceutical composition of the present invention contains at least one of the compounds of formula (I) of the present invention and is prepared in combination with a pharmaceutically acceptable carrier. More illustratively, various dosage forms can be obtained by optionally combining the compound of the present invention with a filler (for example, lactose, sucrose, mannitol, crystalline cellulose or silicic acid), a binder (for example, crystalline cellulose, sugar (mannitol or sucrose), dextrin, hydroxypropylcellulose (HPC), hydroxypropylmethylcellulose (HPMC), polyvinyl pyrrolidone (PVP) or macrogol), a lubricant (for example, magnesium stearate, calcium stearate or talc), a coloring agent, a flavoring agent, a disintegrating agent (for example, corn starch or carboxymethylcellulose), an antiseptic agent, a tonicity agent, a stabilizing agent (for example, a sugar or a sugar alcohol), a dispersing agent, an antioxidant (for example, ascorbic acid, butylhydroxyanisole (BHA), propyl gallate or dl-α-tocopherol), a buffer agent, a preservative agent (for example, paraben, benzyl alcohol or benzalkonium chloride), an aromatic agent (for example, vanillin, 1-menthol or rose oil), a solubility assisting agent (for example, cholesterol or triethanolamine), a suspending agent or an emulsifying agent and a pharmacologically acceptable appropriate carrier or solvent.

Examples of such dosage forms include capsules, pills, tablets, granules, fine subtilaes and powders, as well as suspensions, emulsions, lemonades, elixirs, syrups and the like mixtures for internal use, inhalations, sprays, aerosols, spreading preparations and the like mixtures for external use, solutions for eye-drops and nasal drops, adhesive preparations, ointments, lotions, liniments, poultices, suppositories, aqueous or non-aqueous injections, emulsions or suspensions for injection use and solid injections which are dissolved, emulsified or suspended when used.

Dose of the compound of the present invention when used as a medicament is an amount sufficient enough for treating each disease to be treated, which is optionally changed depending on the dosage form of the medicament, administration method, the number of times of administration per day, degree of symptoms, body weight, age and the like factors. Dose of the compound of the present invention as a medicament is within the range of from 0.01 to 5,000 mg, preferably from 0.1 to 500 mg, more preferably from 0.1 to 100 mg, per day per adult. In the case of oral administration, its dose is within the range of from 0.01 to 5,000 mg, preferably from 0.1 to 300 mg, more preferably from 0.1 to 100 mg, per day per adult. It may be administered once a day or by dividing the daily dose into 2 to 6 doses per day. The compound of the present invention may be used jointly with conventional therapeutic drugs.

## EXAMPLES

Next, the present invention is described further in detail with reference to inventive and reference examples.

Wherein, NMR was measured using JNM-EX270 (manufactured by JEOL) or JEOL JNM-LA300 (manufactured by JEOL) and expressed by δ (ppm) using TMS (tetramethylsilane) as the internal standard. IR was measured by a pellet method using potassium bromide or a liquid film method (indicated as neat) using HORIBA FT-200 (manufactured by Horiba) and expressed by $cm^{-1}$. Melting point was measured using Mettler FP80 or FP90 (both manufactured by Mettler-Trade). Optical rotation was measured using JASCO DIP-1000 (manufactured by JASCO) and expressed by specific rotation [α].

(Reference Example 1)

Synthesis of 4-acetyl-2-(2-phenylethyl)benzimidazole

2,3-Diaminoacetophenone (16.8 g) was dissolved in dry dichloromethane (170 mL) to which was subsequently added triethylamine (16.4 mL) while cooling in an ice bath. To this, cooled in the ice bath, was added dropwise dry dichloromethane (30 mL) solution of 3-phenylpropionic acid chloride (17.5 mL). After stirring for 40 minutes, the mixture

was added to ice water (150 mL), and the layer was separated. The water layer was extracted with dichloromethane (150 mL) and the resulting organic layer was washed with brine. The combined organic layer was dried over anhydrous sodium sulfate and then the solvent was evaporated under a reduced pressure. The resulting residue was crystallized from ether and collected by filtration (26.5 g). The thus obtained crystal was suspended in toluene (530 mL) and added p-toluenesulfonic acid monohydrate (19.6 g). After heating under reflux for 30 minutes, the solvent was evaporated. The resulting residue was crystallized from ether and collected by filtration. The thus obtained crystal was suspended in ethyl acetate (200 mL), saturated sodium bicarbonate aqueous solution (150 mL) added and then the mixture was stirred. After separation of layers, the water layer was extracted with ethyl acetate (150 mL). The organic layer was combined and washed with water (100 mL) and brine (100 mL) in that order. After drying over anhydrous sodium sulfate, the solvent was evaporated under a reduced pressure. The resulting residue was dissolved in ethanol and activated charcoal (5 g) added. The activated charcoal was removed by filtration, and the resulting filtrate was concentrated under a reduced pressure. The resulting residue was crystallized from ether and hexane collected by filtration to obtain the title compound (21.9 g).

NMR ($CDCl_3$) $\delta$ [ppm]: 10.58 (1 H, bs), 7.96 (1 H, d, J = 8 Hz), 7.77 (1 H, d, J = Hz), 7.4 - 7.2 (6 H, m), 3.3 - 3.2 (4 H, m), 2.69 (3 H, s)

IR (KBr) [$cm^{-1}$]: 3294, 3024, 1655, 1595, 1524, 1429, 1269, 1134, 744

Melting point: 102.3 - 105.9°C

(Reference Example 2)

Synthesis of ethyl 2-(2-phenylethyl)benzimidazole-4-carboxylate

Ethyl 2,3-diaminobenzoate (25.3 g) was dissolved in dry dichloromethane (210 mL). While cooling in an ice bath and under an atmosphere of argon, to this solution was added triethylamine (19.7 mL), and to the reaction mixture was then added dropwise dry dichloromethane (38 mL) solution of 3-phenylpropionic acid chloride (21 mL) spending 1 hour, subsequently stirring the resulting mixture for 1 hour at the same temperature. Water (100 mL) was added to the reaction mixture and the layer was separated, and the water layer was extracted with dichloromethane. The organic layer was washed with brine and dried over anhydrous sodium sulfate and then the solvent was evaporated under a reduced pressure. To the resulting residue was added ether and a small amount of hexane, and to the amide compound collected by filtration (35.2 g) was added p-toluenesulfonic acid monohydrate (23.6 g) and toluene (352 mL), and the mixture was heated under reflux for 1 hour. The suspension was concentrated under a reduced pressure, and the resulting residue was crystallized from ether and collected by filtration. The thus obtained crystal was suspended in ethyl acetate (200 mL) and washed by stirring. The crystal was collected by filtration to obtain ethyl 2-(2-phenylethyl) benzimidazole-4-carboxylate p-toluenesulfonate (49.8 g). The thus obtained crystal was suspended in ethyl acetate (600 mL), and the suspension, cooled in an ice bath, was alkalified by adding saturated sodium bicarbonate aqueous solution (300 mL) to carry out separation of layers. The water layer was extracted with ethyl acetate. The organic layers were combined, washed with water (100 mL x 2) and brine (100 mL), dried over anhydrous sodium sulfate and then mixed with activated charcoal. The drying agent and activated charcoal were removed by filtration, and the resulting filtrate was concentrated under a reduced pressure. The resulting residue was crystallized from hexane and collected by filtration to obtain the title compound (26.7 g).

NMR ($CDCl_3$) $\delta$ [ppm]: 10.04 (1 H, bs), 7.93 (1 H, d, J = 8 Hz), 7.87 (1 H, d, J = 8 Hz), 7.4 - 7.2 (6 H, m), 4.43 (2 H, q, J = 7 Hz), 3.3 - 3.1 (4 H, m), 1.43 (3 H, t, J = 7 Hz)

(Reference Example 3)

Synthesis of 2-(2-phenylethyl)benzimidazole-4-carboxylic acid

To ethyl 2-(2-phenylethyl)benzimidazole-4-carboxylate (10 g) obtained in Reference Example 2 was added ethanol (46 mL) and water (68 mL) solution of sodium hydroxide (2.72 g), and the mixture was heated under reflux for 1 hour. After spontaneous cooling, this was mixed with water (100 mL) and washed with ether (200 mL). While cooling in an ice bath, the water layer was adjusted to pH 5. The thus formed precipitate was collected by filtration and washed with ether and ethanol (40 mL). The obtained precipitate was dried under a reduced pressure to obtain the title compound (8.49 g).

NMR (DMSO-$d_6$) $\delta$ [ppm]: 12.25 (1 H, bs), 7.80 (1 H, d, J = 8 Hz), 7.74 (1 H, d, J = 8 Hz), 7.4 - 7.1 (6 H, m), 3.3 - 3.0 (4 H, m)

(Reference Example 4)

Synthesis of 4-acetyl-2-benzylbenzimidazole

According to Reference Example 1, using 2,3-diaminoacetophenone (20 g) and phenylacetyl chloride (18.5 mL), the title compound was obtained as crystal (19.2 g).
NMR ($CDCl_3$) δ [ppm]: 10.56 (1 H, bs), 7.97 (1 H, d, J = 8 Hz), 7.76 (1 H, dd, J = 8, 1 H), 7.4 - 7.3 (6 H, m), 4.33 (2 H, s), 2.67 (3 H, s)

(Reference Example 5)

Synthesis of 3-(2-benzylbenzimidazol-4-yl)-3-oxopropanoic acid

According to Inventive Example 1, which is described later, using 4-acetyl-2-benzylbenzimidazole (19 g) obtained in Reference Example 4, the title compound was obtained as crystal (5.33 g).
NMR (DMSO-$d_6$) δ [ppm]: 12.74 (1 H, bs), 7.9 - 7.8 (2 H, m), 7.4 - 7.2 (7 H, m), 4.27 (2 H, s), 4.14 (1 H, bs)

(Reference Example 6)

Synthesis of ethyl 2-phenylbenzimidazole-4-carboxylate

To a solution of ethyl 2,3-diaminobenzoate (3.0 g) in methanol (60 mL) was added 1 N hydrochloric acid (0.6 mL) and benzaldehyde (1.7 mL), and the mixture was stirred at room temperature for 1 hour. After evaporation of the solvent under a reduced pressure, to the solution of the obtained residue in dichloromethane was added silica gel. The solvent was evaporated under a reduced pressure, and the resulting residue was heated at 100°C for 1 hour. By purifying the reaction mixture by a silica gel column chromatography (eluent: hexane/ethyl acetate), the title compound was obtained as crystal (1.8 g).
NMR ($CDCl_3$) δ [ppm]: 10.71 (1 H, bs), 8.1 - 8.0 (2 H, m), 8.03 (1 H, d, J = 8 Hz), 7.93 (1 H, dd, J = 8, 1 Hz), 7.6 - 7.5 (3 H, m), 7.33 (1 H, t, J = 8 Hz), 4.50 (2 H, q, J = 7 Hz), 1.49 (3 H, t, J = 7 Hz)

(Reference Example 7)

Synthesis of 2-phenylbenzimidazole-4-carboxylic acid

According to Reference Example 3, using ethyl 2-phenylbenzimidazole-4-carboxylate (1.8 g) obtained in Reference Example 6, the title compound was obtained as crystal (1.5 g).
NMR (DMSO-$d_6$) δ [ppm]: 13.26 (1 H, bs), 12.34 (1 H, bs), 8.4-8.3 (2 H, m), 7.93 (1 H, d, J = 8 Hz), 7.83 (1 H, d, J = 8 Hz), 7.6 - 7.5 (3 H, m), 7.34 (1 H, t, J = 8 Hz)

(Reference Example 8)

Synthesis of ethyl 2-(2-(4-benzyloxyphenyl)ethyl)benzimidazole-4-carboxylate

To a solution of ethyl 2,3-diaminobenzoate (2.5 g) in dry dichloromethane (20 mL) to which, while cooling in an ice bath, was subsequently added triethylamine (2.1 mL). To this was added dropwise dry dichloromethane (6 mL) solution of 4-benzyloxyphenylpropionic acid chloride (4 g). After stirring for 75 minutes in an ice bath, the reaction solution was poured into ice water (100 mL) extracted with dichloromethane. The resulting organic layers were combined, washed with water and brine in that order and then dried over anhydrous sodium sulfate. After evaporation of the solvent under a reduced pressure, the resulting residue was crystallized from hexane and collected by filtration to obtain crystal (5.57 g). The thus obtained crystal was added to toluene (50 mL) and p-toluenesulfonic acid monohydrate (2.8 g) and heated under reflux for 30 minutes. After evaporation of the solvent under a reduced pressure, the thus obtained residue was alkalified by adding saturated sodium bicarbonate aqueous solution. Ethyl acetate was added to the solution and the layer was separated. The water layer was extracted with ethyl acetate, and the resulting organic layers were combined and washed with water and brine in that order. After drying the thus treated organic layer over anhydrous sodium sulfate, the solvent was evaporated under a reduced pressure. The resulting residue was crystallized from ether and hexane and collected by filtration to obtain the title compound as crystal (3.3 g).
NMR ($CDCl_3$) δ [ppm]: 10.04 (1 H, bs), 7.93 (1 H, d, J = 8 Hz), 7.87 (1 H, d, J = 8 Hz), 7.5 - 7.3 (6 H, m), 7.17 (2 H, d, J = 8 Hz), 6.93 (2 H, d, J = 8 Hz), 5.05 (2 H, s), 4.43 (2 H, q, J = 7 Hz), 3.3 - 3.1 (4 H, m), 1.43 (3 H, t, J = 7 Hz)

(Reference Example 9)

Synthesis of 2-.(2-(4-benzyloxyphenyl)ethyl)benzimidazole-4-carboxylic acid

Ethyl 2-(2-(4-benzyloxyphenyl)ethyl)benzimidazole-4-carboxylate (3.3 g) obtained in Reference Example 8 was added to ethanol (15 mL) and 1 N sodium hydroxide aqueous solution (16.5 mL), and the mixture was heated under reflux for 40 minutes. The reaction solution was diluted with water and washed with ether, and the thus separated water layer was adjusted to pH 4 to 5 with 4 N hydrochloric acid. The thus precipitate was collected by filtration to obtain the title compound as crystal (2.72 g).
NMR (DMSO-$d_6$) δ [ppm]: 12.23 (1 H, bs), 7.79 (1 H, d, J = 9 Hz), 7.73 (1 H, d, J = 9 Hz), 7.5 - 7.1 (8 H, m), 6.91 (2 H, d, J = 9 Hz), 5.05 (2 H, s), 3.2 - 3.0 (4 H, m)

(Reference Example 10)

Synthesis of ethyl 3-(2-(2-(4-benzyloxyphenyl)ethyl)benzimidazol-4-yl)-3-oxopropanoate

According to Inventive Example 12 which is described later, using 2-(2-(4-benzyloxyphenyl)ethyl)benzimidazole-4-carboxylic acid (2.72 g) obtained in Reference Example 9, the title compound was obtained (1.8 g).
NMR ($CDCl_3$) δ [ppm]: 10.55 (1 H, bs), 7.99 (1 H, d, J = 8 Hz), 7.72 (1 H, dd, J = 8, 1 Hz), 7.5 - 7.3 (6 H, m), 7.16 (2 H, d, J = 9 Hz), 6.92 (2 H, d, J = 9 Hz), 5.05 (2 H, s), 4.24 (2 H, q, J = 7 Hz), 4.10 (2 H, s), 3.3 - 3.1 (4 H, m), 1.28 (3 H, t, J = 7 Hz)

(Reference Example 11)

Synthesis of 6-chloro-4,5-dihydro-2-(2-phenylethyl)-6H-imidazo[4,5,1-i,j]-quinoline hydrochloride

To a solution of 4,5-dihydro-2-(2-phenylethyl)-6H-imidazo[4,5,1-i,j]quinolin-6-ol (118 g) was dissolved in dichloromethane (1.2 L) to which, cooled in an ice bath, was subsequently added dropwise thionyl chloride (95 mL). After 30 minutes of reaction at room temperature, the solvent was evaporated under a reduced pressure. Ether was added to the resulting residue, and the thus formed crystal was collected by filtration to obtain the title compound (139 g).
NMR (DMSO-$d_6$) δ [ppm]: 7.8 - 7.7 (1 H, m), 7.6 - 7.5 (2 H, m), 7.4 - 7.2 (5 H, m), 5.81 (1 H, t, J = 3 Hz), 4.6 - 4.5 (1 H, m), 4.2 - 4.0 (1 H, m), 3.6 - 3.4 (2 H, m), 3.3 - 3.1 (2 H, m)

(Reference Example 12)

Synthesis of 2-(2-phenylethyl)-4H-imidazo[4,5,1-i,j]-quinoline

6-Chloro-4,5-dihydro-2-(2-phenylethyl)-6H-imidazo[4,5,1-i,j]-quinoline hydrochloride (10 g) obtained in Reference Example 11 and potassium carbonate (4.1 g) were suspended in N,N-dimethylformamide (20 mL), and the suspension was heated at 130°C for 50 minutes. After spontaneous cooling, this was mixed with saturated sodium bicarbonate aqueous solution and extracted with ethyl acetate. The organic layer was washed with brine and then dried over anhydrous sodium sulfate. After evaporation of the solvent under a reduced pressure, the resulting residue was purified by a silica gel column chromatography (eluent: hexane/ethyl acetate) to obtain the title compound as crystal (5.6 g).
NMR ($CDCl_3$) δ [ppm]: 7.48 (1 H, d, J = 8 Hz), 7.4 - 7.2 (5 H, m), 7.1 - 7.0 (1 H, m), 6.82 (1 H, d, J = 7 Hz), 6.6 - 6.5 (1 H, m), 5.8 - 5.7 (1 H, m), 4.76 (2 H, bs), 3.3 - 3.0 (4 H, m)

(Reference Example 13)

Synthesis of 2-(2-phenylethyl)-4H-imidazo[4,5,1-i,j]-quinolin-4-on

While stirring and cooling in an ice bath, to dichloromethane (100 mL) solution of 2-(2-phenylethyl)-4H-imidazo [4,5,1-i,j]-quinoline (5.0 g) obtained in Reference Example 12 was added dichloromethane (300 mL) suspension of potassium permanganate (4.56 g) and butyltriethylammonium chloride (6.56 g) spending 1 hour. After stirring at room temperature for 1 hour, to the reaction mixture was added potassium permanganate (1.52 g) and butyltriethylammonium chloride (2.19 g). After stirring overnight at room temperature, the reaction solution was added to 1 N sodium hydroxide aqueous solution (200 mL) and stirred at room temperature. The insoluble material was removed by filtration, and the resulting filtrate was separated. The water layer was extracted with dichloromethane, and the resulting organic layers

were combined. The combined organic layer was washed with water and brine in that order and then dried over anhydrous sodium sulfate. After evaporation of the solvent under a reduced pressure, the resulting residue was purified by a silica gel column chromatography (eluent: hexane/ethyl acetate) to obtain the title compound as colorless crystal (2.61 g).
NMR (DMSO-$d_6$) δ [ppm]: 8.18 (1 H, d, J = 9 Hz), 7.95 (1 H, d, J = 8 Hz), 7.79 (1 H, d, J = 8 Hz), 7.55 (1 H, t, J = 8 Hz), 7.4-7.2 (5 H, m), 6.74 (1 H, d, J = 9 Hz), 3.60 (2 H, t, J = 8 Hz), 3.19 (2 H, t, J = 8 Hz)

(Reference Example 14)

Synthesis of ethyl 1-(4-methoxybenzyl)-2-(2-phenylethyl)benzimidazole-4-carboxylate

Ethyl 2-(2-phenylethyl)benzimidazole-4-carboxylate (10 g) obtained in Reference Example 2, potassium carbonate (7.05 g) and p-methoxybenzyl chloride (7.99 g) were stirred in N,N-dimethylformamide (150 mL) at room temperature for 20 hours. The reaction solution was diluted with water and extracted with ether. The organic layer was washed with water and brine and dried over anhydrous sodium sulfate and then the solvent was evaporated under a reduced pressure. The resulting residue was purified by a silica gel column chromatography (eluent: hexane/ethyl acetate) and the desired fraction was concentrated under a reduced pressure to obtain the title compound as oil (10.1 g).
NMR ($CDCl_3$) δ [ppm]: 7.92 (1 H, dd, J = 8, 1 Hz), 7.38 (1 H, dd, J = 8, 1 Hz), 7.3 - 7.2 (6 H, m), 6.88 (2 H, d, J = 9 Hz), 6.78 (2 H, d, J = 9 Hz), 5.15 (2 H, s), 4.52 (2 H, q, J = 7 Hz), 3.76 (3 H, s), 3.21 (4 H, s), 1.48 (3 H, t, J = 7 Hz)

(Reference Example 15)

Synthesis of 4-hydroxymethyl-1-(4-methoxybenzyl)-2-(2-phenylethyl)benzimidazole

Ethyl 1-(4-methoxybenzyl)-2-(2-phenylethyl)benzimidazole-4-carboxylate (5.5 g) obtained in Reference Example 14 was dissolved in anhydrous tetrahydrofuran (55 mL). While cooling in an ice bath, lithium aluminum hydride (0.30 g) was added in small portions to the solution, and the mixture was stirred at the same temperature for 1 hour. The reaction solution was poured into ice water and mixed with ethyl acetate and then the insoluble material was removed by filtration. The resulting filtrate was separated, the organic layer was washed with brine and dried with anhydrous sodium sulfate, and then the solvent was evaporated under a reduced pressure. The resulting residue was crystallized from ether and hexane and collected by filtration to obtain the title compound (4.35 g).
NMR ($CDCl_3$) δ [ppm]: 7.3 - 7.1 (8 H, m), 6.92 (2 H, d, J = 9 Hz), 6.80 (2 H, d, J = 9 Hz), 5.16 (2 H, bs), 5.13 (2 H, s), 4.35 (1 H, bs), 3.76 (3 H, s), 3.13 (4 H, bs)

(Reference Example 16)

Synthesis of dimethyl (1-(4-methoxybenzyl)-3-(2-(2-phenylethyl)benzimidazol-4-yl)methoxymethyl)phthalate

4-Hydroxymethyl-1-(4-methoxybenzyl)-2-(2-phenylethyl)benzimidazole (1.0 g) obtained in Reference Example 15 was dissolved in anhydrous tetrahydrofuran (15 mL), and 60% sodium hydride (237 mg) was added in small portions to the thus prepared solution which was cooled in an ice bath. After stirring for 20 minutes at the same temperature, this was added to anhydrous tetrahydrofuran (2 mL) solution of dimethyl 3-bromomethylphthalate (927 mg) and stirred at the same temperature for 1.5 hours. The reaction solution was diluted with ice water and extracted with ethyl acetate. The organic layer was washed with brine and dried over anhydrous sodium sulfate, and then the solvent was evaporated under a reduced pressure. The resulting residue was purified by a silica gel column chromatography (eluent: hexane/ethyl acetate) and the desired fraction was concentrated under a reduced pressure to obtain the title compound as oil (1.03 g).
NMR ($CDCl_3$) δ [ppm]: 7.91 (1 H, d, J = 8 Hz), 7.87 (1 H, d, J = 8 Hz), 7.49 (1 H, t, J = 8 Hz), 7.4 - 7.2 (8 H, m), 6.92 (2 H, d, J = 9 Hz), 6.80 (2 H, d, J = 9 Hz), 5.11 (4 H, s), 4.79 (2 H, s), 3.90 (3 H, s), 3.87 (3 H, s), 3.76 (3 H, s), 3.2 - 3.1 (4 H, m)

(Reference Example 17)

Synthesis of 2-bromo-4-methoxy-6-nitroaniline

To a solution of 4-methoxy-2-nitroaniline (35 g) in dichloromethane (350 mL) was subsequently added dropwise bromine (12.9 mL) at -20°C. After stirring for 30 minutes at the same temperature, this was poured into ice water, adjusted to pH 7 to 8 with saturated sodium bicarbonate aqueous solution and then extracted with dichloromethane.

The organic layer was washed with brine and dried over anhydrous sodium sulfate, and then the solvent was evaporated under a reduced pressure. The resulting residue was purified by a silica gel column chromatography (eluent: hexane/ethyl acetate). After concentration of the desired fraction, the resulting residue was crystallized from hexane and ethyl acetate and then collected by filtration to obtain the title compound as reddish orange crystals (29.6 g).
NMR ($CDCl_3$) δ [ppm]: 7.63 (1 H, d, J = 3 Hz), 7.44 (1 H, d, J = 3 Hz), 6.38 (2 H, bs), 3.80 (3 H, s)

(Reference Example 18)

Synthesis of 2-amino-5-methoxy-3-nitrobenzonitrile

To a solution of 2-bromo-4-methoxy-6-nitroaniline (45 g) obtained in Reference Example 17 in N-methyl-2-pyrrolidone (225 mL) was subsequently added copper(I) cyanide (33 g). This was stirred at 150°C for 5.5 hours. After spontaneous cooling, aqueous ammonia was added to the mixture and the resulting mixture was stirred for 20 minutes, and then insoluble material was separated by filtration. The thus separated residue was washed with ethyl acetate, and the filtrate and washed solution were combined to carry out separation of layers. The organic layer was dried over anhydrous sodium sulfate and then the solvent was evaporated under a reduced pressure to obtain the title compound as crystal (14.1 g).
NMR ($CDCl_3$) δ [ppm]: 7.90 (1 H, d, J = 3 Hz), 7.38 (1 H, d, J = 3 Hz), 6.45 (2 H, bs), 3.83 (3 H, s)

(Reference Example 19)

Synthesis of 2,3-diamino-5-methoxybenzonitrile

2-Amino-5-methoxy-3-nitrobenzonitrile (9.6 g) obtained in Reference Example 18 and 10% palladium/carbon (0.96 g) were suspended in methanol (96 mL). The suspension was stirred at room temperature for 16 hours under an atmosphere of hydrogen. The catalyst was removed by filtration, and the resulting filtrate was evaporated under a reduced pressure. The resulting residue was crystallized from ether and collected by filtration to obtain the title compound as crystal (7.0 g).
NMR (DMSO-$d_6$) δ [ppm]: 6.37 (1 H, d, J = 2 Hz), 6.21 (1 H, d, J = 2 Hz), 5.13 (2 H, bs), 4.97 (2 H, bs), 3.60 (3 H, s)

(Reference Example 20)

Synthesis of 4-cyano-6-methoxy-2-(2-phenylethyl)benzimidazole

To a solution of 2,3-diamino-5-methoxybenzonitrile (7.0 g) obtained in Reference Example 19 in dry dichloromethane (140 mL), was added triethylamine (6.0 mL). While cooling in an ice bath, to this was added dropwise dry dichloromethane (70 mL) solution of 3-phenylpropionic acid chloride (6.4 mL) spending 2 hours. After stirring for 20 minutes at the same temperature, ice water (200 mL) was added thereto. Separation of layers was carried out, and the water layer was extracted with dichloromethane. The organic layer was combined and washed with brine and dried over anhydrous sodium sulfate, and the solvent was then evaporated under a reduced pressure. The thus obtained residue was crystallized from ether to obtain an amide compound (10.8 g). The thus obtained amide compound and p-toluenesulfonic acid monohydrate (7.7 g) were suspended in toluene (108 mL). After stirring for 30 minutes at 100°C, the solvent was evaporated under a reduced pressure. The thus obtained residue was crystallized from acetone and collected by filtration to obtain toluenesulfonate of the desired compound. The thus obtained salt was suspended in ethyl acetate (500 mL) and alkalified by adding saturated sodium bicarbonate aqueous solution (200 mL) to carry out separation of layers. The organic layer was washed with brine and dried over anhydrous sodium sulfate, and then the solvent was evaporated under a reduced pressure. The resulting residue was crystallized from ether and collected by filtration to obtain the title compound (5.9 g).
NMR (DMSO-$d_6$) δ [ppm]: 12.65 (1 H, bs), 7.3 - 7.2 (7 H, m), 3.83 (3 H, s), 3.12 (4 H, bs)

(Reference Example 21)

Synthesis of 6-methoxy-2-(2-phenylethyl)benzimidazole-4-carboxylic acid

4-Cyano-6-methoxy-2-(2-phenylethyl)benzimidazole (1.23 g) obtained in Reference Example 20 was added to 90% KOH aqueous solution (40 mL) and ethylene glycol (60 mL), and the mixture was heated under reflux for 8 hours. This was diluted with water and washed with ethyl acetate. The resulting water layer was adjusted to pH 6 with concentrated hydrochloric acid, and the thus precipitate was collected by filtration to obtain the title compound (1.27 g).

NMR (DMSO-$d_6$) δ [ppm]: 7.3 - 7.1 (7 H, m), 3.81 (3 H, s), 3.2-3.0 (4 H, m)

(Reference Example 22)

Synthesis of 6-hydroxy-2-(2-phenylethyl)benzimidazole-4-carboxylic acid

4-Cyano-6-methoxy-2-(2-phenylethyl)benzimidazole (5.4 g) obtained in Reference Example 20 was dissolved in acetic acid (135 mL), and to the solution was added 48% hydrobromic acid (135 mL) and the mixture was heated under reflux for 8 hours. The reaction solution was poured into ice water (200 mL) and adjusted to pH 4 with 6 N sodium hydroxide. The thus precipitate was collected by filtration and washed with ethanol and ether to obtain the title compound as gray crystals (5.3 g). NMR (DMSO-$d_6$) δ [ppm]: 11.95 (1 H, bs), 9.28 (1 H, bs), 7.3-7.1 (7 H, m), 3.2 - 3.0 (4 H, m)

(Reference Example 23)

Synthesis of 6-t-butyldimethylsilyloxy-2-(2-phenylethyl)benzimidazole-4-carboxylic acid

Dry N,N-dimethylformamide (28 mL) was added to a mixture consisting of 6-hydroxy-2-(2-phenylethyl)benzimidazole-4-carboxylic acid (2.8 g) obtained in Reference Example 22, t-butyldimethylsilyl chloride (6.0 g) and N,N-dimethyl-4-aminopyridine (0.24 g). Triethylamine (5.5 mL) was added to the resulting mixture which was cooled in an ice bath, and then the mixture was stirred at room temperature for 2 hours. This was diluted with ice water (60 mL) and adjusted to pH 4 to 5 with 4 N hydrochloric acid. The thus precipitate was collected by filtration and washed with water. The thus obtained crystal was dissolved in tetrahydrofuran and dried over anhydrous sodium sulfate, and then the solvent was evaporated under a reduced pressure. The resulting residue was crystallized from hexane to obtain the title compound as crystal (3.4 g).
NMR (DMSO-$d_6$) δ [ppm]: 12.14 (1 H, bs), 7.3 - 7.2 (7 H, m), 3.2 - 3.0 (4 H, m), 0.97 (9 H, s), 0.19 (6 H, s)

(Reference Example 24)

Synthesis of ethyl 3-(6-t-butyldimethylsilyloxy-2-(2-phenylethyl)benzimidazol-4-yl)-3-oxopropanoate

Triethylamine (2.65 mL) and magnesium chloride (2.16 g) were added to dry acetonitrile (37.5 mL) suspension of potassium ethylmalonate (3.23 g), and the mixture was vigorously stirred at room temperature for 16 hours under in an atmosphere of argon. Next, a catalytically effective amount of N,N-dimethylaminopyridine and 1,1'-carbonyldiimidazole (1.35 g) were added to anhydrous tetrahydrofuran (30 mL) solution of 6-t-butyldimethylsilyloxy-2-(2-phenylethyl)benzimidazole-4-carboxylic acid (3.0 g) obtained in Reference Example 23, and the mixture was stirred for 1 hour under an atmosphere of argon. This was diluted to the previously prepared suspension of ethyl malonate magnesium salt while cooling in an ice bath, and the mixture was stirred at room temperature for 2 hours. This was diluted with ice water (50 mL) and, while cooling in an ice bath, adjusted to pH 1 with 4 N hydrochloric acid. While cooling in an ice bath, this was adjusted to pH 8 to 9 with saturated sodium bicarbonate aqueous solution and extracted with ethyl acetate. The organic layer was washed with brine and dried over anhydrous sodium sulfate, and then the solvent was evaporated under a reduced pressure. The resulting residue was purified by a silica gel column chromatography (eluent: hexane/ethyl acetate) and the desired fraction was concentrated to obtain the title compound as colorless oil (3.44 g).

(Reference Example 25)

Synthesis of ethyl 3-(6-t-butyldimethylsilyloxy-2-(2-phenylethyl)benzimidazol-4-yl)-3-hydroxypropanoate

Ethyl 3-(6-t-butyldimethylsilyloxy-2-(2-phenylethyl)benzimidazol-4-yl)-3-oxopropanoate (2.0 g) obtained in Reference Example 24 was dissolved in anhydrous tetrahydrofuran (30 mL). Sodium borohydride (49 mg) was added to the solution which was cooled in an ice bath, and the mixture was stirred at room temperature for 3 hours. This was diluted with ice water, extracted with ethyl acetate and then washed with brine. The organic layer was dried over anhydrous sodium sulfate, and then the solvent was evaporated under a reduced pressure. The resulting residue was purified by a silica gel column chromatography (eluent: hexane/ethyl acetate). By concentrating the desired fraction, the title compound was obtained as colorless oil (700 mg).
NMR ($CDCl_3$) δ [ppm]: 9.46 (1 H, bs), 7.3 - 7.0 (6 H, m), 6.50 (1 H, s), 5.34 (1 H, bs), 4.22 (2 H, q, J = 7 Hz), 3.17 (4 H, s), 2.76 (2 H, d, J = 6 Hz), 1.30 (3 H, t, J = 7 Hz)

(Reference Example 26)

Synthesis of ethyl 3-(6-t-butyldimethylsilyloxy-2-(2-phenylethyl)benzimidazol-4-yl)propenoate

Ethyl 3-(6-t-butyldimethylsilyloxy-2-(2-phenylethyl)benzimidazol-4-yl)-3-hydroxypropanoate (530 mg) obtained in Reference Example 25 was dissolved in dichloromethane (8 mL). To this was added pyridine (0.14 mL) and then, while cooling in an ice bath, thionyl chloride (0.11 mL), and the mixture was stirred at the same temperature for 10 minutes. The reaction solution was diluted with ice water, adjusted to pH 7 to 8 with saturated sodium bicarbonate aqueous solution and then extracted with dichloromethane. The organic layer was washed with water and brine and dried over anhydrous sodium sulfate, and then the solvent was evaporated to obtain a chloro compound as oil. The thus obtained oily product was dissolved in dichloromethane (8 mL), and the solution which was cooled in an ice bath was mixed with 1,8-diazabicyclo[5.4.0]-7-undecene (0.17 mL) and stirred at the same temperature for 5 minutes. The reaction solution was mixed with ice water and extracted with dichloromethane. The organic layer was washed with water and brine and dried over anhydrous sodium sulfate and then the solvent was evaporated. The resulting residue was purified by a silica gel column chromatography (eluent: hexane/ethyl acetate). By concentrating the desired fraction, the title compound was obtained as oil (318 mg).
NMR ($CDCl_3$) $\delta$ [ppm]: 8.60 (1 H, bs), 8.02 (1 H, d, J = 16 Hz), 7.4 - 7.2 (5 H, m), 6.90 (1 H, d, J = 2 Hz), 6.79 (1 H, d, J = 2 Hz), 4.29 (2 H, q, J = 7 Hz), 3.3 - 3.2 (4 H, m), 1.36 (3 H, t, J = 7 Hz), 0.99 (9 H, s), 0.19 (6 H, s)

(Reference Example 27)

Synthesis of ethyl 6-chloro-2-(2-phenylethyl)benzimidazole-4-carboxylate

According to Reference Example 2, using ethyl 2,3-diamino-5-chlorobenzoate (2.3 g), the title compound was obtained as crystal (3.10 g).
NMR ($CDCl_3$) $\delta$ [ppm]: 10.00 (1 H, bs), 7.88 (1 H, d, J = 2 Hz), 7.83 (1 H, d, J = 2 Hz), 7.4 - 7.2 (5 H, m), 4.43 (2 H, q, J = 7 Hz), 3.3 - 3.2 (4 H, m), 1.43 (3 H, t, J = 7 Hz)

(Reference Example 28)

Synthesis of 6-chloro-2-(2-phenylethyl)benzimidazole-4-carboxylic acid

According to Reference Example 3, using ethyl 6-chloro-2-(2-phenylethyl)benzimidazole-4-carboxylate (3.09 g) obtained in Reference Example 27, the title compound was obtained as crystal (2.8 g).
NMR (DMSO-$d_6$) $\delta$ [ppm]: 8.02 (1 H, d, J = 2 Hz), 7.80 (1 H, d, J = 2 Hz), 7.3 - 7.2 (6 H, m), 3.4 - 3.3 (2 H, m), 3.3 - 3.1 (2 H, m)

(Reference Example 29)

Synthesis of 4-hydroxymethyl-2-(2-phenylethyl)benzimidazole

While cooling in an ice bath, anhydrous tetrahydrofuran (63 mL) solution of ethyl 2-(2-phenylethyl)benzimidazole-4-carboxylate (20 g) obtained in Reference Example 2 was added dropwise to anhydrous tetrahydrofuran (63 mL) suspension of lithium aluminum hydride (5.2 g), and the mixture was stirred at room temperature for 15 minutes. After addition of ice water (200 mL) in small portions, ethyl acetate (400 mL) was added and insoluble material was removed by filtration. The resulting filtrate was separated, the organic layer was washed with water and brine and dried over anhydrous sodium sulfate, and then the solvent was evaporated under a reduced pressure. The resulting residue was crystallized from ether and collected by filtration to obtain the title compound as crystal (15.7 g).
NMR (DMSO-$d_6$) $\delta$ [ppm]: 12.22 (0.4 H, bs), 12.11 (0.6 H, bs), 7.5 - 7.0 (8 H, m), 5.22 (0.6 H, t, J = 6 Hz), 5.08 (0.4 H, t, J = 6 Hz), 4.88 (0.8 H, d, J = 6 Hz), 4.74 (1.2 H, d, J = 6 Hz), 3.2-3.1 (4 H, m)

(Reference Example 30)

Synthesis of 4-formyl-2-(2-phenylethyl)benzimidazole

Anhydrous tetrahydrofuran (45 mL) was added to 4-hydroxymethyl-2-(2-phenylethyl)benzimidazole (15.0 g) obtained in Reference Example 29 and activated manganese dioxide (51.7 g), and the mixture was vigorously stirred at room temperature for 45 minutes. Insoluble material was removed by filtration and the resulting filtrate was concentrated under a reduced pressure. The thus obtained residue was purified by a column chromatography (eluent: hexane/ethyl acetate) and the desired fraction was concentrated under a reduced pressure. The resulting residue was crystallized from hexane and collected by filtration to obtain the title compound as crystal (12.2 g).
NMR ($CDCl_3$) δ [ppm]: 10.35 (1 H, bs), 10.08 (1 H, s), 8.01 (1 H, d, J = 8 Hz), 7.69 (1 H, d, J = 8 Hz), 7.4 - 7.2 (4 H, m), 3.4-3.1 (4 H, m)

(Reference Example 31)

Synthesis of methyl 5-fluoro-2-(2-phenylethyl)benzimidazole-4-carboxylate

According to Reference Example 2, using methyl 2,3-diamino-6-fluorobenzoate (3.9 g), the title compound was obtained as oil (2.53 g).
NMR ($CDCl_3$) δ [ppm]: 10.15 (1 H, bs), 7.83 (1 H, dd, J = 9, 4 Hz), 7.4 - 7.2 (5 H, m), 7.02 (1 H, dd, J = 12, 9 Hz), 3.98 (3 H, s), 3.3 - 3.1 (4 H, m)

(Reference Example 32)

Synthesis of 5-fluoro-2-(2-phenylethyl)benzimidazole-4-carboxylic acid

According to Reference Example 3, using methyl 5-fluoro-2-(2-phenylethyl)benzimidazole-4-carboxylate (2.53 g) obtained in Reference Example 31, the title compound was obtained as crystal (2.3 g).
NMR (DMSO-$d_6$) δ [ppm]: 7.77 (1 H, dd, J = 9, 4 Hz), 7.3 - 7.1 (5 H, m), 7.08 (1 H, dd, J = 12, 9 Hz), 3.3 - 2.9 (4 H, m)

(Inventive Example 1)

Synthesis of 3-(2-(2-phenylethyl)benzimidazol-4-yl)-3-oxopropanoic acid

4-Acetyl-2-(2-phenylethyl)benzimidazole (16 g) obtained in Reference Example 1 was dissolved in dimethyl sulfoxide (150 mL), and to the solution was added 18-crown-6 ether (16 g) and potassium carbonate (50 g). Carbon dioxide was bubbled for 6 hours into the resulting mixture which was stirred at room temperature. The reaction solution was poured into ice water (700 mL) and extracted with ethyl acetate. The water layer was adjusted to pH 5 to 6 by adding 6 N hydrochloric acid, and the precipitate was collected by filtration. The thus precipitate was dissolved in 1 N sodium hydroxide aqueous solution (150 mL), and the solution was washed with ether and then adjusted to pH 5 to 6 with 6 N hydrochloric acid. The resulting precipitate was collected by filtration to obtain the title compound (8.46 g).
NMR (DMSO-$d_6$) δ [ppm]: 12.71 (1 H, bs), 7.9 - 7.8 (2 H, m), 7.3 - 7.2 (6 H, m), 4.23 (2 H, s), 3.2 - 3.0 (4 H, m)
IR (KBr) [$cm^{-1}$]: 3379, 1701, 1651, 1275, 1200, 1115
Melting point: 107.1 - 107.5°C

(Inventive Example 2)

Synthesis of methyl 3-(2-(2-phenylethyl)benzimidazol-4-yl)-3-oxopropanoate

3-(2-(2-Phenylethyl)benzimidazol-4-yl)-3-oxopropanoic acid (4.0 g) obtained in Inventive Example 1 was suspended in methanol (25 mL) and tetrahydrofuran (100 mL), to which was subsequently added 2 M trimethylsilyldiazomethane-hexane solution (8.45 mL) in small portions. After stirring for 45 minutes at room temperature, the reaction solution was concentrated under a reduced pressure. The resulting residue was purified by a silica gel column chromatography (eluent: hexane/ethyl acetate) to obtain the title compound as slightly yellow crystal (3.45 g).
NMR ($CDCl_3$) δ [ppm]: 10.60 (1 H, bs), 7.99 (1 H, d, J = 8 Hz), 7.71 (1 H, d, J = 8 Hz), 7.3 - 7.2 (6 H, m), 4.11 (2 H, s), 3.77 (3 H, s), 3.3 - 3.2 (4 H, m)
IR (KBr) [$cm^{-1}$]: 2939, 1740, 1674, 1518, 1271, 1109
Melting point: 56.8 - 59.1°C

(Inventive Example 3)

Synthesis of ethyl 3-(2-(2-phenylethyl)benzimidazol-4-yl)-3-oxopropanoate

3-(2-(2-Phenylethyl)benzimidazol-4-yl)-3-oxopropanoic acid (6.0 g) obtained in Inventive Example 1 was added to 25% hydrochloric acid-ethanol (120 mL) and the mixture was stirred overnight at room temperature. The reaction

solution was concentrated under a reduced pressure and adjusted to pH 7 to 8 by adding saturated sodium bicarbonate aqueous solution. This was extracted with ethyl acetate and washed with water and brine in that order. The organic layer was dried over anhydrous sodium sulfate and then concentrated under a reduced pressure. The resulting residue was crystallized from ether and hexane and collected by filtration to obtain the title compound as slightly reddish white crystal (4.3 g).
NMR ($CDCl_3$) δ [ppm]: 10.56 (1 H, bs), 7.99 (1 H, d, J = 8 Hz), 7.72 (1 H, d, J = 8 Hz), 7.3 - 7.2 (6 H, m), 4.24 (2 H, q, J = 7 Hz), 4.09 (2 H, s), 3.3 - 3.2 (4 H, m), 1.28 (3 H, t, J = 7 Hz)
IR (KBr) [$cm^{-1}$]: 2974, 1738, 1662, 1271, 1188, 1142
Melting point: 108.0 - 109.7°C

(Inventive Example 4)

Synthesis of t-butyl 3-(2-(2-phenylethyl)benzimidazol-4-yl)-3-oxopropanoate

2-Chloro-1,3-dimethylimidazolinium hexafluorophosphate (1.9 g), t-butanol (0.50 g) and triethylamine (0.94 mL) were added to dichloromethane (20 mL), and the mixture was stirred at room temperature for 1 hour. 3-(2-(2-Phenylethyl)benzimidazol-4-yl)-3-oxopropanoic acid (0.60 g) obtained in Inventive Example 1 was dissolved in dichloromethane (20 mL) and added dropwise to the just described solution which was cooled in an ice bath. After stirring for 1 hour, the reaction solution was mixed with saturated sodium bicarbonate aqueous solution and dichloromethane to carry out separation of layers. The organic layer was washed with brine and dried over anhydrous sodium sulfate. After evaporation of the solvent under a reduced pressure, the resulting residue was purified by a silica gel column chromatography (eluent: hexane/ethyl acetate). The title compound was obtained as white crystal (0.26 g).
NMR ($CDCl_3$) δ [ppm]: 10.57 (1 H, bs), 7.98 (1 H, d, J = 8 Hz), 7.71 (1 H, d, J = 7 Hz), 7.4 - 7.2 (6 H, m), 4.00 (2 H, s), 3.3 - 3.2 (4 H, m), 1.46 (9 H, s)
IR (KBr) [$cm^{-1}$]: 2974, 1749, 1728, 1670, 1524, 1375, 1273, 1111
Melting point: 121.5 - 123.0°C

(Inventive Example 5)

Synthesis of methyl 3-(2-(2-phenylethyl)benzimidazol-4-yl)-3-hydroxypropanoate

Methyl 3-(2-(2-phenylethyl)benzimidazol-4-yl)-3-oxopropanoate (3.4 g) obtained in Inventive Example 2 was dissolved in anhydrous methanol (40 mL), and sodium borohydride (203 mg) was added in small portions to the resulting solution which was cooled in an ice bath. After stirring for 30 minutes in an ice bath and subsequent stirring for 40 minutes at room temperature, water (100 mL) was added to the reaction solution. This was extracted with ethyl acetate, and the organic layer was washed with brine. The organic layer was dried over anhydrous sodium sulfate and then the solvent was evaporated under a reduced pressure. The resulting residue was purified by a silica gel column chromatography (eluent: hexane/ethyl acetate). The desired fraction was concentrated, and the resulting residue was crystallized from hexane and ether and collected by filtration to obtain the title compound as crystal (2.72 g).
NMR ($CDCl_3$) δ [ppm]: 9.57 (1 H, s), 7.64 (1 H, d, J = 8 Hz), 7.3 - 7.1 (6 H, m), 6.93 (1 H, d, J = 7 Hz), 5.43 (1 H, t, J = 5 Hz), 3.87 (1 H, s), 3.77 (3 H, s), 3.3 - 3.2 (4 H, m), 2.80 (2 H, d, J = 6 Hz)
IR (KBr) [$cm^{-1}$]: 3028, 1740, 1433, 1032, 1003, 756
Melting point: 156.4 - 157.4°C

(Inventive Example 6)

Synthesis of ethyl 3-(2-(2-phenylethyl)benzimidazol-4-yl)-3-hydroxypropanoate

Ethyl 3-(2-(2-phenylethyl)benzimidazol-4-yl)-3-oxopropanoate (0.20 g) obtained in Inventive Example 3 was dissolved in ethanol (5 mL), and to the solution was added sodium borohydride (8 mg) while cooling in an ice bath and then the mixture was stirred at room temperature for 30 minutes. The reaction solution was diluted with water, and ethanol was removed under a reduced pressure. The remaining aqueous solution was extracted with ethyl acetate. The organic layer was washed with brine and then dried over anhydrous sodium sulfate. After evaporation of the solvent under a reduced pressure, the resulting residue was purified by a silica gel column chromatography (eluent: hexane/ethyl acetate) to obtain the title compound as white crystal (0.13 g).
NMR ($CDCl_3$) δ [ppm]: 9.60 (1 H, s), 7.63 (1 H, d, J = 8 Hz), 7.3 - 7.1 (6 H, m), 6.93 (1 H, d, J = 7 Hz), 5.43 (1 H, t, J = 6 Hz), 4.23 (2 H, q, J = 7 Hz), 3.95 (1 H, s), 3.3 - 3.1 (4 H, m), 2.78 (2 H, d, J = 6 Hz), 1.30 (3 H, t, J = 7 Hz)
IR (KBr) [$cm^{-1}$]: 3433, 3182, 1736, 1433, 1030, 748

Melting point: 125.8 - 127.7°C

(Inventive Example 7)

Synthesis of 3-(2-(2-phenylethyl)benzimidazol-4-yl)-3-hydroxypropanoic acid

Methyl 3-(2-(2-phenylethyl)benzimidazol-4-yl)-3-hydroxypropanoate (3.4 g) obtained in Inventive Example 5 was suspended in ethanol (5 mL), and to the suspension was added 1 N sodium hydroxide aqueous solution (10 mL). After stirring for 1 hour at room temperature, the reaction solution was concentrated under a reduced pressure. The thus obtained residue was diluted with water and extracted with ethyl acetate. The water layer was adjusted to pH 5 to 6 with 1 N hydrochloric acid, and the thus precipitate was collected by filtration to obtain the title compound as crystal (3.0 g).
NMR ($CD_3OD$) δ [ppm] : 7.44 (1 H, d, J = 8 Hz), 7.3 - 7.1 (7 H, m), 5.51 (1 H, t, J = 7 Hz), 3.3 - 3.1 (4 H, m), 2.77 (2 H, d, J = 7 Hz)
IR (KBr) [$cm^{-1}$]: 3396, 1635, 1581, 1412, 1389, 754
Melting point: 104.5 - 109.6°C

(Inventive Examples 8 and 9)

Synthesis of (+)-3-(2-(2-phenylethyl)benzimidazol-4-yl)-3-hydroxypropanoic acid (Inventive Example 8) and (-)-3-(2-(2-phenylethyl)benzimidazol-4-yl)-3-hydroxypropanoic acid

(Inventive Example 9)

To a solution of methyl 3-(2-(2-phenylethyl)benzimidazol-4-yl)-3-hydroxypropanoate (2.65 g) obtained in Inventive Example 5 in pyridine (25 mL) was added camphoric acid chloride (3.19 g) while cooling in an ice bath. After stirring for 1.5 hours in an ice bath and subsequent 6 hours of stirring at room temperature, this was mixed with ethyl acetate and 1 N hydrochloric acid. After separation of layers, the organic layer was washed with brine. The organic layer was dried over anhydrous sodium sulfate and then concentrated under a reduced pressure. The thus obtained residue was crystallized from hexane and ether and collected by filtration (4.0 g). The thus obtained crystal was separated (first fraction and second fraction) by a high performance liquid chromatography (ChiralCell OD™, manufactured by Daicel Chemical Industries; eluent: hexane/ethanol). The first fraction (0.80 g) was dissolved in methanol (80 mL) to which, cooled in an ice bath, was subsequently added 2 N sodium hydroxide (20 mL) in small portions. After stirring for 1 hour in an ice bath, the reaction solution was adjusted to pH 6 to 7 with 1 N hydrochloric acid and then extracted with ethyl acetate. The organic layer was combined, washed with brine and then dried over anhydrous sodium sulfate. After concentration of the organic layer under a reduced pressure, the resulting residue was purified by a silica gel column chromatography (eluent: dichloromethane/methanol) to obtain the intended (+)-3-(2-(2-phenylethyl)benzimidazol-4-yl)-3-hydroxypropanoic acid as crystal (0.23 g). In the same manner, the intended (-)-3-(2-(2-phenylethyl)benzimidazol-4-yl)-3-hydroxypropanoic acid was obtained from the second fraction (0.24 g).
(+)-3-(2-(2-Phenylethyl)benzimidazol-4-yl)-3-hydroxypropanoic acid (Inventive Example 8)
NMR ($CD_3OD$) δ [ppm]: 7.44 (1 H, dd, J = 8, 1 Hz), 7.3 - 7.1 (7 H, m), 5.51 (1 H, t, J = 7 Hz), 3.3 - 3.1 (4 H, m), 2.77 (2 H, d, J = 7 Hz)
IR (KBr) [$cm^{-1}$]: 1637, 1581, 1497, 1395, 750, 700
Melting point: 103.0 - 105.3°C
Optical rotation: $[\alpha]_D$ = +12.74° (c 1.01, MeOH)
(-)-3-(2-(2-Phenylethyl)benzimidazol-4-yl)-3-hydroxypropanoic acid (Inventive Example 9)
NMR ($CD_3OD$) δ [ppm]: 7.44 (1 H, dd, J = 8, 1 Hz), 7.3 - 7.1 (7 H, m), 5.51 (1 H, t, J = 7 Hz), 3.3 - 3.1 (4 H, m), 2.77 (2 H, d, J = 7 Hz)
IR (KBr) [$cm^{-1}$]: 1637, 1581, 1497, 1396, 750, 700
Melting point: 100.6 - 102.6°C
Optical rotation: $[\alpha]_D$ = -12.03° (c 1.01, MeOH)

(Inventive Example 10)

Synthesis of ethyl 3-(1-methyl-2-(2-phenylethyl)benzimidazol-4-yl)-3-oxopropanoate

To a solution of ethyl 3-(2-(2-phenylethyl)benzimidazol-4-yl)-3-oxopropanoate (3.2 g) obtained in Inventive Example 3 in acetone (150 mL) was added sodium bicarbonate (2.2 g) and dimethyl sulfate (2.3 mL) and the mixture

was heated under reflux for 4 hours. After allowing the reaction solution to stand overnight, the insoluble material was removed by filtration and the resulting filtrate was concentrated under a reduced pressure. The thus obtained residue was dissolved in dichloromethane and washed with water and brine in that order. The organic layer was dried over anhydrous sodium sulfate, and the solvent was evaporated under a reduced pressure. The resulting residue was purified by a silica gel column chromatography (eluent: dichloromethane) to obtain the title compound as crystal (2.3 g).
NMR ($CDCl_3$) δ [ppm]: 7.95 (1 H, dd, J = 8, 1 Hz), 7.46 (1 H, dd, J = 8, 1 Hz), 7.3 - 7.2 (6 H, m), 4.68 (2 H, s), 4.22 (2 H, q, J = 7 Hz), 3.56 (3 H, s), 3.3 - 3.1 (4 H, m), 1.26 (3 H, t, J = 7 Hz)
IR (KBr) [$cm^{-1}$]: 2941, 1720, 1668, 1466, 1244, 1217, 1036, 756
Melting point: 143.2 - 145.5°C

(Inventive Example 11)

Synthesis of ethyl 3-(1-methyl-2-(2-phenylethyl)benzimidazol-4-yl)-3-hydroxypropanoate

To a suspension of ethyl 3-(1-methyl-2-(2-phenylethyl)benzimidazol-4-yl)-3-oxopropanoate (0.80 g) obtained in Inventive Example 10 in anhydrous methanol (16 mL), was added sodium borohydride in small portions which was cooled in an ice bath. After stirring for 20 minutes at room temperature, the reaction solution was poured into water and concentrated under a reduced pressure. The thus obtained residue was extracted with ethyl acetate, and the organic layer was washed with water and brine in that order. The organic layer was dried over anhydrous sodium sulfate and then the solvent was evaporated under a reduced pressure. The resulting residue was purified by a silica gel column chromatography (eluent: hexane/ethyl acetate) to obtain the title compound as white crystal (0.39 g).
NMR ($CDCl_3$) δ [ppm]: 7.3 - 7.1 (8 H, m), 5.7 - 5.6 (1 H, m), 5.27 (1 H, d, J = 7 Hz), 4.19 (2 H, q, J = 7 Hz), 3.53 (3 H, s), 3.2 - 3.1 (4 H, m), 3.1 - 2.9 (2 H, m), 1.27 (3 H, t, J = 7 Hz)
IR (KBr) [$cm^{-1}$]: 3452, 1730, 1427, 1331, 1284, 1161, 756, 704
Melting point: 126.8 - 127.8°C

(Inventive Example 12)

Synthesis of ethyl 3-(2-(2-phenylethyl)benzimidazol-4-yl)-3-oxopropanoate (compound of Inventive Example 3)

Potassium ethylmalonate (72.4 g) was suspended in anhydrous acetonitrile (1 L). Triethylamine (59.3 mL) and magnesium chloride (48.6 g) were added to the suspension and vigorously stirred at room temperature for 3 hours under an atmosphere of argon. Next, a catalytically effective amount of N,N-dimethylaminopyridine and 1,1'-carbonyldiimidazole (30.3 g) were added to anhydrous tetrahydrofuran (450 mL) suspension of 2-(2-phenylethyl)benzimidazole-4-carboxylic acid (45.3 g) obtained in Reference Example 3, and the mixture was stirred for 2 hours under an atmosphere of argon. The reaction solution was added to the previously prepared suspension of ethyl malonate magnesium salt while cooling in an ice bath, and the mixture was stirred at room temperature for 1 hour. This was adjusted to pH 1 with concentrated hydrochloric acid while cooling in an ice bath. At the same temperature, this was alkalified with saturated sodium bicarbonate aqueous solution and extracted with ethyl acetate. The organic layer was washed with water and brine and dried over anhydrous sodium sulfate, and then the solvent was evaporated under a reduced pressure. The resulting residue was crystallized from hexane and collected by filtration to obtain the title compound as crystal (43 g). Respective spectra and melting point of the thus obtained crystal coincided with the data obtained in Inventive Example 3.

(Inventive Example 13)

Synthesis of ethyl 3-(2-(2-phenylethyl)benzimidazol-4-yl)-3-acetyloxypropionate

Ethyl 3-(2-(2-phenylethyl)benzimidazol-4-yl)-3-hydroxypropionate (3.0 g) obtained in Inventive Example 6 was suspended in dichloromethane (25 mL). While cooling in an ice bath, pyridine (2.2 mL) and acetic anhydride (1.7 mL) were added to the suspension and stirred at the same temperature for 40 minutes. The reaction solution was diluted with dichloromethane, washed with saturated sodium bicarbonate aqueous solution, water and brine and dried over anhydrous sodium sulfate, and then the solvent was evaporated. The resulting residue was purified by a silica gel column chromatography (eluent: hexane/ethyl acetate) and the desired fraction was concentrated to obtain the title compound as colorless crystal (1.14 g).

(Inventive Example 14)

Synthesis of ethyl 3-(2-benzylbenzimidazol-3-yl)-3-oxopropanoate

According to Inventive Example 3, using 2-benzylbenzimidazole-3-oxopropanoic acid (1.3 g) obtained in Reference Example 5, the title compound was obtained as colorless crystal (1.0 g).

(Inventive Example 15)

Synthesis of ethyl 3-(2-benzylbenzimidazol-3-yl)-3-hydroxypropanoate

According to Inventive Example 6, using ethyl 3-(2-benzylbenzimidazol-3-yl)-3-oxopropanoate (0.68 g) obtained in Inventive Example 14, the title compound was obtained as colorless crystals (0.38 g).

(Inventive Example 16)

Synthesis of ethyl 3-(2-phenylbenzimidazol-3-yl)-3-oxopropanoate

According to Inventive Example 12, using 2-phenylbenzimidazole-3-carboxylic acid (1.46 g) obtained in Reference Example 7, the title compound was obtained as oil (1.28 g).

(Inventive Example 17)

Synthesis of ethyl 3-(2-phenylbenzimidazol-3-yl)-3-hydroxypropanoate

According to Inventive Example 6, using ethyl 3-(2-phenylbenzimidazol-3-yl)-3-oxopropanoate (0.80 g) obtained in Inventive Example 16, the title compound was obtained as colorless crystal (0.44 g).

(Inventive Example 18)

Synthesis of ethyl 3-(2-(2-(4-hydroxyphenyl)ethyl)benzimidazol-4-yl)-3-oxopropanoate

Ethyl 3-(2-(2-(4-benzyloxyphenyl)ethyl)benzimidazol-4-yl)-3-oxopropanoate (1.8 g) obtained in Reference Example 10 was dissolved in ethanol (10 mL) and dichloromethane (5 mL), and to the solution was added with acetic acid (1 mL) and 10% palladium/carbon (0.09 g) and the mixture was stirred overnight at room temperature under an atmosphere of hydrogen. After removal of the catalyst by filtration, the resulting filtrate was concentrated under a reduced pressure. The thus obtained residue was diluted with water, adjusted to pH 8 with saturated sodium bicarbonate aqueous solution and then extracted with ethyl acetate. The organic layer was washed with water and brine in that order and then dried over anhydrous sodium sulfate. After evaporation of the solvent under a reduced pressure, the resulting residue was purified by a silica gel column chromatography (eluent: hexane/ethyl acetate) to obtain the title compound as crystal (1.0 g).

(Inventive Example 19)

Synthesis of ethyl 3-(2-(2-(4-hydroxyphenyl)ethyl)benzimidazol-4-yl)-3-hydroxypropanoate

According to Inventive Example 6, using ethyl 3-(2-(2-(4-benzyloxyphenyl)ethyl)benzimidazol-4-yl)-3-oxopropanoate (0.75 g) obtained in Inventive Example 18, the title compound was obtained as colorless crystal (0.50 g).

(Inventive Example 20)

Synthesis of ethyl 3-(2-(2-phenylethyl)benzimidazol-4-yl) -3-aminopropanoate dihydrochloride

To a suspension of ethyl 3-(2-(2-phenylethyl)benzimidazol-4-yl)-3-oxopropanoate (3.5 g) obtained in Inventive Example 12 and O-benzylhydroxylamine hydrochloride (4.15 mL) in methanol was added pyridine (2.1 mL), and the mixture was stirred at room temperature for 16 hours. The mixture was diluted with water and extracted with ethyl acetate. The organic layer was washed with water and brine and dried over anhydrous sodium sulfate, and then the solvent was evaporated under a reduced pressure. The thus obtained residue was crystallized from ether and hexane

and collected by filtration to obtain a benzyloxime compound as colorless crystals. The thus obtained crystal was dissolved in methanol (100 mL) and acetic acid (100 mL). 10% Palladium/carbon (2.5 g) was added to the resulting solution and the mixture was stirred for 16 hours under an atmosphere of hydrogen. The catalyst was removed by filtration, and the resulting filtrate was alkalified with saturated sodium bicarbonate aqueous solution and then extracted with dichloromethane. The organic layer was washed with brine and dried over anhydrous sodium sulfate, and then the solvent was evaporated. Ethyl 3-(2-(2-phenylethyl)benzimidazol-4-yl)-3-aminopropanoate was obtained as the resulting residue of oil (3.0 g). The thus obtained oil was dissolved in hydrochloric acid/ethanol and concentrated. This was crystallized from ether and collected by filtration to obtain the title compound.

(Inventive Example 21)

Synthesis of ethyl 3-(2-(2-phenylethyl)benzimidazol-4-yl) -3-acetylaminopropanoate

To a solution of ethyl 3-(2-(2-phenylethyl)benzimidazol-4-yl)-3-aminopropanoate (400 mg) obtained in Inventive Example 20 in dichloromethane (50 mL) was added pyridine (0.096 mL), cooled in an ice bath and then added acetyl chloride (0.084 mL). The mixture was diluted with water and extracted with dichloromethane. The organic layer was washed with brine and dried with anhydrous sodium sulfate, and then the solvent was evaporated. The resulting residue was purified by a silica gel column chromatography (eluent: hexane/ethyl acetate) and the desired fraction was concentrated to obtain the title compound as crystal (330 mg).

(Inventive Example 22)

Synthesis of ethyl 3-(2-(2-phenylethyl)benzimidazol-4-yl)-3-benzenesulfonylaminopropanoate hydrochloride

According to Inventive Example 21, ethyl 3-(2-(2-phenylethyl)benzimidazol-4-yl)-3-aminopropanoate (400 mg) obtained in Inventive Example 20 and benzenesulfonyl chloride (0.15 mL) were reacted, and the thus obtained oil was dissolved in hydrochloric acid/ethanol. The solvent was evaporated under a reduced pressure, and the resulting residue was crystallized from ether and collected by filtration to obtain the title compound as crystal (110 mg).

(Inventive Example 23)

Synthesis of ethyl 3-(2-(2-phenylethyl)benzimidazol-4-yl) -3-(2-methoxybenzoylamino)propanoate

According to Inventive Example 21, using ethyl 3-(2-(2-phenylethyl)benzimidazol-4-yl)-3-aminopropanoate (500 mg) obtained in Reference Example 20 and 2-methoxybenzoyl chloride (0.22 mL), the title compound was obtained (530 mg).

(Inventive Example 24)

Synthesis of 3-(2-(2-phenylethyl)benzimidazol-4-yl)-3-oxopropanoic acid dimethylamide

Pyridine (0.365 mL) and magnesium chloride (215 mg) were added to dry dichloromethane (2.2 mL) solution of N,N-dimethylacetamide, and the mixture was vigorously stirred at room temperature for 1.5 hours under an atmosphere of argon. Next, a catalytically effective amount of N,N-dimethylaminopyridine and 1,1'-carbonyldiimidazole (134 mg) were added to anhydrous tetrahydrofuran (2 mL) suspension of 2-(2-phenylethyl)benzimidazole-4-carboxylic acid (0.2 g) obtained in Reference Example 3, and the mixture was stirred for 1.5 hours under an atmosphere of argon. The reaction solution was added to the previously prepared N,N-dimethylacetamide suspension of magnesium salt while cooling in an ice bath, and the mixture was stirred at room temperature for 2 hours. This was diluted with ice water and adjusted to pH 1 with 1 N hydrochloric acid while cooling in an ice bath. At the same temperature, this was neutralized with saturated sodium bicarbonate aqueous solution and extracted with ethyl acetate. The organic layer was washed with brine and dried over anhydrous sodium sulfate, and then the solvent was evaporated under a reduced pressure. The resulting residue was purified by a silica gel column chromatography (eluent: hexane/ethyl acetate) and the desired fraction was concentrated to obtain the title compound as colorless crystal (43 mg).

(Inventive Example 25)

Synthesis of 3-(2-(2-phenylethyl)benzimidazol-4-yl)-3-oxopropionitrile

Triethylamine (5.2 mL) and magnesium chloride (4.3 g) were added to dry acetonitrile (30 mL) solution of cyanoacetic acid (1.6 g), and the mixture was vigorously stirred at room temperature for 23 hours under an atmosphere of argon. Next, a catalytically effective amount of N,N-dimethylaminopyridine and 1,1'-carbonyldiimidazole (1.34 g) were added to anhydrous tetrahydrofuran (20 mL) suspension of 2-(2-phenylethyl)benzimidazole-4-carboxylic acid (2.0 g) obtained in Reference Example 3, and the mixture was stirred for 2.5 hours under an atmosphere of argon. The reaction solution was added to the previously prepared cyanoacetic acid suspension of magnesium salt while cooling in an ice bath, and the mixture was stirred at room temperature for 2 hours. The reaction mixture was diluted with ice water (30 mL) and, at the same temperature, adjusted to pH 1 with 4 N hydrochloric acid. At the same temperature, this was adjusted to pH 9 with saturated sodium bicarbonate aqueous solution and extracted with ethyl acetate. The organic layer was washed with water and brine and dried over anhydrous sodium sulfate, and then the solvent was evaporated under a reduced pressure. The resulting residue was purified by a silica gel column chromatography (eluent: hexane/ethyl acetate) and the desired fraction was concentrated to obtain the title compound as colorless crystal (1.03 g).

(Inventive Example 26)

Synthesis of 3-(2-(2-phenylethyl)benzimidazol-4-yl)-3-hydroxypropionitrile

To a suspension of 3-(2-(2-Phenylethyl)benzimidazol-4-yl)-3-oxopropionitrile (400 mg) obtained in Inventive Example 25 in ethanol (4.8 mL) while cooling in an ice bath, was added sodium borohydride (26 mg), and the mixture was stirred at the same temperature for 1 hour. The reaction solution was poured into ice water (20 mL) and extracted with ethyl acetate. The organic layer was washed with water and brine and dried over anhydrous sodium sulfate, and then the solvent was evaporated under a reduced pressure. The resulting residue was crystallized from ether and collected by filtration to obtain the title compound as crystal (297 mg).

(Inventive Example 27)

Synthesis of 3-(2-(2-phenylethyl)benzimidazol-4-yl)-3-hydroxypropanoic acid amide maleate

Ethyl 3-(2-(2-phenylethyl)benzimidazol-4-yl)-3-hydroxypropanoate (100 mg) obtained in Inventive Example 6 was dissolved in 20% ammonia/methanol (5 mL), and the solution was stirred at room temperature for 1 hour. Concentrated aqueous ammonia (5 mL) was added to the mixture and the mixture was stirred at room temperature for 19 hours. Methanol was evaporated, and the thus obtained residue was extracted with ethyl acetate. After drying over anhydrous sodium sulfate, the solvent was evaporated under a reduced pressure. The thus obtained residue was dissolved in ethyl acetate/ethanol and maleic acid (24 mg) was added to the mixture. After evaporation of the solvent under a reduced pressure, the resulting residue was crystallized from ether and collected by filtration to obtain the title compound as colorless crystal (68 mg).

(Inventive Example 28)

Synthesis of cis-3-(2-(2-phenylethyl)benzimidazol-4-yl)-2-propenoic acid

2-(2-Phenylethyl)-4H-imidazo[4,5,1-i,j]-quinolin-4-on (1.0 g) obtained in Reference Example 13 was dissolved in tetrahydrofuran (30 mL) and methanol (5 mL), and 10% sodium hydroxide aqueous solution (15 mL) was added in small portions to the solution which was cooled in an ice bath. After stirring for 2 hours at room temperature, the reaction solution was diluted with water (20 mL). This was adjusted to pH 2 using 1 N hydrochloric acid, and the thus precipitate was collected by filtration to obtain the title compound as colorless crystal (0.84 g).

(Inventive Example 29)

Synthesis of 3-(2-(2-phenylethyl)benzimidazol-4-yl)-propanoic acid

To a solution of cis-3-(2-(2-Phenylethyl)benzimidazol-4-yl)-2-propenoic acid (1.0 g) obtained in Inventive Example 28 in methanol (20 mL) was added 10% palladium/carbon (100 mg) and the mixture was stirred at room temperature for 2 hours under an atmosphere of hydrogen. The catalyst was removed by filtration and the resulting filtrate was concentrated. The resulting residue was crystallized from ethyl acetate and hexane and collected by filtration to obtain the title compound as colorless crystal (0.86 g).

(Inventive Example 30)

Synthesis of ethyl trans-3-(2-(2-phenylethyl)benzimidazol-4-yl)propenoate

To a solution of ethyl 3-(2-(2-phenylethyl)benzimidazol-4-yl)-3-hydroxypropionate (3.0 g) obtained in Inventive Example 6 in dichloromethane (50 mL) was added pyridine (1.08 mL). While cooling in an ice bath, thionyl chloride (0.87 mL) was added to the mixture, and the mixture was stirred at the same temperature for 5 minutes. The reaction solution was diluted with water, alkalified with saturated sodium bicarbonate aqueous solution and then extracted with dichloromethane. The organic layer was washed with water and brine and dried over anhydrous sodium sulfate, and then the solvent was evaporated under a reduced pressure. The thus obtained oil was dissolved in dichloromethane (50 mL). While cooling in an ice bath, this was mixed with 1,8-diazabicyclo[5.4.0]-7-undecene (1.34 mL) was added to the mixture and the mixture was stirred at the same temperature for 5 minutes. The reaction solution was diluted with dichloromethane, washed with water and brine and dried over anhydrous sodium sulfate, and then the solvent was evaporated under a reduced pressure. The resulting residue was purified by a silica gel column chromatography (eluent: hexane/ethyl acetate) and the title fraction was concentrated to obtain the desired compound as colorless crystal (2.0 g).

(Inventive Example 31)

Synthesis of 3-(2-(2-phenylethyl)benzimidazol-4-yl)-3-hydroxypropanoylglycine ethyl ester

3-(2-(2-Phenylethyl)benzimidazol-4-yl)-3-hydroxypropanoate (310 mg) obtained in Inventive Example 7, benzotriazol-1-yloxytris(dimethylamino)phosphonium hexafluorophosphate (490 mg) and ethylglycine hydrochloride (160 mg) were suspended in tetrahydrofuran (3 mL). While cooling in an ice bath, triethylamine (0.31 mL) was added to the mixture and the mixture was stirred at room temperature for 1 hour. The reaction solution was diluted with water (5 mL) and extracted with ethyl acetate. The organic layer was washed with water and brine and dried over anhydrous sodium sulfate, and then the solvent was evaporated under a reduced pressure. The resulting residue was purified by a silica gel column chromatography (eluent: hexane/ethyl acetate). The desired fraction was concentrated, and the resulting residue was crystallized from ether and collected by filtration to obtain the title compound as colorless crystal (170 mg).

(Inventive Example 32)

Synthesis of 3-(2-(2-phenylethyl)benzimidazol-4-yl)-3-hydroxypropanoyl(L)-phenylalanine ethyl ester

3-(2-(2-Phenylethyl)benzimidazol-4-yl)-3-hydroxypropanoate (310 mg) obtained in Inventive Example 7, benzotriazol-1-yloxytris(dimethylamino)phosphonium hexafluorophosphate (490 mg) and ethyl(L)-phenylalanine hydrochloride (252 mg) were dissolved in N,N-dimethylformamide (3 mL). While cooling in an ice bath, triethylamine (0.31 mL) was added to the mixture and the mixture was stirred at 70 to 80°C for 2 hours. The reaction solution was diluted with water and extracted with ethyl acetate. The organic layer was washed with saturated sodium bicarbonate aqueous solution, 5% citric acid aqueous solution and brine and dried over anhydrous sodium sulfate, and then the solvent was evaporated under a reduced pressure. The resulting residue was purified by a silica gel column chromatography (eluent: hexane/ethyl acetate). The desired fraction was concentrated, and the resulting residue was crystallized from ethyl acetate/ether and collected by filtration to obtain the title compound as crystal (200 mg).

(Inventive Example 33)

Synthesis of 3-(2-(2-phenylethyl)benzimidazol-4-yl)-3-hydroxypropanoyl(D)-phenylalanine ethyl ester

3-(2-(2-Phenylethyl)benzimidazol-4-yl)-3-hydroxypropanoate (310 mg) obtained in Inventive Example 7, benzotriazol-1-yloxytris(dimethylamino)phosphonium hexafluorophosphate (490 mg) and ethyl(D)-phenylalanine hydrochloride (252 mg) were dissolved in N,N-dimethylformamide (3 mL). While cooling in an ice bath, triethylamine (0.31 mL) was added to the mixture and the mixture was stirred at 70 to 80°C for 2 hours. The reaction solution was diluted with water and extracted with ethyl acetate. The organic layer was washed with saturated sodium bicarbonate aqueous

solution, citric acid aqueous solution and brine and dried over anhydrous sodium sulfate, and then the solvent was evaporated under a reduced pressure. The resulting residue was purified by a silica gel column chromatography (eluent: hexane/ethyl acetate). The desired fraction was concentrated and the resulting residue was crystallized from ethyl acetate/ether. By recrystallizing from ethanol/ethyl acetate and collecting by filtration, the title compound was obtained as colorless crystal (340 mg).

(Inventive Example 34)

Synthesis of dimethyl 3-((2-(2-phenylethyl)benzimidazol-4-yl)methoxymethyl)phthalate

To a solution of dimethyl (1-(4-methoxybenzyl)-3-(2-(2-phenylethyl)benzimidazol-4-yl)methoxymethyl)phthalate (970 mg) obtained in Reference Example 16 in acetonitrile:water = 9:1 (10 mL) was added ammonium cerium nitrate (5.0 g), and the mixture was stirred at room temperature for 16 hours. The reaction solution was diluted with water and extracted with ethyl acetate. The organic layer was washed with brine and dried over anhydrous sodium sulfate, and then the solvent was evaporated under a reduced pressure. The resulting residue was purified by a silica gel column chromatography (eluent: hexane/ethyl acetate) and the desired fraction was concentrated under a reduced pressure to obtain the title compound as oil (630 mg).

(Inventive Example 35)

Synthesis of 3-((2-(2-phenylethyl)benzimidazol-4-yl)methoxymethyl)phthalic acid

To a solution of dimethyl 3-((2-(2-phenylethyl)benzimidazol-4-yl)methoxymethyl)phthalate (1.2 g) obtained in Inventive Example 34 in methanol was added water (10 mL) solution of potassium hydroxide (0.6 g), and the mixture was heated under reflux for 13 hours. Methanol was evaporated under a reduced pressure and the thus obtained residue was adjusted to pH 5 to 6 with 1 N hydrochloric acid. The thus precipitate was collected by filtration and washed with ethanol and ether to obtain the title compound as colorless crystal (730 mg).

(Inventive Example 36)

Synthesis of ethyl 2-((2-(2-phenylethyl)benzimidazol-4-yl)methoxymethyl)benzoate

According to Reference Example 16 and Inventive Example 34, using 4-hydroxymethyl-1-p-methoxybenzyl-2-(2-phenylethyl)benzimidazole (1.0 g) obtained in Reference Example 15 and ethyl 2-bromomethylbenzoate (550 mg) the title compound was obtained as oil (470 mg).

(Inventive Example 37)

Synthesis of 2-((2-(2-phenylethyl)benzimidazol-4-yl)methoxymethyl)benzoic acid

According to Inventive Example 35, using ethyl 2-((2-(2-phenylethyl)benzimidazol-4-yl)methoxymethyl)benzoate (350 mg) obtained in Inventive Example 36, the title compound was obtained as crystal (280 mg).

(Inventive Example 38)

Synthesis of ethyl 4-((2-(2-phenylethyl)benzimidazol-4-yl)methoxymethyl)benzoate

According to Reference Example 16 and Inventive Example 34, using 4-hydroxymethyl-1-(4-methoxybenzyl)-2-(2-phenylethyl)benzimidazole (2.01 g) obtained in Reference Example 15 and ethyl 2-bromomethylbenzoate (1.36 g), the reaction was carried out. The thus obtained oil was mixed with 10% hydrochloric acid/ether and crystallized with ether to obtain the title compound (450 mg).

(Inventive Example 39)

Synthesis of methyl 2-((2-(2-phenylethyl)benzimidazol-4-yl)methoxymethyl)benzoate hydrochloride

According to Reference Example 16 and Inventive Example 34, using 4-hydroxymethyl-1-(4-methoxybenzyl)-2-(2-phenylethyl)benzimidazole (2.54 g) obtained in Reference Example 15 and methyl 3-bromomethylbenzoate (1.78

g), the reaction was carried out. The thus obtained oil was dissolved in 10% hydrochloric acid/methanol, the solvent was evaporated under a reduced pressure and then the resulting residue was crystallized from ether to obtain the title compound as crystal (468 mg).

(Inventive Example 40)

Synthesis of ethyl 3-(6-methoxy-2-(2-phenylethyl)benzimidazol-4-yl)-3-oxopropanoate

Triethylamine (1.3 mL) and magnesium chloride (1.06 g) were added to anhydrous acetonitrile (15 mL) suspension of potassium ethylmalonate (1.58 g), and the mixture was vigorously stirred at room temperature for 18 hours under an atmosphere of argon. Next, a catalytically effective amount of N,N-dimethylaminopyridine and N,N-carbonyldiimidazole (663 mg) were added to anhydrous tetrahydrofuran (12 mL) suspension of 6-methoxy-2-(2-phenylethyl) benzimidazole-4-carboxylic acid (1.1 g) obtained in Reference Example 21, and the mixture was stirred for 2 hours under an atmosphere of argon. The reaction solution was added to the previously prepared suspension of ethyl malonate magnesium salt while cooling in an ice bath, and the mixture was stirred at room temperature for 2 hours. While cooling in an ice bath, this was diluted with water and adjusted to pH 1 with 4 N hydrochloric acid. At the same temperature, this was adjusted to pH 7 to 8 with saturated sodium bicarbonate aqueous solution and extracted with ethyl acetate. The organic layer was washed with brine and dried over anhydrous sodium sulfate, and then the solvent was evaporated under a reduced pressure. The resulting residue was purified by a silica gel column chromatography (eluent: hexane/ethyl acetate) and the desired fraction was concentrated to obtain the title compound as crystal (620 mg).

(Inventive Example 41)

Synthesis of ethyl 3-(6-methoxy-2-(2-phenylethyl)benzimidazol-4-yl)-3-hydroxypropanoate

To a solution of ethyl 3-(6-methoxy-2-(2-phenylethyl)benzimidazol-4-yl)-3-oxopropanoate (400 mg) obtained in Inventive Example 40 in anhydrous tetrahydrofuran (16 mL), while cooling in an ice bath, was added sodium borohydride (12.4 mg) and the mixture was stirred at room temperature for 3.5 hours. The reaction solution was diluted with ice water, extracted with ethyl acetate and then washed with brine. After drying the organic layer over anhydrous sodium sulfate, the solvent was evaporated under a reduced pressure. The resulting residue was purified by a silica gel column chromatography (eluent: hexane/ethyl acetate). Thereafter, the desired fraction was concentrated to obtain the title compound as colorless crystal (110 mg).

(Inventive Example 42)

Synthesis of ethyl 3-(6-hydroxy-2-(2-phenylethyl)benzimidazol-4-yl)-3-oxopropanoate

Ethyl 3-(6-t-butyldimethylsilyloxy-2-(2-phenylethyl)benzimidazol-4-yl)-3-oxopropanoate (3.03 g) obtained in Reference Example 24 was dissolved in anhydrous tetrahydrofuran (31 mL). At a temperature of -40°C, to this was added dropwise 1.0 M tetrahydrofuran solution (7.1 mL) of tetra-(n-butyl)ammonium fluoride. After stirring for 30 minutes at the same temperature, this was mixed with ice water (20 mL) and adjusted to pH 5 with 4 N hydrochloric acid. This was extracted with ethyl acetate and washed with water and brine. After drying over anhydrous sodium sulfate, the solvent was evaporated. The resulting residue was purified by a silica gel column chromatography (eluent: hexane/ethyl acetate) and the desired fraction was concentrated. The resulting residue was crystallized from ethyl acetate/ether and collected by filtration to obtain the title compound as crystal (1.71 g).

(Inventive Example 43)

Synthesis of ethyl 3-(6-hydroxy-2-(2-phenylethyl)benzimidazol-4-yl)-3-hydroxypropanoate hydrochloride

To a suspension of ethyl 3-(6-hydroxy-2-(2-phenylethyl)benzimidazol-4-yl)-3-oxopropanoate (800 mg) obtained in Inventive Example 42 in anhydrous tetrahydrofuran (16 mL), while cooling in an ice bath, was added sodium borohydride (26 mg) and the mixture was stirred at room temperature for 3 hours. The reaction solution was diluted with ice water (10 mL) and adjusted to pH 1 with 4 N hydrochloric acid. This was neutralized with saturated sodium bicarbonate aqueous solution, extracted with ethyl acetate and then washed with brine. The organic layer was dried over anhydrous sodium sulfate and then the solvent was evaporated under a reduced pressure. The resulting residue was purified by a silica gel column chromatography (eluent: hexane/ethyl acetate). The desired fraction was concentrated and dissolved in hydrochloric acid/ethanol. The solvent was replaced by ether and then ether was evaporated to obtain the title compound as colorless crystal (350 mg).

(Inventive Example 44)

Synthesis of 1,3-dihydroxypropyl-6-hydroxy-2-(2-phenylethyl)benzimidazole

To a suspension of Ethyl 3-(6-hydroxy-2-(2-phenylethyl)benzimidazol-4-yl)-3-oxopropanoate (500 mg) obtained in Inventive Example 42 in anhydrous ethanol (10 mL), while cooling in an ice bath, was added sodium borohydride (81 mg), and the mixture was stirred at room temperature for 2 hours. The reaction solution was diluted with ice water (20 mL) and extracted with ethyl acetate. The organic layer was washed with water and brine and dried over anhydrous sodium sulfate, and then the solvent was evaporated under a reduced pressure. The resulting residue was crystallized from ether to obtain the desired compound as colorless crystal (424 mg).

(Inventive Example 45)

Synthesis of 1,3-dihydroxypropyl-2-(2-phenylethyl)benzimidazole

Using ethyl 3-(2-(2-phenylethyl)benzimidazol-4-yl)-3-oxopropanoate obtained in Inventive Example 12, the procedure of Inventive Example 44 was repeated to obtain the title compound.

(Inventive Example 46)

Synthesis of ethyl 3-(6-hydroxy-2-(2-phenylethyl)benzimidazol-4-yl)propenoate

To a solution of ethyl 3-(6-t-butyldimethylsilyloxy-2-(2-phenylethyl)benzimidazol-4-yl)propenoate (318 mg) obtained in Reference Example 26 in anhydrous tetrahydrofuran (4 mL), while cooling in an ice bath, was added 1.0 M tetrahydrofuran solution (0.85 mL) of tetra-(n-butyl)ammonium fluoride, and the mixture was stirred at the same temperature for 10 minutes. The reaction solution was diluted with water and adjusted to pH 5 with 4 N hydrochloric acid. This was adjusted to pH 7 to 8 with saturated sodium bicarbonate aqueous solution and extracted with ethyl acetate. The organic layer was washed with water and brine and dried over anhydrous sodium sulfate, and then the solvent was evaporated under a reduced pressure. The resulting residue was crystallized from hexane and collected by filtration to obtain the title compound as crystal (200 mg).

(Inventive Example 47)

Synthesis of ethyl 3-(6-chloro-2-(2-phenylethyl)benzimidazol-4-yl)-3-oxopropanoate

According to Inventive Example 12, using 6-chloro-2-(2-phenylethyl)benzimidazole-4-carboxylic acid (2.8 g) obtained in Reference Example 28, the title compound was obtained as crystal (2.37 g).

(Inventive Example 48)

Synthesis of ethyl 3-(6-chloro-2-(2-phenylethyl)benzimidazol-4-yl)-3-hydroxopropanoate

According to Inventive Example 6, using ethyl 3-(6-chloro-2-(2-phenylethyl)benzimidazol-4-yl)-3-oxopropanoate (1.5 g) obtained in Inventive Example 47, the title compound was obtained as crystal (620 mg).

(Inventive Example 49)

Synthesis of 4-(3-(imidazol-2-yl)-2-propenoyl)-2-(2-phenylethyl)benzimidazole

Ethanol (15 mL) was added to 4-acetyl-2-(2-phenylethyl)benzimidazole (1 g) obtained in Reference Example 1 and 2-imidazolecarboxyaldehyde (304 mg). Sodium hydroxide (405 mg) was added to the mixture and the mixture was stirred overnight at room temperature. The reaction solution was poured into ice water, adjusted to pH 8 with 4 N hydrochloric acid and then extracted with ethyl acetate. The organic layer was washed with water and brine in that order and then dried over anhydrous sodium sulfate. After evaporation of the solvent under a reduced pressure, the resulting residue was purified by a silica gel column chromatography (eluent: hexane/ethyl acetate) to obtain the title

compound (0.35 g).

(Inventive Example 50)

Synthesis of 2-(2-(2-phenylethyl)benzimidazol-4-yl)-2-hydroxyacetic acid

To a solution of 4-formyl-2-(2-phenylethyl)benzimidazole (1.0 g) obtained in Reference Example 30 in dry dichloromethane was added trimethylsilyl cyanide (0.64 mL) and cerium chloride (100 mg), and the mixture was stirred at room temperature for 16 hours. The insoluble material was removed by filtration and the resulting filtrate was concentrated under a reduced pressure. To the solution of thus obtained residue was dissolved in ethanol (1.7 mL) was added concentrated hydrochloric acid (3.3 mL), and the mixture was heated under reflux for 13 hours. The reaction solution was adjusted to pH 5 with 1 N sodium hydroxide. The supernatant was discarded by decantation, and the resulting residue was crystallized by adding ether/methanol and collected by filtration to obtain the title compound as colorless crystal (510 mg).

(Inventive Example 51)

Synthesis of ethyl 2-(2-(2-phenylethyl)benzimidazol-4-yl)-2-hydroxyacetate

To the suspension of 2-(2-(2-Phenylethyl)benzimidazol-4-yl)-2-hydroxyacetic acid (400 mg) obtained in Inventive Example 50 in ethanol (4 mL), while cooling in an ice bath, was added 20% hydrochloric acid/ethanol (4 mL), and the mixture was stirred at room temperature for 40 hours. The reaction solution was poured into ice water (20 mL) and adjusted to pH 9 with saturated sodium bicarbonate aqueous solution. Thus precipitate was collected by filtration and the resulting filtrate was extracted with ethyl acetate. The organic layer was combined with the collected crystals and dissolved by adding dichloromethane/ethanol, and then the solution was washed with brine. The organic layer was dried over anhydrous sodium sulfate, and the solvent was evaporated under a reduced pressure. The resulting residue was crystallized from ethanol/ethyl acetate and collected by filtration to obtain the title compound as crystal (341 mg).

(Inventive Example 52)

Synthesis of ethyl 3-(5-fluoro-2-(2-phenylethyl)benzimidazol-4-yl)-3-oxopropanoate

According to Inventive Example 12, using 5-fluoro-2-(2-phenylethyl)benzimidazole-4-carboxylic acid (1.7 g) obtained in Reference Example 32, the title compound was obtained as crystal (1.78 g).

(Inventive Example 53)

Synthesis of ethyl 3-(5-fluoro-2-(2-phenylethyl)benzimidazol-4-yl)-3-hydroxypropanoate

According to Inventive Example 6, using ethyl 3-(5-fluoro-2-(2-phenylethyl)benzimidazol-4-yl)-3-oxopropanoate (1.2 g) obtained in Inventive Example 52, the title compound was obtained as colorless crystal (0.50 g).

Structural formulae of the compounds synthesized in reference examples and examples according to the invention are shown in Figs. 1 to 8. The number attached to each structural formula means the number of example according to the invention in which the corresponding compound was synthesized, and this number was used as the compound number. Data on physiological properties of the compounds synthesized in Inventive Examples 13 to 53, including NMR spectrum, IR spectrum and melting point, are shown in Table 2.

Table 2

| Example No. | IR $cm^{-1}$ | NMR ppm | M.P. (°C) |
|---|---|---|---|
| 13 | KBr: 1747, 1736, 1416, 1238, 1217, 1180, 1024, 750 | $CDCl_3$: 7.8-7.6 (1H, m), 7.4-7.2 (8H, m), 6.6-6.5 (1H, m), 4.15 (2H, q, J=7Hz), 3.3-2.8 (6H, m), 2.05 (3H, s), 123 (3H, t J=7Hz) | 96.4-97.7 |

Table 2 (continued)

| Example No. | IR $cm^{-1}$ | NMR ppm | M.P. (°C) |
|---|---|---|---|
| 14 | KBr: 3496, 1718, 1672, 1311, 1275, 1153, 1111, 1032 | $CDCl_3$: 10.52 (1H, bs), 8.00 (1H, d, J=8Hz), 7.71 (1H, d, J=8Hz), 7.4-7.3 (6H, m), 4.33 (2H, s), 423 (2H, q, J=7Hz), 4.07 (2H, s), 1.27 (3H, t, J=7Hz) | 85.6-89.9 |
| 15 | KBr: 1736, 1435, 1425, 1421, 1284, 1165, 1032, 750 | DMSO-$d_6$: 12.28 (1H, bs), 7.5-7.0 (8H, m), 5.67 (0.5H, d, J=4Hz), 5.7-5.5 (0.5H, m), 5.45 (0.5H, d, J=5Hz), 5.4-52 (0.5H, m), 4.19(2H, s), 4.2-3.9 (2H, m), 3.0-2.5 (2H, m), 1.3-1.1 (3H, m) | 123.7-124.8 |
| 16 | neat: 2981, 1740, 1734, 1666, 1477, 1454, 1367, 1302, 1286, 692 | $CDCl_3$: 11.16 (1H, bs), 8.2-8.0 (3H, m), 7.77 (1H, d, J=8Hz), 7.6-7.5 (3H, m), 7.36 (1H, t J=8Hz), 4.27 (2H, q, J=7Hz), 4.14 (2H, s), 1.30 (3H, t, J=7Hz) | Oil |
| 17 | KBr: 3458, 3136, 1734, 1458, 1255, 756 | DMSO-$d_6$: 12.95 (0.7H, bs), 12.57 (0.3H, bs), 8.4-8.1 (2H, m), 7.6-7.1 (6H, m), 5.8-5.5 (2H, m), 4.2-4.0 (2H, m), 3.2-2.5 (2H, m), 1.3-1.1 (3H, m) | 154.7-156.0 |
| 18 | KBr: 3340, 1726, 1680, 1539, 1516, 1275, 1192, 1111, 739 | $CDCl_3$: 10.56 (1H, bs), 7.96 (1H, d, J=8Hz), 7.72 (1H, d, J=8Hz), 7.31 (1H, t J=8Hz), 7.04 (2H, d, J=8Hz), 6.70 (2H, d, J=8Hz), 6.09 (1H, bs), 4.25 (2H, q, J=7Hz), 4.10 (2H, s), 3.24 (2H, t, J=7Hz), 3.12 (2H, t, J=7Hz), 129 (3H, t J=7Hz) | 149.5-152.1 |
| 19 | KBr: 3462, 3429, 3408, 3392, 3365, 3342, 1713, 1516, 1024 | DMSO-$d_6$: 12.25 (0.5H, bs), 12.10 (0.5H, bs), 9.18 (1H, s), 7.5-7.0 (5H, m), 6.7-6.6 (2H, m), 5.66 (0.5H, d, J=4Hz), 5.6-5.5 (0.5H, m), 5.45 (0.5H, d, J=5Hz), 5.3-5.2 (0.5H, m), 4.2-4.0 (2H, m), 3.1-2.9 (4H, m), 2.9-2.5 (2H, m), 1.3-1.1(3H, m) | 167.8-171.3 |
| 20 | KBr: 3408, 2872, 2623, 1741, 1497, 1205 | DMSO-$d_6$: 8.98 (2H, bs), 7.8-7.7 (2H, m), 7.54 (1H, t J=8Hz), 7.3-7.1 (5H, m), 5.4-5.3 (1H, m), 4.0-3.8 (2H, m), 3.6-3.2 (6H, m), 0.97 (3H, t, J=7Hz) | 199.5 (dec.) |
| 21 | KBr: 3105, 3028, 1734, 1635, 1551, 1433, 1375, 1281, 748 | DMSO-$d_6$: 12.31 (0.7H, bs), 12.21 (0.3H, bs), 8.46 (0.3H, d, J=8Hz), 8.38 (0.7H, d, J=9Hz), 7.5-7.0 (8H, m), 5.8-5.7 (0.7H, m), 5.6-5.5 (0.3H, m), 4.1-4.0 (2H, m), 3.1-3.0 (5H, m), 2.8-2.6 (1H, m), 1.2-1.1 (3H, m) | 159.6-162.1 |
| 22 | KBr: 1734, 1633, 1448, 1329, 1163, 1092, 752 | DMSO-$d_6$: 8.80 (1H, d, J=9Hz), 7.44 (1H, d, J=8Hz), 7.4-7.2 (11H, m), 7.05 (1H, t, J=8Hz), 5.16 (1H, q, J=8Hz), 3.91 (2H, q, J=7Hz), 3.44 (2H, t, J=8Hz), 3.25 (2H, t J=8Hz), 3.1-2.8 (2H, m), 1.00 (3H, t, J=7Hz) | 75.0-79.0 |

Table 2 (continued)

| Example No. | IR $cm^{-1}$ | NMR ppm | M.P. (°C) |
|---|---|---|---|
| 23 | KBr: 3201, 1730, 1637, 1533, 1483, 1244, 756 | DMSO-$d_6$: 1241 (1H, bs), 9.43 (1H, d, J=9Hz), 7.87 (1H, dd, J=8, 2Hz), 7.5-7.0 (11H, m), 5.9-5.8 (1H, m), 3.98 (2H, q, J=7Hz), 3.90 (3H, s), 3.2-3.0 (6H, m), 1.05 (3H, t, J=7Hz) | 52.0-56.6 |
| 24 | KBr: 3300, 1659, 1633, 1427, 1271, 1122, 752 | $CDCl_3$: 10.81 (1H, bs), 7.98 (1H, d, J=8Hz), 7.83 (1H, d, J=8Hz), 7.4-7.2 (6H, m), 4.20 (2H, s), 3.3-3.2 (4H, m), 3.11 (3H, s), 3.03 (3H, s) | 97.3-101.7 |
| 25 | KBr: 3028, 2918, 2260, 1682, 1525, 1269, 1113, 752 | $CDCl_3$: 10.46 (1H, bs), 8.04 (1H, d, J=8Hz), 7.63 (1H, d, J=8Hz), 7.4-7.2 (6H, m), 4.21 (2H, s), 3.4-3.2 (4H, m) | 144.9-145.5 |
| 26 | KBr: 3138, 2927, 2684, 2254, 1622, 1543, 1427, 1076, 1043, 762 | DMSO-$d_6$: 12.35 (0.6H, bs), 12.16 (0.4H, bs), 7.5-7.1(8H, m), 6.14 (0.4H, d, J=4Hz), 5.99 (0.6H, d, J=5Hz), 5.5-5.4 (0.6H, m), 5.3-5.2 (0.4H, m), 3.12 (4H, s), 3.2-2.8 (2H, m) | 136.3-138.0 |
| 27 | KBr: 3390, 3213, 1662, 1585, 1497, 1387, 1194, 1001, 702 | DMSO-$d_6$: 7.7-7.5 (1H, m), 7.4-7.2 (8H, m), 6.91 (1H, bs), 6.12 (2H, s), 5.73 (1H, bs), 5.5-5.4 (1H, m), 3.30 (2H, t, J=8Hz), 3.14 (2H, t, J=8Hz), 2.6-2.5 (2H, m) | 118.6-121.8 |
| 28 | KBr: 3431, 3114, 2914, 2742, 1687, 1647, 1572, 1250, 822, 752 | DMSO-$d_6$: 7.73 (1H, d, J=8Hz), 7.67 (1H, d, J=8Hz), 7.48 (1H, t, J=8Hz), 7.36 (1H, d, J=12Hz), 7.3-7.2 (5H, m), 6.31 (1H, d, J=12Hz), 3.42 (2H, t, J=8Hz), 3.20 (2H, t, J=8Hz) | 98.8-100.9 |
| 29 | KBr: 2858, 1734, 1633, 1572, 1491, 1394, 1192, 1169, 752 | DMSO-$d_6$: 14.94 (1H, bs), 7.61 (1H, d, J=8Hz), 7.46 (1H, t, J=8Hz), 7.4-7.2 (6H, m), 3.44 (2H, t, J=8Hz), 3.22 (2H, t, J=8Hz), 3.15 (2H, t, J=8Hz), 2.69 (2H, t, J=8Hz) | 1712-173.7 |
| 30 | KBr: 3273, 2980, 1699, 1632, 1535, 1423, 1321, 1211, 1184 | $CDCl_3$: 8.02 (1H, d, J=16Hz), 7.6-6.9 (9H, m), 4.29 (2H, q, J=7Hz), 3.4-32 (4H, m), 1.35 (3H, t, J=7Hz) | 132.1-133.9 |
| 31 | KBr: 3290, 1749, 1737, 1649, 1545, 1425, 1201, 1038, 748, 702 | $CDCl_3$: 7.50 (1H, bs), 7.4-6.9 (9H, m), 5.5-5.4 (1H, m), 4.23 (2H, q, J=7Hz), 4.11 (1H, dd, J=18, 5Hz), 4.03 (1H, dd, J=18, 5Hz), 3.2-3.0 (4H, m), 2.9-2.6 (2H, m), 1.30 (3H, t, J=7Hz) | 123.6-128.7 |
| 32 | KBr: 3317, 3180, 3166, 1732, 1645, 1533, 1417, 1203, 700 | DMSO-$d_6$: 12.26 (0.5H, bs), 12.02 (0.5H, bs), 8.4-8.3 (1H, m), 7.5-7.0 (14H, m), 5.7-5.5 (1H, m), 5.36 (0.5H, d, J=4Hz), 5.3-5.2 (0.5H, m), 4.6-4.4 (1H, m), 4.1-3.9 (2H, m), 3.09 (4H, s), 3.1-2.5 (4H, m)1.2-1.0 (3H, m) | 133.3-154.2 |

Table 2 (continued)

| Example No. | IR $cm^{-1}$ | NMR ppm | M.P. (°C) |
|---|---|---|---|
| 33 | KBr: 3340, 3192, 1732, 1643, 1533, 1417, 760, 700 | DMSO-$d_6$: 1226 (0.5H, bs), 1204 (0.5H, bs), 8.4-8.3 (1H, m), 7.5-7.0 (14H, m), 5.6-5.5 (1H, m), 5.37 (0.5H, d, J=5Hz), 5.3-5.2 (0.5H, m), 4.6-4.4 (1H, m), 4.1-3.9 (2H, m), 3.10 (4H, s), 3.1-2.5 (4H, m)1.2-1.0 (3H, m) | 134.1-135.5 |
| 34 | neat: 3313, 2953, 1728, 1454, 1431, 1282, 1198, 1151, 1119, 1072, 750, 700 | $CDCl_3$: 10.54 (1H, bs), 8.01 (1H, d, J=7Hz), 7.65 (1H, d, J=8Hz), 7.57 (1H, d, J=6Hz), 7.49 (1H, t, J=8Hz), 7.3-7.2 (5H, m), 7.13 (1H, t, J=8Hz), 7.00 (1H, d, J=7Hz), 4.79 (2H, s), 4.65 (2H, s), 3.94 (3H, s), 3.89 (3H, s), 3.3-3.1 (4H, m) | Oil |
| 35 | KBr: 2922, 2868, 1707, 1581, 1564, 1456, 1381, 1271. 752 | DMSO-$d_6$: 7.80 (1H, d, J=8Hz), 7.74 (1H, d, J=8Hz), 7.51 (1H, t, J=8Hz), 7.43 (1H, d, J=7Hz), 7.4-7.1 (7H, m), 4.84 (2H, s), 4.66 (2H, s), 3.12 (4H, s) | 160.3-163.5 |
| 36 | neat: 1714, 1450, 1443, 1433, 1263, 1136, 1078, 744 | $CDCl_3$: 10.38 (1H, bs), 7.97 (1H, d, J=8Hz), 7.7-7.0 (11H, m), 4.91 (2H, s), 4.89 (2H, s), 4.36 (2H, q, J=7Hz), 320 (4H, s), 1.36 (3H, t J=7Hz) | Oil |
| 37 | KBr: 2927, 2864, 1583, 1549, 1443, 1383, 1084, 750 | $CDCl_3$: 7.93-7.87 (1H, m), 7.62 (1H, d, J=8Hz), 7.4-7.3 (3H, m), 7.21 (1H, t, J=8Hz), 7.14 (1H, d, J=7Hz), 6.9-6.7 (5H, m), 4.88 (2H, s), 4.81 (2H, s), 3.28 (2H, t, J=8Hz), 2.91 (2H, t, J=8Hz) | 86.3-88.1 |
| 38 | KBr: 3490, 2856, 1718,1277,1109 | $CDCl_3$: 14.34 (1H, bs), 7.94 (2H, d, J=8Hz), 7.70 (1H, s), 7.5-7.3 (4H, m), 7.09 (2H, s), 6.98 (3H, s), 4.99 (2H, s), 4.75 (2H, s), 4.35 (2H, q, J=7Hz), 3.53 (2H, bs), 3.24 (2H, bs), 1.37 (3H, t, J=7Hz) | 145.4-148.6 |
| 39 | KBr: 3426, 2854, 1722, 1288, 1203, 1105, 748 | $CDCl_3$: 14.25 (1H, bs), 7.98 (1H, s), 7.90 (1H, d, J=7Hz), 7.70 (1H, d, J=5Hz), 7.54 (1H, d, J=7Hz), 7.4-7.3 (3H, m), 7.10 (2H, s), 6.99 (3H, s), 4.99 (2H, s), 4.73 (2H, s), 3.85 (3H, s), 3.55 (2H, bs), 3.25 (2H, bs) | 83.7-87.5 |
| 40 | KBr: 3404, 2916, 1732, 1660, 1522, 1336, 1269, 1228, 1203, 1151 | $CDCl_3$: 10.34(1H, bs), 7.53 (1H, d, J=2Hz), 7.4-7.2 (6H, m), 4.24 (2H, q, J=7Hz), 4.05 (2H, s), 3.90 (3H, s), 3.3-3.1 (4H, m), 1.29 (3H, t, J=7Hz) | 138.5-139.2 |
| 41 | KBr: 3302, 1722, 1630, 1452, 1425, 1198, 1180, 1149 | $CDCl_3$: 9.47 (1H, bs), 7.4-7.1 (7H, m), 6.61 (1H, bs), 5.37 (1H, bs), 4.22 (2H, q, J=7Hz), 3.83 (3H, s), 3.3-3.1 (4H, m), 2.9-2.7 (2H, m), 1.30 (3H, t, J=7Hz) | 126.9-127.7 |

Table 2 (continued)

| Example No. | IR $cm^{-1}$ | NMR ppm | M.P. (°C) |
|---|---|---|---|
| 42 | KBr: 3410, 2980, 1734, 1666, 1524, 1427, 1346, 1290, 1153, 1132, 700 | DMSO-$d_6$: 12.34 (0.4H, bs), 12.32 (0.6H, bs), 9.46 (0.4H, s), 9.37 (0.6H, s), 7.3-7.0 (6.6H, m), 4.54 (0.8H, s), 4.20 (1.2H, s), 4.2-4.0 (2H, m), 3.11 (4H, s), 1.2-1.1 (3H, m) | 169.1-170.0 |
| 43 | KBr: 3184, 3136, 1726, 1637, 1497, 1159, 700 | DMSO-$d_6$: 10.02 (1H, bs), 7.3-7.2 (5H, m), 7.0-6.8 (2H, m), 6.07 (1H, bs), 5.28 (1H, t, J=7Hz), 4.05 (2H, q, J=7Hz), 3.40 (2H, t J=8Hz), 3.16 (2H, t, J=8Hz), 2.8-2.7 (2H, m), 1.14 (3H, t, J=7Hz) | 189.3-192.5 |
| 44 | KBr: 3304, 2931, 1632, 1606, 1454, 1427, 1151, 700 | DMSO-$d_6$: 11.89 (0.6H, bs), 11.69 (0.4H, bs), 8.97 (0.6H, bs), 8.78 (0.4H, bs), 7.4-7.2 (5H, m), 6.8-6.6 (2H, m), 5.4-5.3 (0.4H, m), 5.3-5.2 (0.6H, m), 5.2-5.0 (0.6H, m), 5.0-4.8 (0.4H, m), 4.8-4.6 (0.6H, m), 4.5-4.4 (0.4H, m), 3.6-3.3 (2H, m), 3.04 (4H, s), 1.9-1.7 (2H, m) | 77.2-80.2 |
| 45 | KBr: 3113, 3109, 3062, 2918, 1454, 1423, 1106, 1066 | DMSO-$d_6$: 12.26 (0.4H, bs), 11.96 (0.6H, bs), 7.5-7.0 (8H, m), 5.35 (0.6H, d, J=4Hz), 5.4-5.3 (0.4H, m), 5.11 (0.4H, d, J=5Hz), 5.0-4.9 (0.6H, m), 4.61 (0.4H, t, J=5Hz), 4.42 (0.6H, t, J=5Hz), 3.6-3.4 (2H, m), 3.10 (4H, s), 2.1-1.7 (2H, m) | 146.4-148.6 |
| 46 | KBr: 3481, 3427, 3398, 3367, 1697, 1635, 1417, 1288, 1180 | DMSO-$d_6$: 12.19 (1H, s), 9.29 (1H, s), 7.82 (1H, d, J=16Hz), 7.4-7.1 (6H, m), 6.86 (2H, s), 4.20 (2H, q, J=7H), 3.10 (4H, s), 1.28 (3H, t, J=7Hz) | 169.2-172.7 |
| 47 | KBr: 3348, 2985, 2939, 1732, 1666, 1522, 1281, 1257, 716 | $CDCl_3$: 10.48(1H, bs), 7.95(1H, d, J=2Hz), 7.68 (1H, d, J=2Hz), 7.4-7.2 (5H, m), 4.25 (2H, q, J=7Hz), 4.05 (2H, s), 3.3-3.2 (4H, m), 1.30(3H, t, J=7Hz) | 118.8-119.7 |
| 48 | KBr: 3116, 1718, 1527, 1452, 1419, 1294, 1178, 1028, 854, 700 | DMSO-$d_6$: 12.46 (0.5H, bs), 12.36 (0.5H, bs), 7.5-7.1 (7H, m), 5.82 (0.5H, d, J=4Hz), 5.7-5.5 (1H, m), 5.3-5.2 (0.5H, m), 4.2-4.0 (2H, m), 3.2-3.0 (4.5H, m), 2.8-2.7 (1H, m), 2.6-2.5 (0.5H, m), 1.3-1.1 (3H, m) | 106.1-107.3 |
| 49 | KBr: 3045, 1659, 1614, 1599, 1522, 1429, 1267, 1122, 739 | DMSO-$d_6$: 12.93 (1H, bs), 12.70 (1H, bs), 8.07 (1H, d, J=16Hz), 8.01 (1H, d, J=8Hz), 7.90 (1H, J=8Hz), 7.58 (1H, d, J=16Hz), 7.47 (1H, brs), 7.4-7.2 (7H, m), 3.3-3.1 (4H, m) | 1720-174.3 |
| 50 | KBr: 3398, 3061, 2927, 1633, 1601, 1439, 1369, 1080, 750 | DMSO-$d_6$: 7.47 (1H, dd, J=7, 1Hz), 7.4-7.1 (7H, m), 5.55 (1H, s), 3.3-3.1 (4H, m) | 127.6-130,2 |

Table 2 (continued)

| Example No. | IR $cm^{-1}$ | NMR ppm | M.P. (°C) |
|---|---|---|---|
| 51 | KBr: 2983, 2638, 1738, 1497, 1421, 1207, 1018, 760 | DMSO-$d_6$: 12.37 (0.5H, bs), 12.14 (0.5H, bs), 7.5-7.0 (8H, m), 6.20 (0.5H, d, J=5Hz), 5.97 (0.5H, d, J=6Hz), 5.74 (0.5H, d, J=6Hz), 5.51 (0.5H, d, J=5Hz), 4.4-4.0 (2H, m), 3.12 (4H, s), 1.10 (3H, t, J=7Hz) | 202.6-204.2 |
| 52 | KBr: 3430, 1751, 1670, 1601, 1373, 1157 | $CDCl_3$: 10.65 (1H, brs), 7.89 (1H, dd, J=9, 5Hz), 7.4-7.2 (5H, m), 7.04 (1H, dd, J=12, 9Hz), 4.25 (2H, q, J=7Hz), 4.08 (2H, d, J=4Hz), 3.3-3.1 (4H, m), 1.29 (3H, t, J=7Hz) | 83.9-85.1 |
| 53 | KBr: 3211, 2979, 1736, 1443, 1257, 1039, 812 | DMSO-$d_6$: 12.42 (0.3H, bs), 12.11 (0.7H, bs), 7.41 (0.7H, dd, J=9, 5Hz), 7.4-7.2 (5.3H, m), 7.0-6.9 (1H, m), 5.96 (0.7H, d, J=4Hz), 5.7-5.5 (1.3H, m), 4.1-4.0 (2H, m), 3.2-3.0 (4H, m), 3.0-2.6 (2H, m), 1.14 (3H, t, J=7Hz) | 114.1-116.9 |

Examples of the formulation of pharmaceutical preparations which contain compounds of the present invention are shown in the following, but the invention is not restricted thereby.

| (Formulation Example 1 Capsules) | |
|---|---|
| Components | Amount used (g) |
| Compound of Inventive Example 6 | 50 |
| Lactose | 935 |
| Magnesium stearate | 15 |

The above components were respectively weighed and uniformly mixed. By filling appropriate hard capsules with the thus obtained powder mixture in 200 mg portions, capsules were successfully produced.

| (Formulation Example 2 Tablets) | |
|---|---|
| Components | Amount used (g) |
| Compound of Inventive Example 2 | 100 |
| Lactose | 350 |
| Potato starch | 120 |
| Polyvinyl alcohol | 15 |
| Magnesium stearate | 15 |

After weighing each of the above components, the title compound was uniformly mixed with lactose and potato starch. Aqueous solution of polyvinyl alcohol was added to the mixture to prepare granules by a wet granulation method. The granules were dried, mixed with magnesium stearate and then made into tablets, each having 300 mg in weight, preparing use of a compressive tablet making machine.

| (Formulation Example 3 Granules) | |
|---|---|
| Components | Amount used (g) |
| Compound of Inventive Example 3 | 200 |
| Lactose | 450 |
| Corn starch | 300 |
| Hydroxypropylcellulose | 50 |

After weighing and uniformly mixing the above components, granules were successfully produced in the usual way.

| (Formulation Example 4 Injections) | |
|---|---|
| Components | Amount used (g) |
| Compound of Inventive Example 7 | 2 g |
| Sodium bicarbonate | 10 g |
| Distilled water for injection use | 1,000 mL |

Sodium bicarbonate was dissolved in distilled water for injection use, and the compound of Inventive Example 7 was dissolved in the solution. The resulting solution was sterilized by filtration and dispensed in 5 mL portions into 10 mL capacity ampoules which were then melt-sealed, thereby obtaining injections.

INDUSTRIAL APPLICABILITY

When the compound of the present invention having benzimidazole nucleus is used, strong action to inhibit eosinophilia is exerted. Also, since the compound of the present invention having benzimidazole nucleus has excellent safety due to its extremely low toxicity, it exerts strong action to inhibit eosinophilia in the clinical field (human) or in animals, so that excellent preventive and/or therapeutic effects on diseases exhibiting eosinophilia, bronchial asthma and allergic diseases can be expected.

Also, prevention and/or treatment of diseases exhibiting eosinophilia, bronchial asthma and allergic diseases can be achieved by the medicaments and pharmaceutical compositions of the present invention. Illustratively, since it strongly inhibits eosinophilia, the inventive compound is effective for the prevention and/or treatment of diseases in which eosinophils are probably concerned in their morbid states, namely parasite infection, hypereosinophilic syndrome (HES), eosinophilic pneumonia, eosinophilic enterogastritis, bronchial asthma, atopic dermatitis, allergic rhinitis and the like diseases. In addition, it is also effective for the prevention and/or treatment of diseases caused by IgE antibody, such as hay fever, angioneurotic edema, serous otitis media, pollinosis, allergic enterogastritis, food allergy, drug allergy and the like allergic diseases.

**Claims**

**1.** A compound represented by the following formula (I)

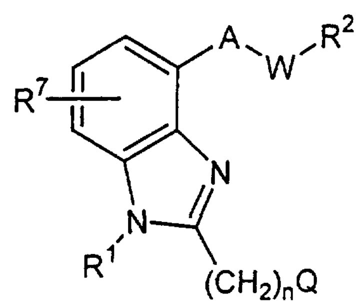

(I)

(wherein $R^1$ represents hydrogen atom or a straight- or branched-chain alkyl group having 1 to 4 carbon atoms, $R^2$ represents cyano group, hydroxymethyl group, 2-(2-imidazolyl)ethenyl group, a phenyl group substituted by one or two $-COOR^3$ groups, or a group $-COOR^3$ or $-CONR^4R^5$, $R^3$ represents hydrogen atom or a straight- or branched-chain alkyl group having 1 to 4 carbon atoms, each of $R^4$ and $R^5$ represents hydrogen atom, an alkyl group having 1 or 2 carbon atoms or a group $-CH_2COOR^6$ or $-CH(CH_2Ph)COOR^6$, wherein $R^4$ and $R^5$ may be the same or different from each other but, when one of $R^4$ and $R^5$ is a group $-CH_2COOR^6$ or $-CH(CH_2Ph)COOR^6$, the other one is hydrogen atom, A represents any one of groups selected from the class consisting of -CO-, $-CH(OR^8)-$, $-CH_2O-$, $-CH(NHR^9)CH_2-$, -CH=CH- and $-CH_2CH_2-$, W represents a group $-CH_2-$ or a single bond, Q represents a phenyl group which may be substituted by one hydroxyl group, n is from 0 to 2, $R^6$ represents a straight- or branched-chain alkyl group having 1 to 4 carbon atoms, $R^7$ represents hydrogen atom, hydroxyl group, a halogen

atom or a straight- or branched-chain alkoxyl group having 1 to 4 carbon atoms, $R^8$ represents hydrogen atom or acetyl group and $R^9$ represents hydrogen atom, acetyl group, phenylsulfonyl group or a benzoyl group which may be substituted by one methoxy group) or a salt thereof.

**2.** The compound or a salt thereof according to claim 1 wherein n is 2.

**3.** The compound or a salt thereof according to claim 1 or 2 wherein $R^2$ is a phenyl group substituted by one or two $-COOR^3$ groups or a group $-COOR^3$ or $-CONR^4R^5$.

**4.** The compound or a salt thereof according to any one of claims 1 to 3 wherein $R^1$ is hydrogen atom, W is a group $-CH_2-$, A is any one of groups selected from the class consisting of -CO-, -CH(OR)- and $-CH_2O-$, and $R^2$ is a group $-COOR^3$ or a phenyl group substituted by one or two $-COOR^3$ groups.

**5.** A process for producing the compound of formula (I) described in claim 1 or a salts thereof, which comprises treating a compound represented by the following formula (III)

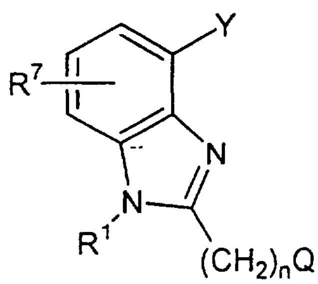

(III)

(wherein Y represents acetyl group, $-COOR^3$, a halogen atom, formyl group, chloroformyl group or bromoformyl group, each of $R^1$ and $R^3$ represents hydrogen atom or a straight- or branched-chain alkyl group having 1 to 4 carbon atoms, $R^7$ represents hydrogen atom, hydroxyl group, a halogen atom or a straight- or branched-chain alkoxyl group having 1 to 4 carbon atoms, Q represents a phenyl group which may be substituted by one hydroxyl group and n is from 0 to 2) or a salt thereof in accordance with any one of steps selected from the group consisting of the following steps (a) to (k), and if necessary, subsequently subjecting the thus obtained compound to a reduction, an oxidation or a substituent change:

(a) the compound is allowed to react with carbon dioxide in the presence of an inorganic base or an organic base and also in the presence, if necessary, of a phase-transfer catalyst, magnesium chloride, sodium iodide or diphenylurea, or with a carbamato complex in an inert solvent, thereby obtaining corresponding carboxylic acid derivatives which, if necessary, is further subjected to esterification under appropriate conditions,
(b) the compound is allowed to react with halogeno-formic acid ester, dialkyl carbonate, phosphonoformic acid ester or oxalic acid ester in the presence of a base, and then subjected to hydrolysis if necessary,
(c) the compound is allowed to react with malonic acid ester in the presence of a base, and then subjected to hydrolysis and subsequent decarboxylation, followed by esterification if necessary,
(d) an acetic acid or an acetic acid ester is prepared into a metal reagent using an appropriate metalating agent, and then the compound is allowed to react with the reagent,
(e) a halogeno-acetic acid derivative is prepared into Reformatsky reagent, and then the compound is allowed to react with the reagent,
(f) the compound is allowed to react with Meldrum's acid in the presence of a base to convert it into acyl Meldrum's acid which is then subjected to solvolysis and decarboxylation using an appropriate alcohol, followed by hydrolysis if necessary,
(g) the compound is allowed to react with a malonic acid ester, and then subjected to hydrolysis and decarboxylation if necessary,
(h) using a transition metal complex, the compound is allowed to undergo cross-coupling reaction with an acetylene compound, and then hydration reaction is carried out,
(i) the compound is subjected to halogen-metal exchange reaction using an organic lithium reagent, allowed to react with ethylmalonyl chloride and then subjected to hydrolysis and decarboxylation,
(j) the compound is reduced using a metal hydride, allowed to react with substituted benzyl halides in the

presence of a base, and then hydrolyzed in the substituted group if necessary,
(k) the compound is allowed to react with hydrogen cyanide or trimethylsilyl cyanide in the presence of a Lewis acid, and then hydrolyzed, if necessary further carrying out esterification.

**6.** An agent for preventing and/or treating diseases exhibiting eosinophilia, which contains a compound represented by the formula (I) described in claim 1 or a salt thereof as the active ingredient.

**7.** An agent for preventing and/or treating bronchial asthma, which contains a compound represented by the formula (I) described in claim 1 or a salt thereof as the active ingredient.

**8.** An agent for preventing and/or treating allergic diseases, which contains a compound represented by the formula (I) described in claim 1 or a salt thereof as the active ingredient.

**9.** A medicament which contains at least one of the compounds represented by the formula (I) of claim 1 or salts thereof as the active ingredient.

**10.** An optically active compound according to claim 1 represented by the following formula (I)-d

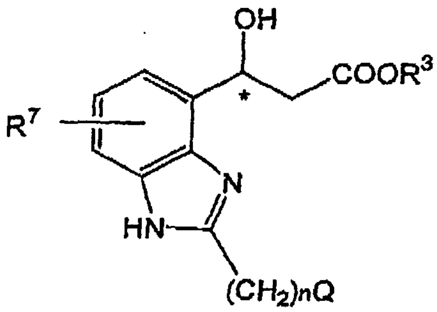

(I)-d

wherein $R^3$ represents hydrogen atom or a straight- or branched-chain alkyl group having 1 to 4 carbon atoms, Q represents a phenyl group which may be substituted by one hydroxyl group, n is from 0 to 2, $R^7$ represents hydrogen atom, hydroxyl group, a halogen atom or a straight- or branched-chain alkoxyl group having 1 to 4 carbon atoms; or a salt thereof.

**11.** The compound or a salt thereof according to claim 10 wherein Q is a phenyl group, n is 2, and $R^7$ is a hydrogen atom.

**Patentansprüche**

**1.** Verbindung, dargestellt durch die folgende Formel (I)

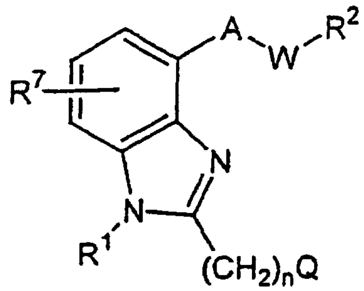

(I)

wobei $R^1$ ein Wasserstoffatom oder eine geradkettige oder verzweigte Alkylgruppe mit 1 bis 4 Kohlenstoffatomen darstellt, $R^2$ eine Cyanogruppe, eine Hydroxymethylgruppe, eine 2-(2-Imidazolyl)ethenylgruppe, eine Phenylgruppe, substituiert mit einer oder zwei -$COOR^3$ Gruppen, oder eine Gruppe -$COOR^3$ oder -$CONR^4R^5$ darstellt, $R^3$ ein

Wasserstoffatom oder eine geradkettige oder verzweigte Alkylgruppe mit 1 bis 4 Kohlenstoffatomen darstellt, $R^4$ und $R^5$ jeweils ein Wasserstoffatom, eine Alkylgruppe mit 1 oder 2 Kohlenstoffatomen oder eine Gruppe $-CH_2COOR^6$ oder $-CH(CH_2Ph)COOR^6$ darstellen, wobei $R^4$ und $R^5$ gleich oder verschieden voneinander sein können aber, wenn einer von $R^4$ und $R^5$ eine Gruppe $-CH_2COOR^6$ oder $-CH(CH_2Ph)COOR^6$ ist, der jeweils andere ein Wasserstoffatom ist, A irgendeine der Gruppen darstellt, ausgewählt aus der Gruppe bestehend aus -CO-, $-CH(OR^8)-$, $-CH_2O-$, $-CH(NHR^9)CH_2-$, -CH=CH- und $-CH_2CH_2-$, W eine Gruppe $-CH_2-$ oder eine Einfachbindung darstellt, Q eine Phenylgruppe darstellt, die durch eine Hydroxylgruppe substituiert sein kann, n von 0 bis 2 ist, $R^6$ eine geradkettige oder verzweigte Alkylgruppe mit 1 bis 4 Kohlenstoffatomen darstellt, $R^7$ ein Wasserstoffatom, eine Hydroxylgruppe, ein Halogenatom oder eine geradkettige oder verzweigte Alkoxylgruppe mit 1 bis 4 Kohlenstoffatomen darstellt, $R^8$ ein Wasserstoffatom oder eine Acetylgruppe darstellt und $R^9$ ein Wasserstoffatom, eine Acetylgruppe, eine Phenylsulfonylgruppe oder eine Benzoylgruppe darstellt, die mit einer Methoxygruppe substituiert sein kann,

**2.** Verbindung oder ein Salz davon in Übereinstimmung mit Anspruch 1, wobei n 2 ist.

**3.** Verbindung oder ein Salz davon in Übereinstimmung mit Anspruch 1 oder 2, wobei $R^2$ eine Phenylgruppe ist, substituiert mit einer oder zwei Gruppen $-COOR^3$, oder eine Gruppe $-COOR^3$ oder $-CONR^4R^5$.

**4.** Verbindung oder ein Salz davon in Übereinstimmung mit irgendeinem der Ansprüche 1 bis 3, wobei $R^1$ ein Wasserstoffatom ist, W die Gruppe $-CH_2-$ ist, A irgendeine der Gruppen ist, ausgewählt aus der Gruppe bestehend aus -CO-, CH(OR)- und $-CH_2O-$, und $R^2$ eine Gruppe $-COOR^3$ oder eine Phenylgruppe, substituiert mit einer oder zwei $-COOR^3$ Gruppen ist.

**5.** Verfahren zur Herstellung der Verbindung der Formel (I), beschrieben in Anspruch 1, oder eines Salzes davon, umfassend die Behandlung einer Verbindung, dargestellt durch die folgende Formel (III)

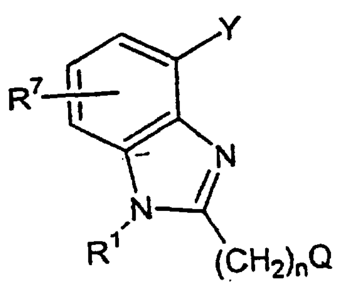

(III)

wobei Y eine Acetylgruppe, $-COOR^3$, ein Halogenatom, eine Formylgruppe, eine Chloroformylgruppe oder eine Bromoformylgruppe darstellt, $R^1$ und $R^3$ jeweils ein Wasserstoffatom oder eine geradkettige oder verzweigte Alkylgruppe mit 1 bis 4 Kohlenstoffatomen darstellen, $R^7$ ein Wasserstoffatom, eine Hydroxylgruppe, ein Halogenatom oder eine geradkettige oder verzweigte Alkoxylgruppe mit 1 bis 4 Kohlenstoffatomen darstellt, Q eine Phenylgruppe darstellt, die mit einer Hydroxylgruppe substituiert sein kann, und n von 0 bis 2 ist, oder eines Salzes davon,
in Übereinstimmung mit irgendeiner der Stufen, ausgewählt aus der Gruppe bestehend aus den folgenden Stufen (a) bis (k) und, falls notwendig, anschließendes Unterwerfen der so erhaltenen Verbindung einer Reduktion, einer Oxidation oder einer Substituentenaustauschreaktion:

(a) Erlauben, dass die Verbindung mit Kohlenstoffdioxid in der Gegenwart einer anorganischen oder organischen Base reagiert, und ebenso in der Gegenwart, falls notwendig, eines Phasentransferkatalysatoren, Magnesiumchlorid, Natriumchlorid oder Diphenylharnstoff, oder mit einem Carbamatocomplex in einem inerten Lösungsmittel, wodurch die korrespondierenden Carboxylsäurederivate erhalten werden, die, falls notwendig, weiter einer Veresterung unter geeigneten Bedingungen unterworfen werden;

(b) Erlauben, dass die Verbindung mit Halogenoameisensäureester, Dialkylcarbonat, Phosphonoameisenester oder Oxalsäureester reagiert, in der Gegenwart einer Base, und anschließendes Unterwerfen einer Hydrolyse, falls notwendig;

(c) Erlauben, dass die Verbindung mit Malonsäureester, in der Gegenwart einer Base reagiert, und anschließend einer Hydrolyse und anschließend einer Decarboxylierung, gefolgt von Veresterung, falls notwendig, unterworfen wird;

(d) Umwandeln einer Essigsäure oder eines Essigsäureesters in ein Metallreagenz unter Verwendung eines geeigneten Metallierungsmittels und anschließendes Erlauben, dass die Verbindung mit dem Reagenz reagiert;

(e) Umwandeln eines Halogenoessigsäurederivats in ein Reformatsky-Reagenz und Erlauben, dass die Verbindung mit dem Reagenz reagiert;

(f) Erlauben, dass die Verbindung mit Meldrum-Säure in der Gegenwart einer Base reagiert, um die Verbindung in ein Acylderivat der Meldrum-Säure umzuwandeln, welches dann einer Solvolyse und einer Decarboxylierung unter Verwendung eines geeigneten Alkohols unterworfen wird, gefolgt von Hydrolyse, falls notwendig;

(g) Erlauben, dass die Verbindung mit einem Malonsäureester reagiert, und anschließendes Unterwerfen, falls notwendig, einer Hydrolyse und Decarboxylierung;

(h) Erlauben, unter Verwendung eines Übergangsmetallkomplexes, dass die Verbindung eine Crosskupplungsreaktion mit einer Acetylenverbindung eingeht und anschließendes Durchführen einer Hydratation;

(i) Unterwerfen der Verbindung einer Halogen-Metall-Austauschreaktion unter Verwendung eines organischen Lithiumreagenz, Erlauben des Reagierens mit Ethylmalonylchlorid und anschließendes Unterwerfen einer Hydrolyse und Decarboxylierung;

(j) Reduzieren der Verbindung unter Verwendung eines Metallhydrids, Erlauben der Reaktion mit einem substituierten Benzylhalogenid in der Gegenwart einer Base und anschließendes Hydrolisieren der substituierten Gruppe, falls notwendig;

(k) Erlauben, dass die Verbindung mit Wasserstoffcyanid oder Trimethylsilylcyanid reagiert in der Gegenwart einer Lewissäure, und anschließendes Hydrolysieren, so wie, falls notwendig, weiteres Durchführen einer Veresterung.

**6.** Mittel zur Veränderung und/oder Behandlung von Störungen die Eosinophilie zeigen, enthaltend eine Verbindung, dargestellt durch die Formel (I), beschrieben in Anspruch 1 oder ein Salz davon als aktiven Bestandteil.

**7.** Mittel für die Verhinderung und/oder Behandlung von bronchialem Asthma, enthaltend eine Verbindung, dargestellt durch die Formel (I), beschrieben in Anspruch 1 oder ein Salz davon, als aktiven Bestandteil.

**8.** Mittel zur Verhinderung und/oder Behandlung von allergenen Störungen, enthaltend eine Verbindung, dargestellt durch die Formel (I), beschrieben in Anspruch 1 oder ein Salz davon als aktiven Bestandteil.

**9.** Medikament, enthaltend mindestens eine der Verbindungen, dargestellt durch die Formel (I) des Anspruchs 1 oder Salze davon als aktiven Bestandteil.

**10.** Optisch aktive Verbindung in Übereinstimmung mit Anspruch 1, dargestellt durch die folgende Formel (I)-d

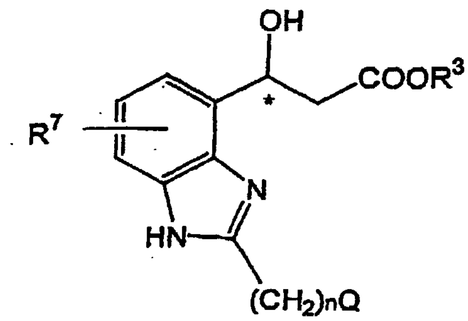

(I)-d

wobei $R^3$ ein Wasserstoffatom oder eine geradkettige oder verzweigte Alkylgruppe mit 1 bis 4 Kohlenstoffatomen darstellt, Q eine Phenylgruppe darstellt, die mit einer Hydroxylgruppe substituiert sein kann, n von 0 bis 2 ist, $R^7$ ein Wasserstoffatom, eine Hydroxylgruppe, ein Halogenatom oder eine geradkettige oder verzweigte Alkoxylgruppe mit 1 bis 4 Kohlenstoffatomen darstellt; oder ein Salz davon.

**11.** Verbindung oder ein Salz davon in Übereinstimmung mit Anspruch 10, wobei Q eine Phenylgruppe ist, n 2 ist und $R^7$ ein Wasserstoffatom ist.

**Revendications**

**1.** Composé représenté par la formule (I) suivante :

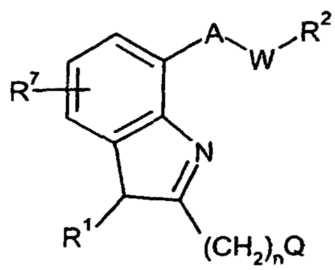

(I)

(dans laquelle $R^1$ représente un atome d'hydrogène ou un groupe alkyle à chaîne droite ou ramifiée ayant de 1 à 4 atomes de carbone, $R^2$ représente un groupe cyano, un groupe hydroxyméthyle, un groupe 2-(2-imidazolyl) éthényle, un groupe phényle substitué par un ou deux groupes $-COOR^3$, ou un groupe $-COOR^3$ ou $-CONR^4R^5$, $R^3$ représente un atome d'hydrogène ou un groupe alkyle à chaîne droite ou ramifiée ayant de 1 à 4 atomes de carbone, chacun de $R^4$ et $R^5$ représente un atome d'hydrogène, un groupe alkyle ayant 1 ou 2 atomes de carbone ou un groupe $-CH_2COOR^6$ ou $-CH(CH_2Ph)COOR^6$, où $R^4$ et $R^5$ peuvent être mutuellement identiques ou différents mais, quand l'un de $R^4$ et $R^5$ est un groupe $-CH_2COOR^6$ ou $-CH(CH_2Ph)COOR^6$, l'autre est un atome d'hydrogène, A représente l'un quelconque des groupes choisis dans l'ensemble constitué par -CO-, $-CH(OR^8)-$, $-CH_2O-$, $-CH(NHR^9)CH_2-$, -CH=CH- et $-CH_2CH_2-$, W représente un groupe $-CH_2-$ ou une liaison simple, Q représente un groupe phényle qui peut être substitué par un groupe hydroxyle, n vaut de 0 à 2, $R^6$ représente un groupe alkyle à chaîne droite ou ramifiée ayant de 1 à 4 atomes de carbone, $R^7$ représente un atome d'hydrogène, un groupe hydroxyle, un atome d'halogène ou un groupe alcoxy à chaîne droite ou ramifiée ayant de 1 à 4 atomes de carbone, $R^8$ représente un atome d'hydrogène ou un groupe acétyle et $R^9$ représente un atome d'hydrogène, un groupe acétyle, un groupe phénylsulfonyle ou un groupe benzoyle qui peut être substitué par un groupe méthoxy) ou un de ses sels.

**2.** Composé ou son sel selon la revendication 1, dans lequel n vaut 2.

**3.** Composé ou son sel selon la revendication 1 ou 2, dans lequel $R^2$ est un groupe phényle substitué par un ou deux groupes $-COOR^3$, ou un groupe $-COOR^3$ ou $-CONR^4R^5$.

**4.** Composé ou son sel selon l'une quelconque des revendications 1 à 3, dans lequel $R^1$ est un atome d'hydrogène, W est un groupe $-CH_2-$, A est l'un quelconque parmi les groupes choisis dans l'ensemble constitué par -CO-, -CH (OR)- et $-CH_2O-$, et $R^2$ est un groupe $-COOR^3$ ou un groupe phényle substitué par un ou deux groupes $-COOR^3$.

**5.** Procédé pour produire le composé de formule (I) décrit dans la revendication 1 ou un de ses sels, qui comprend le traitement d'un composé représenté par la formule (III) suivante :

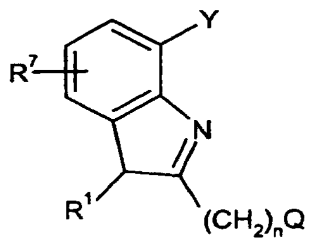

(III)

(dans laquelle Y représente un groupe acétyle, $-COOR^3$, un atome d'halogène, un groupe formyle, un groupe chloroformyle ou un groupe bromoformyle, chacun de $R^1$ et $R^3$ représente un atome d'hydrogène ou un groupe alkyle à chaîne droite ou ramifiée ayant de 1 à 4 atomes de carbone, $R^7$ représente un atome d'hydrogène, un groupe hydroxyle, un atome d'halogène ou un groupe alcoxy à chaîne droite ou ramifiée ayant de 1 à 4 atomes de carbone, Q représente un groupe phényle qui peut être substitué par un groupe hydroxyle et n vaut de 0 à 2) ou d'un de ses sels conformément à l'une quelconque des étapes choisies dans l'ensemble constitué par les étapes (a) à (k) suivantes, et, si nécessaire, ensuite la soumission du composé ainsi obtenu à une réduction, une oxydation ou un changement de substituant :

(a) le composé est laissé à réagir avec du dioxyde de carbone en présence d'une base minérale ou d'une base organique et aussi en présence, si nécessaire, d'un catalyseur de transfert de phase, de chlorure de magnésium, d'iodure de sodium ou de diphénylurée, ou avec un complexe carbamate dans un solvant inerte, de façon à donner ainsi un dérivé d'acide carboxylique correspondant qui, si nécessaire, est en outre soumis à une estérification dans des conditions appropriées,
(b) le composé est laissé à réagir avec un ester d'acide halogéno-formique, un carbonate de dialkyle, un ester d'acide phosphonoformique ou un ester d'acide oxalique en présence d'une base, et ensuite soumis à une hydrolyse si nécessaire,
(c) le composé est laissé à réagir avec de l'acide malonique en présence d'une base et ensuite soumis à une hydrolyse puis à une décarboxylation, suivies d'une estérification si nécessaire,
(d) un acide acétique ou un ester d'acide acétique est préparé dans un réactif métallique au moyen d'un agent de métallation approprié, et ensuite le composé est laissé à réagir avec le réactif,
(e) un dérivé d'acide halogéno-acétique est préparé dans un réactif de Reformatsky, et ensuite le composé est laissé à réagir avec le réactif,
(f) le composé est laissé à réagir avec un acide de Meldrum en présence d'une base pour être converti en acide d'acyl-Metdrum qui est ensuite soumis à une hydrolyse et une décarboxylation utilisant un alcool approprié, suivies d'une hydrolyse si nécessaire,
(g) le composé est laissé à réagir avec un ester d'acide malonique et ensuite soumis à une hydrolyse et une décarboxylation si nécessaire,
(h) par utilisation d'un complexe de métal de transition, le composé est laissé à subir une réaction de réticulation avec un composé d'acétylène, et ensuite la réaction d'hydratation est mise en oeuvre,
(i) le composé est soumis à une réaction d'échange halogène-métal utilisant un réactif organique lithié, laissé à réagir avec du chlorure d'éthytmalonyle et ensuite soumis à une hydrolyse et une décarboxylation,
(j) le composé est réduit au moyen d'un hydrure métallique, laissé à réagir avec des halogénures de benzyle substitués en présence d'une base, et ensuite hydrolysé dans le groupe substitué si nécessaire,
(k) le composé est laissé à réagir avec du cyanure d'hydrogène ou du cyanure de triméthylsilyle en présence d'un acide de Lewis, et ensuite hydrolysé, si nécessaire en outre avec mise en oeuvre d'une estérification.

**6.** Agent pour prévenir et/ou traiter des maladies présentant une éosinophilie, qui contient un composé représenté par la formule (I) décrit dans la revendication 1 ou un de ses sels à titre d'ingrédient actif.

**7.** Agent pour prévenir et/ou traiter l'asthme bronchique, qui contient un composé représenté par la formule (I) décrit

dans la revendication 1 ou un de ses sels à titre d'ingrédient actif.

**8.** Agent pour prévenir et/ou traiter des maladies allergiques, qui contient un composé représenté par la formule (I) décrit dans la revendication 1 ou un de ses sels à titre d'ingrédient actif.

**9.** Médicament qui contient au moins l'un des composés représentés par la formule (I) de la revendication 1 ou de leurs sels à titre d'ingrédient actif.

**10.** Composé optiquement actif selon la revendication 1, représenté par la formule (I)-d :

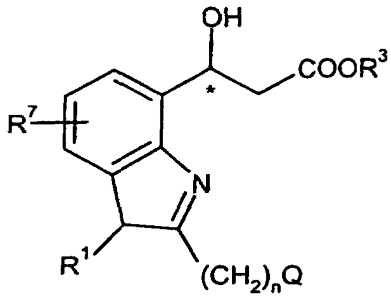

(I)-d

(dans laquelle $R^3$ représente un atome d'hydrogène ou un groupe alkyle à chaîne droite ou ramifiée ayant de 1 à 4 atomes de carbone, Q représente un groupe phényle qui peut être substitué par un groupe hydroxyle, n vaut de 0 à 2, $R^7$ représente un atome d'hydrogène, un groupe hydroxyle, un atome d'halogène ou un groupe alcoxy à chaîne droite ou ramifiée ayant de 1 à 4 atomes de carbone) ; ou un de ses sels.

**11.** Composé ou son sel selon la revendication 10, dans lequel Q est un groupe phényle, n vaut 2, et $R^7$ est un atome d'hydrogène.

Fig. 1

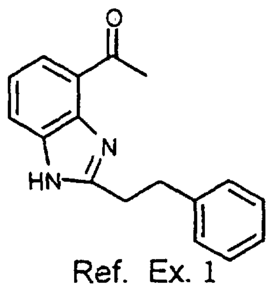

Ref. Ex. 1

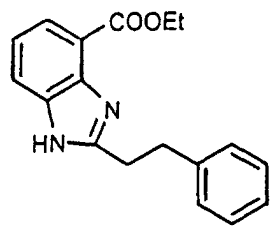

Ref. Ex. 2

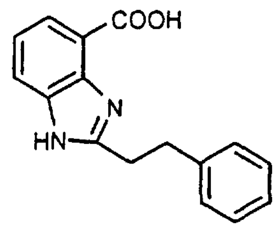

Ref. Ex. 3

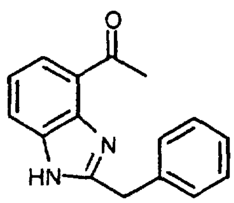

Ref. Ex. 4

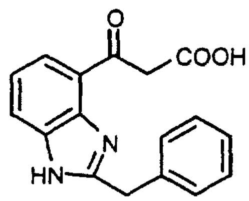

Ref. Ex. 5

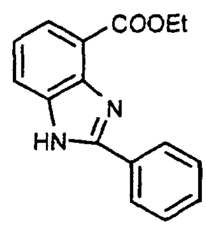

Ref. Ex. 6

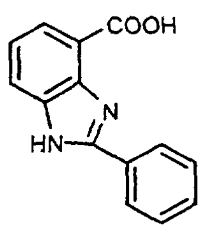

Ref. Ex. 7

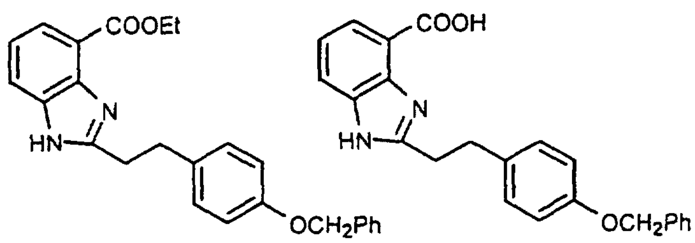

Ref. Ex. 8

Ref. Ex. 9

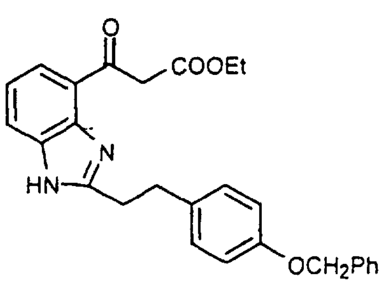

Ref. Ex. 10

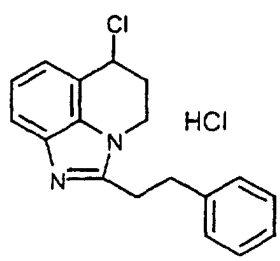

HCl

Ref. Ex. 11

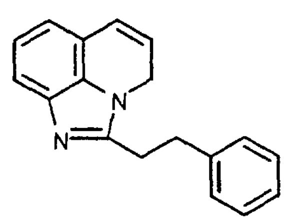

Ref. Ex. 12

Fig. 2

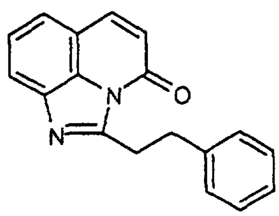

Ref. Ex. 13

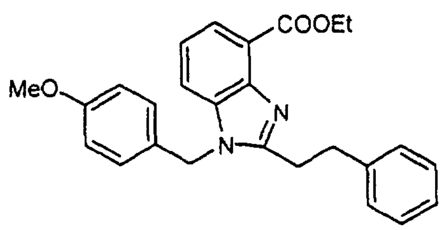

Ref. Ex. 14

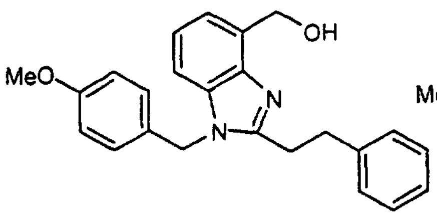

Ref. Ex. 15

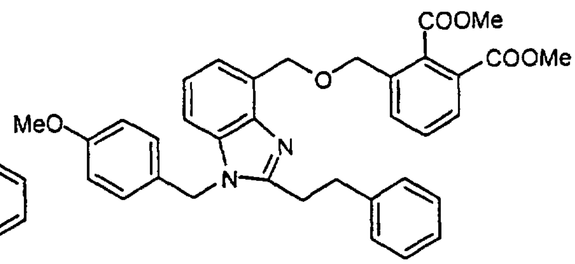

Ref. Ex. 16

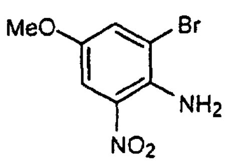

Ref. Ex. 17

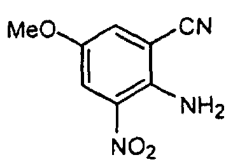

Ref. Ex. 18

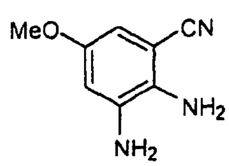

Ref. Ex. 19

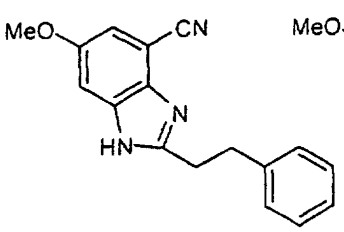

Ref. Ex. 20

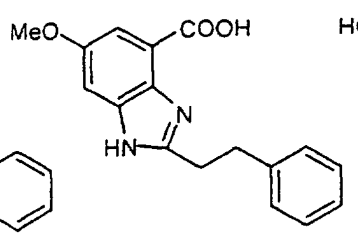

Ref. Ex. 21

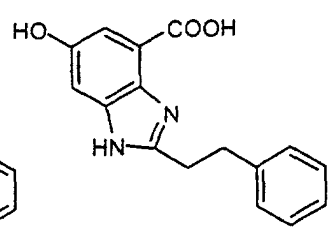

Ref. Ex. 22

Fig. 3

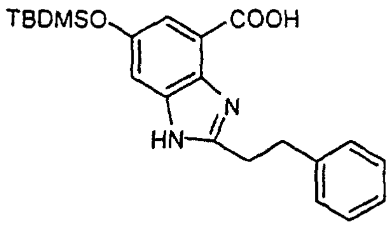

Ref. Ex. 23

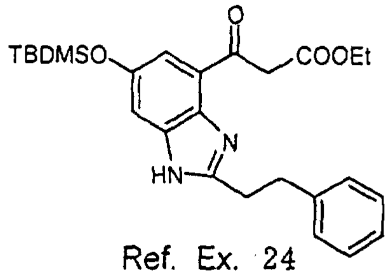

Ref. Ex. 24

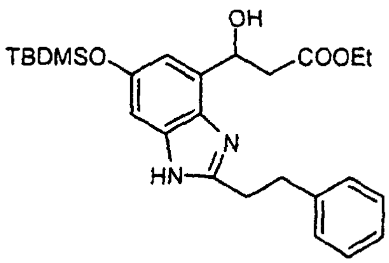

Ref. Ex. 25

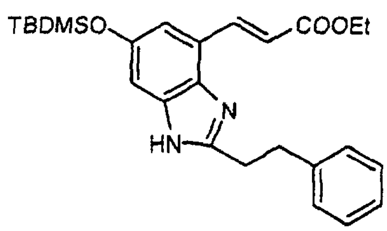

Ref. Ex. 26

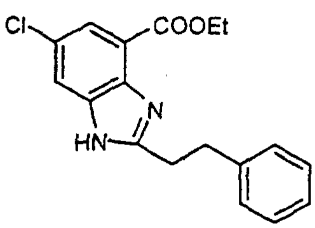

Ref. Ex. 27

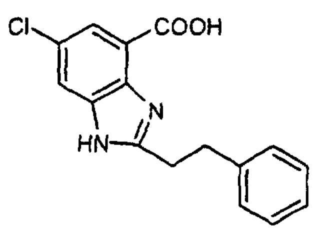

Ref. Ex. 28

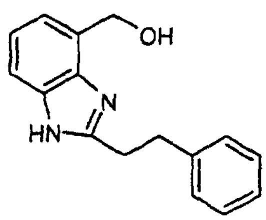

Ref. Ex. 29

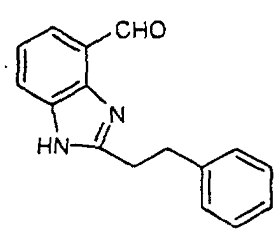

Ref. Ex. 30

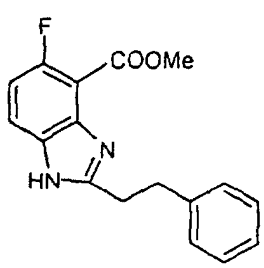

Ref. Ex. 31

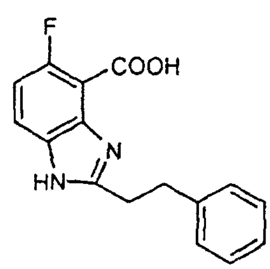

Ref. Ex. 32

Fig. 4

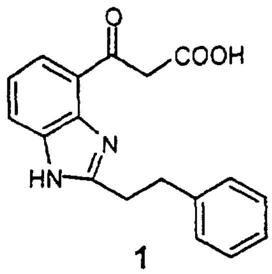

1

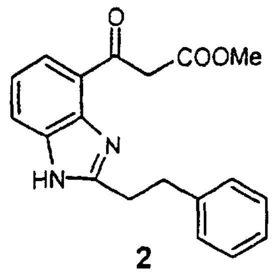

2

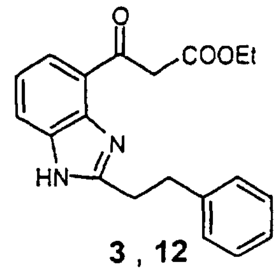

3 , 12

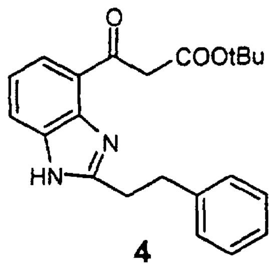

4

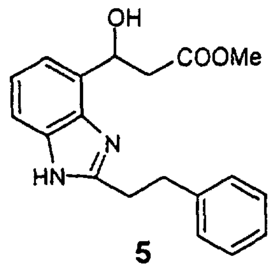

5

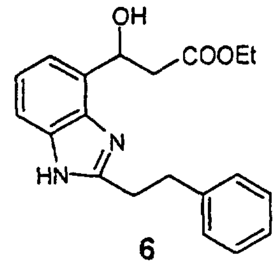

6

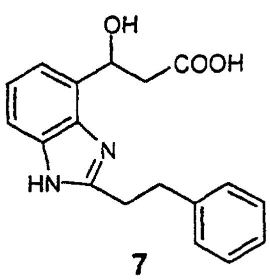

7

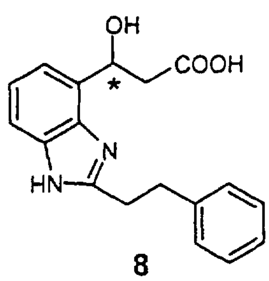

8

(+) −form

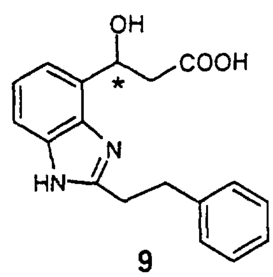

9

(−) −form

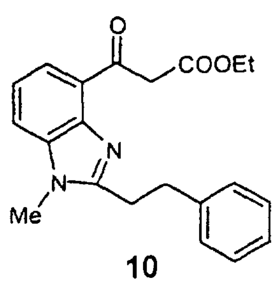

10

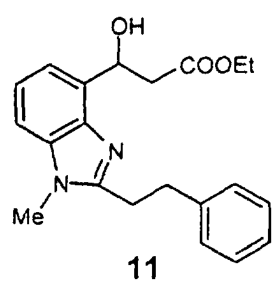

11

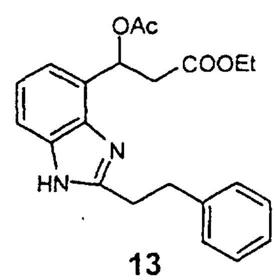

13

# Fig. 5

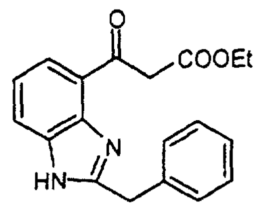

14

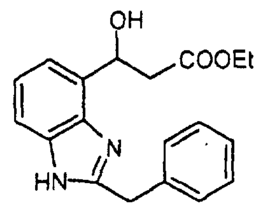

15

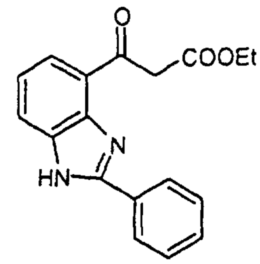

16

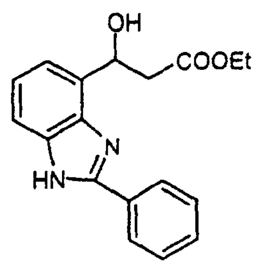

17

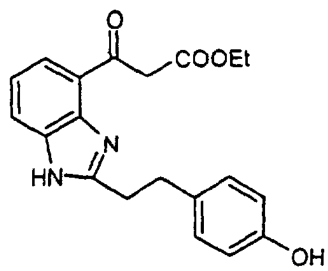

18

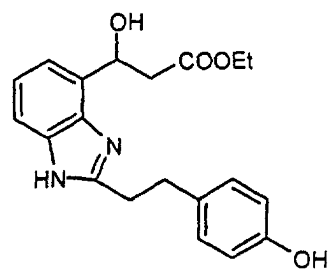

19

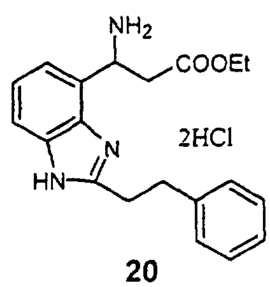

20

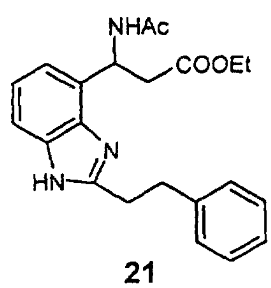

21

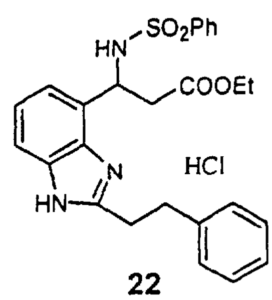

22

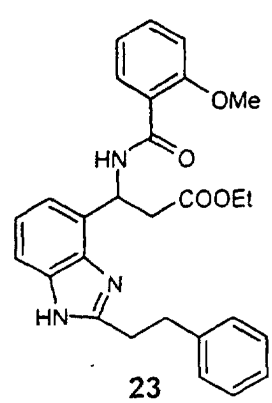

23

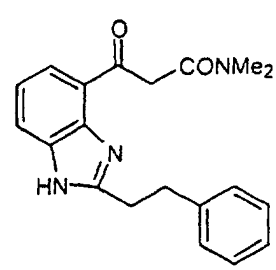

24

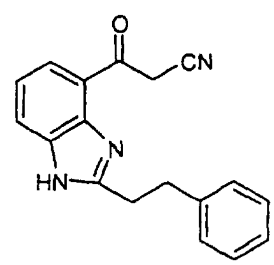

25

Fig. 6

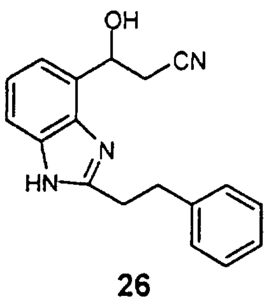

26

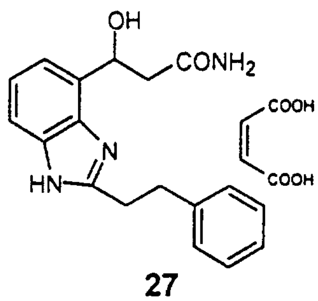

27

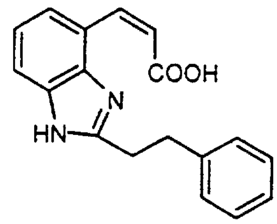

28

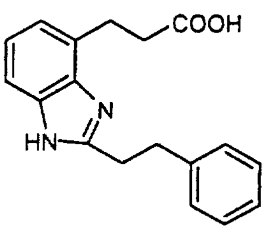

29

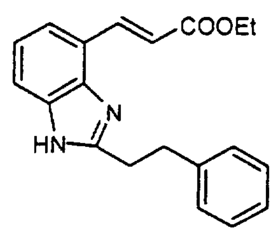

30

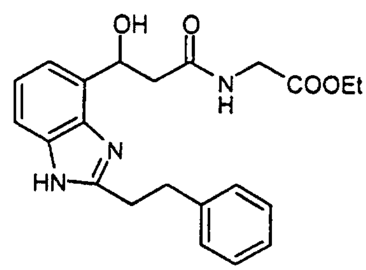

31

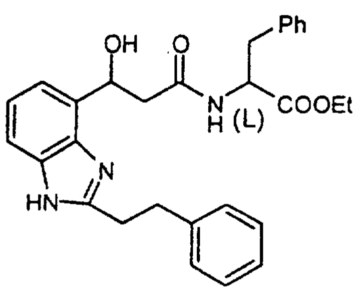

32

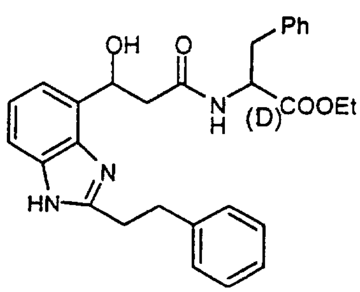

33

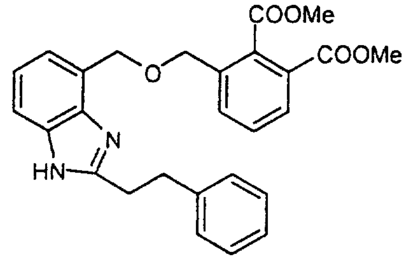

34

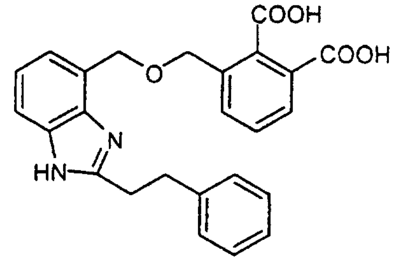

35

Fig. 7

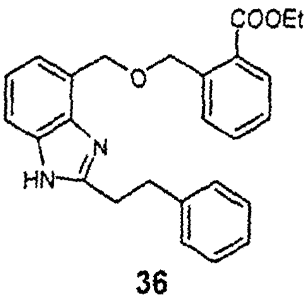

**36**

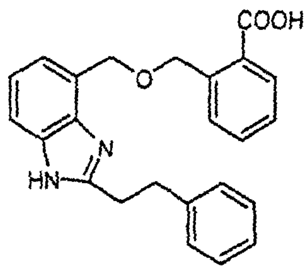

**37**

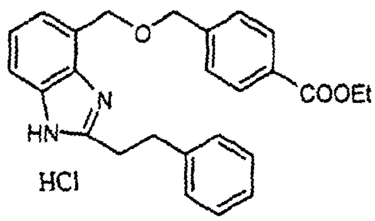

**38**

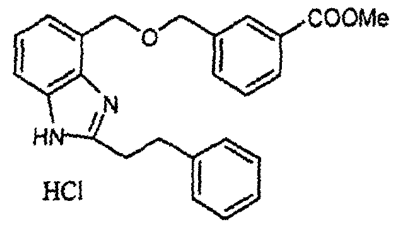

**39**

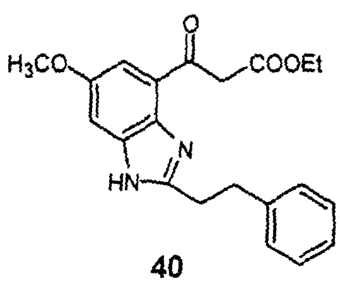

**40**

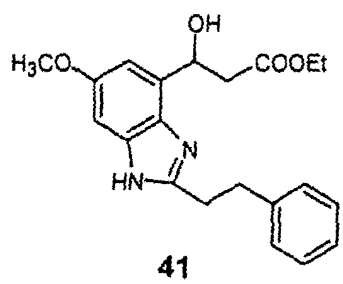

**41**

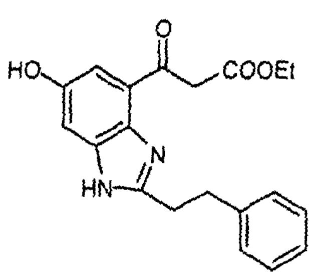

**42**

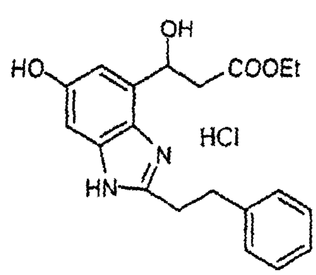

**43**

Fig. 8

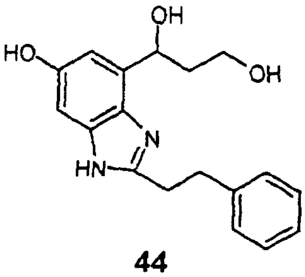

44

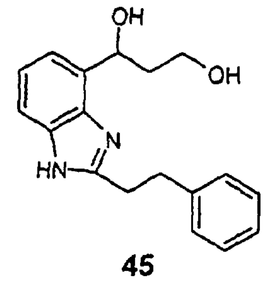

45

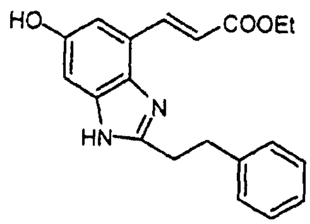

46

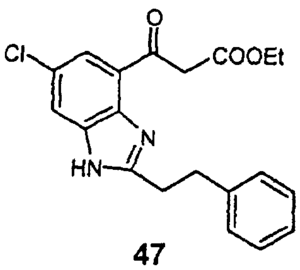

47

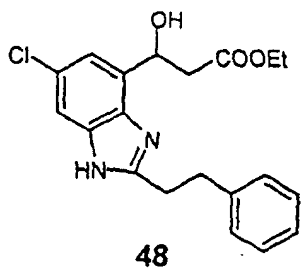

48

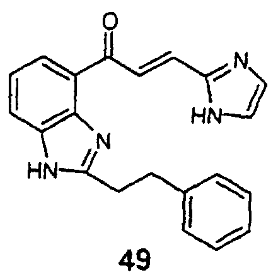

49

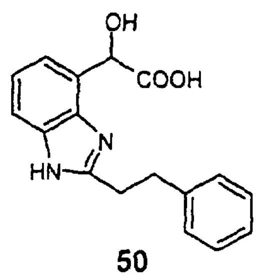

50

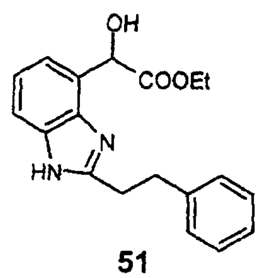

51

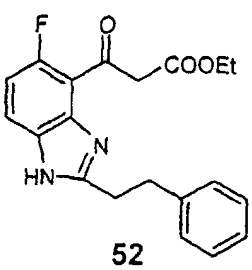

52

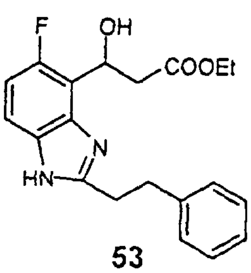

53